(19) Europäisches Patentamt / European Patent Office / Office européen des brevets

(11) EP 2 955 178 A1

(12) # EUROPEAN PATENT APPLICATION
published in accordance with Art. 153(4) EPC

(43) Date of publication:
**16.12.2015 Bulletin 2015/51**

(21) Application number: **14748779.7**

(22) Date of filing: **31.01.2014**

(51) Int Cl.:
***C07D 413/04*** *(2006.01)* ***A01N 43/76*** *(2006.01)*
***A01N 47/02*** *(2006.01)* ***A01P 7/02*** *(2006.01)*
***A01P 7/04*** *(2006.01)* ***A61K 31/4439*** *(2006.01)*
***A61P 33/14*** *(2006.01)*

(86) International application number:
**PCT/JP2014/052813**

(87) International publication number:
**WO 2014/123205 (14.08.2014 Gazette 2014/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **06.02.2013 JP 2013021234**

(71) Applicant: **Sumitomo Chemical Company Limited**
**Tokyo 104-8260 (JP)**

(72) Inventors:
- **TANABE, Takamasa**
**Takarazuka-shi**
**Hyogo 665-8555 (JP)**
- **ITO, Mai**
**Takarazuka-shi**
**Hyogo 665-8555 (JP)**
- **SHIMIZU, Chie**
**Takarazuka-shi**
**Hyogo 665-8555 (JP)**
- **NOKURA, Yoshihiko**
**Takarazuka-shi**
**Hyogo 665-8555 (JP)**

(74) Representative: **Vossius & Partner**
**Patentanwälte Rechtsanwälte mbB**
**Siebertstrasse 3**
**81675 München (DE)**

(54) **CONDENSED HETEROCYCLIC COMPOUND**

(57) A fused heterocyclic compound represented by formula (1) :

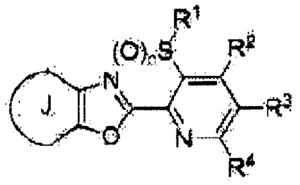

(1)

wherein $R^1$ represents a C1 to C6 alkyl group optionally having one or more halogen atoms or one or more cyclopropyl groups or the like; $R^2$ and $R^4$ represent a hydrogen atom or the like; $R^3$ represents a C1 to C6 alkyl group optionally having one or more halogen atoms or the like; n and m each represent 0, 1 or 2; and ring J represents ring J1 or ring J2 of the following formula:

EP 2 955 178 A1

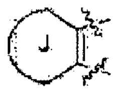 = 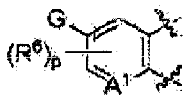 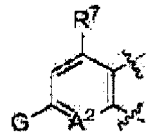

(J) (J1) (J2)

wherein $A^1$ and $A^2$ represent N or the like, G represents a C1 to C3 perhaloalkyl group or the like, $R^6$ and $R^7$ represent a C1 to C6 alkyl group optionally having one or more halogen atoms or the like, and p represents 0, 1 or 2, or an N-oxide thereof has an excellent pest controlling effect.

## Description

TECHNICAL FIELD

The present invention relates to a certain type of fused heterocyclic compound and a use thereof for pest control.

BACKGROUND ART

So far, for the purpose of pest control, various compounds have been studied and put to practical use.
On the other hand, in JP-A-2004-34438, it is known that a certain type of fused heterocyclic compound can be used in an optical recording medium.

SUMMARY OF THE INVENTION

The present invention provides a compound having an excellent control effect on pests.
The present invention is as described bellow.

[1] A fused heterocyclic compound represented by formula (1) :

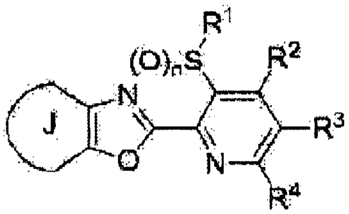
(1)

wherein

$R^1$ represents a C1 to C6 alkyl group optionally having one or more halogen atoms or one or more cyclopropyl groups (wherein the cyclopropyl group optionally has one or more halogen atoms or one or more C1 to C3 alkyl groups), a C2 to C6 alkenyl group optionally having one or more halogen atoms, a C2 to C6 alkynyl group optionally having one or more halogen atoms or a C3 to C6 cycloalkyl group optionally having one or more halogen atoms or one or more C1 to C3 alkyl groups;
$R^2$ and $R^4$ are the same or different and represent a C1 to C3 alkyl group optionally having one or more halogen atoms, a halogen atom or a hydrogen atom;
$R^3$ represents a C1 to C6 alkyl group optionally having one or more halogen atoms, a C2 to C6 alkenyl group optionally having one or more halogen atoms, a C2 to C6 alkynyl group optionally having one or more halogen atoms, a 5- or 6-membered heterocyclic group (wherein the 5- or 6-membered heterocyclic group optionally has one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a cyano group, a nitro group, a halogen atom, a hydrogen atom or $SF_5$;
$R^5$ represents a C1 to C6 alkyl group optionally having one or more halogen atoms or a hydrogen atom;
n represents 0, 1 or 2;
m represents 0, 1 or 2; and
ring J represents ring J1 or ring J2 of the following formula:

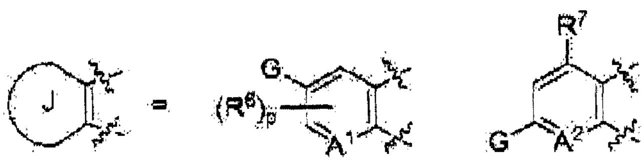
(J) (J1) (J2)

wherein
$A^1$ represents N, CH or $CR^6$;

$A^2$ represents N or $CR^8$;

G represents a C1 to C3 perhaloalkyl group, $OR^9$ or $S(O)_mR^9$ (wherein $R^3$ represents a C1 to C3 perhaloalkyl group);

$R^6$ represents a C1 to C6 alkyl group optionally having one or more halogen atoms, a C2 to C6 alkenyl group optionally having one or more halogen atoms, a C2 to C6 alkynyl group optionally having one or more halogen atoms, $OR^5$, $S(O)_mR^5$, $S(O)_zNR^5R^{10}$, $NR^5R^{10}$, $NR^5C(O)R^{10}$, $NR^5CO_2R^10$, $C(O)R^5$, $CO_2R^5$, $C(O)NR^5R^{10}$, a cyano group or a halogen atom (wherein, when p is 2, $R^6$s may be different from each other);

$R^7$ and $R^8$ are the same or different and represent a C1 to C6 alkyl group optionally having one or more halogen atoms, a C2 to C6 alkenyl group optionally having one or more halogen atoms, a C2 to C6 alkynyl group optionally having one or more halogen atoms, $OR^5$, $S(O)_mR^5$, $S(O)_2NR^5R^{10}$, $NR^5R^{10}$, $NR^5C(O)R^{10}$, $NR^5CO_2R^{10}$, $C(O)R^5$, $CO_2R^5$, $C(O)NR^5R^{10}$, a cyano group, a halogen atom or a hydrogen atom;

$R^{10}$ represents a C1 to C6 alkyl group optionally having one or more halogen atoms or a hydrogen atom; and

p represents 0, 1 or 2 (wherein, when p is 0, $A^1$ is $CR^6$), wherein, in $S(O)_mR^5$, when m is 1 or 2, $R^5$ does not represent a hydrogen atom or

an N-oxide thereof (hereinafter, the fused heterocyclic compound represented by formula (1) and the N-oxide thereof are referred to as the compound of the present invention) (in the case of the N-oxide, n represents 2, and m represents 2).

[2] The compound according to [1], wherein, in the formula (1),

$R^1$ is a C1 to C6 alkyl group optionally having one or more halogen atoms or one or more cyclopropyl groups (wherein the cyclopropyl group optionally has one or more halogen atoms or one or more C1 to C3 alkyl groups);

both $R^2$ and $R^4$ are a hydrogen atom;

$R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a halogen atom or a hydrogen atom;

$R^5$ is a C1 to C6 alkyl group optionally having one or more halogen atoms;

$A^1$ is CH or $CR^6$;

$A^2$ is CH;

G is a C1 to C3 perfluoroalkyl group, $OR^{11}$ or $S(O)_mR^{11}$ (wherein $R^{11}$ represents a C1 to C3 perfluoroalkyl group);

$R^6$ is a C1 to C6 alkyl group optionally having one or more halogen atoms, $OR^5$, $S(O)_mR^5$, $NR^5R^{10}$ or a halogen atom;

$R^7$ is a C1 to C6 alkyl group optionally having one or more halogen atoms, $OR^5$, $S(O)_mR^5$, $NR^5R^{10}$, a halogen atom or a hydrogen atom; and

p is 0 or 1 (wherein, when p is 0, $A^1$ is $CR^6$).

[3] The compound according to [1] or [2], wherein the ring J is ring J1.

[4] The compound according to [1] or [2], wherein the ring J is ring J2.

[5] A pest control agent which comprises the compound as defined in any of [1] to [4], and an inert carrier.

[6] A method for controlling pests comprising applying an effective amount of the compound as defined in any of [1] to [4] to a pest or a pest-infested area.

## MODE FOR CARRYING OUT THE INVENTION

The groups used in the description of the present specification will be described below with examples.

The halogen atom in this invention refers to a fluorine atom, a chlorine atom, a bromine atom, and an iodine atom.

Examples of the C1 to C6 alkyl group in this invention include a methyl group, an ethyl group, a propyl group, an isopropyl group, a butyl group, an isobutyl group, a sec-butyl group, a tert-butyl group, a pentyl group, a neopentyl group, and a hexyl group.

Examples of the C2 to C6 alkenyl group in this invention include a vinyl group, a 1-propenyl group, a 2-propenyl group, a 1-methylvinyl group, a 2-methyl-1-propenyl group, a 1-butenyl group, a 2-butenyl group, a 3-butenyl group, a 1-pentenyl group, and a 1-hexenyl group.

Examples of the C2 to C6 alkynyl group in this invention include an ethynyl group, a propargyl group, a 2-butynyl group, a 3-butynyl group, a 1-pentynyl group, and a 1-hexynyl group.

Examples of the C3 to C6 cycloalkyl group in this invention include a cyclopropyl group, a cyclobutyl group, a

cyclopentyl group, and a cyclohexyl group.

Examples of the C1 to C6 alkyl group optionally having one or more halogen atoms in this invention include a methyl group, an ethyl group, a propyl group, an isopropyl group, a butyl group, an isobutyl group, a sec-butyl group, a tert-butyl group, a pentyl group, a neopentyl group, a hexyl group, a fluoromethyl group, a chloromethyl group, a bromomethyl group, an iodomethyl group, a difluoromethyl group, a dichloromethyl group, a trifluoromethyl group, a chlorodifluoromethyl group, a bromodifluoromethyl group, a trichloromethyl group, a 2-fluoroethyl group, a 2-chloroethyl group, a 2-bromoethyl group, a 2, 2-difluoromethyl group, a 2,2,2-trifluoroethyl group, a pentafluoroethyl group, a heptafluoropropyl group, and a heptafluoroisopropyl group.

Examples of the C1 to C3 alkyl group optionally having one or more halogen atoms in this invention include a methyl group, an ethyl group, a propyl group, an isopropyl group, a fluoromethyl group, a chloromethyl group, a bromomethyl group, an iodomethyl group, a difluoromethyl group, a dichloromethyl group, a trifluoromethyl group, a chlorodifluoromethyl group, a bromodifluoromethyl group, a trichloromethyl group, a 2-fluoroethyl group, a 2-chloroethyl group, a 2-bromoethyl group, a 2, 2-difluoroethyl group, a 2,2,2-trifluoroethyl group, a pentafluoroethyl group, a heptafluoropropyl group, and a heptafluoroisopropyl group.

Examples of the C2 to C6 alkenyl group optionally having one or more halogen atoms in this invention include a vinyl group, a 1-propenyl group, a 2-propenyl group, a 1-methylvinyl group, a 2-methyl-1-propenyl group, a 1-butenyl group, a 2-butenyl group, a 3-butenyl group, a 1-pentenyl group, a 1-hexenyl group, a 1,1-difluoroallyl group, and a pentafluoroallyl group. Examples of the C2 to C6 alkynyl group optionally having one or more halogen atoms in this invention include an ethynyl group, a propargyl group, a 2-butynyl group, a 3-butynyl group, a 1-pentynyl group, a 1-hexynyl group, and a 4,4,4-trifluoro-2-butynyl group.

Examples of the C3 to C6 cycloalkyl group optionally having one or more halogen atoms or one or more C1 to C3 alkyl groups in this invention include a cyclopropyl group, a 1-methylcyclopropyl group, a 2-methylcyclopropyl group, a 1-fluorocyclopropyl group, a 2,2-difluorocyclopropyl group, a 2, 2-dichlorocyclopropyl group, a 2,2-dibromocyclopropyl group, a cyclobutyl group, a cyclopentyl group, and a cyclohexyl group.

Examples of the C1 to C6 alkyl group optionally having one or more halogen atoms or one or more cyclopropyl groups (wherein the cyclopropyl group optionally has one or more halogen atoms or one or more C1 to C3 alkyl groups) in this invention include a methyl group, an ethyl group, a propyl group, an isopropyl group, a butyl group, an isobutyl group, a sec-butyl group, a tert-butyl group, a pentyl group, a neopentyl group, a hexyl group, a fluoromethyl group, a chloromethyl group, a bromomethyl group, an iodomethyl group, a difluoromethyl group, a dichloromethyl group, a trifluoromethyl group, a chlorodifluoromethyl group, a bromodifluoromethyl group, a trichloromethyl group, a 2-fluoroethyl group, a 2-chloroethyl group, a 2-bromoethyl group, a 2,2-difluoroethyl group, a 2,2,2,-trifluoroethyl group, a pentafluoroethyl group, a heptafluoropropyl group, a heptafluoroisopropyl group, a cyclopropylmethyl group, a 2,2-difluorocyclopropylmethyl group, a 2,2-dimethylcyclopropylmethyl group, a 2-cyclopropylethyl group, and a 1-cyclopropylethyl group.

The C1 to C3 perhaloalkyl group in this invention represents a group in which all hydrogen atoms of the C1 to C3 alkyl group are substituted by a halogen atom, and those halogen atoms may be the same or different from each other. Examples of the C1 to C3 perhaloalkyl group include a C1 to C3 perfluoroalkyl group, a C1 to C3 perchloroalkyl group, a C1 to C3 perbromoalkyl group, and the like, and more specifically include a trifluoromethyl group, a pentafluoroethyl group, a heptafluoropropyl group, a heptafluoroisopropyl group, a bromodifluoromethyl group, a 1,1-dichloro-2,2,2-trifluoroethyl group, and the like.

The C1 to C3 perfluoroalkyl group in this invention represents a group in which all hydrogen atoms of the C1 to C3 alkyl group are substituted by a fluorine atom. Examples of the C1 to C3 perfluoroalkyl group include a trifluoromethyl group, a pentafluoroethyl group, a heptafluoropropyl group, and a heptafluoroisopropyl group.

The heterocyclic group in this invention represents a heterocyclic compound residue containing one or more nitrogen atoms, oxygen atoms or sulfur atoms, other than carbon atoms as ring-constituting atoms, in the ring structure. Examples of the heterocyclic group include 5-membered non-aromatic heterocyclic groups such as a pyrrolidyl group, a tetrahydrofuryl group and a tetrahydrothienyl group, 5-membered aromatic heterocyclic groups such as a pyrrolyl group, a pyrazolyl group, an imidazolyl group, a furyl group, a thienyl group, an oxazolyl group and a thiazolyl group, 6-membered non-aromatic heterocyclic groups such as a piperidyl group, a tetrahydropyranyl group, a tetrahydrothiopyranyl group, a piperazinyl group and a morpholinyl group (including a morpholino group that is a 4-morpholinyl group), and 6-membered aromatic heterocyclic groups such as a pyridyl group, a pyrimidinyl group, a pyridazinyl group, and a pyrazinyl group.

In addition, in this invention, the 5-membered heterocyclic group refers to a 5-membered non-aromatic heterocyclic group and a 5-membered aromatic heterocyclic group, and the 6-membered heterocyclic group refers to a 6-membered non-aromatic heterocyclic group and a 6-membered aromatic heterocyclic group.

Examples of the 5- or 6-membered heterocyclic group (wherein the 5- or 6-membered heterocyclic group optionally has one or more atoms or groups selected from the group consisting of halogen atoms, C1 to C3 alkyl groups optionally having one or more halogen atoms, and C1 to C3 alkoxy groups optionally having one or more halogen atoms) in this invention include 5-or 6-membered non-aromatic heterocyclic groups such as a pyrrolidin-1-yl group, a 3,3,4,9-tetrafluoropyrrolidin-1-yl group, a tetrahydrofuran-2-yl group, a piperidyl group, a morpholinyl group (including a morpholino group that is a 4-morpholinyl group) and a thiomorpholinyl group (including a thiomorpholino group that is a 4-thiomorpholinyl group); and 5- or 6-membered aromatic heterocyclic groups such as a 2-pyrrolyl group, a 2-furyl group, a 3-furyl group, a 5-pyrazolyl group, a 4-pyrazolyl group, a 1-pyrrolyl group, a 1-methyl-2-pyrrolyl group, a 2-methylsulfanyl-1-pyrrolyl group, a 2-methylsulfinyl-1-pyrrolyl group, a 2-methylsulfonyl-1-pyrrolyl group, a 2-methylamino-1-pyrrolyl group, a 2-dimethylamino-1-pyrrolyl group, a 5-bromo-2-furyl group, a 5-nitro-2-furyl group, a 5-cyano-2-furyl group, a 5-methoxy-2-furyl group, a 5-acetyl-2-furyl group, a 5-methoxycarbonyl-2-furyl group, a 2-methyl-3-furyl group, a 2,5-dimethyl-3-furyl group, a 2,4-dimethyl-3-furyl group, a 5-methyl-2-thienyl group, a 3-methyl-2-thienyl group, a 1-methyl-3-trifluoromethyl-5-pyrazolyl group, a 5-chloro-1,3-dimethyl-4-pyrazolyl group, a pyrazol-1-yl group, a 3-chloro-pyrazol-1-yl group, a 3-bromopyrazol-1-yl group, a 4-chloropyrazol-1-yl group, a 4-bromopyrazol-1-yl group, an imidazol-1-yl group, a 1,2,4-triazol-1-yl group, a 3-chloro-1,2,4-triazol-1-yl group, a 1,2,3,4-tetrazol-1-yl group, a 1,2,3,5-tetrazol-1-yl group, a 2-thienyl group, a 3-thienyl group, a 3-trifluoromethyl-1,2,4-triazol-1-yl group, a 4-trifluoromethylpyrazol-1-yl group, a pyrazinyl group, a 4-pyrimidinyl group, a 5-pyrimidinyl group, a 2-pyridyl group, a 3-pyridyl group, a 4-pyridyl group, a 3-fluoro-2-pyridyl group, a 4-fluoro-2-pyridyl group, a 5-fluoro-2-pyridyl group, a 6-fluoro-2-pyridyl group, a 2-pyrimidinyl group, a 3-chloro-5-trifluoromethylpyridin-2-yl group, a 5-trifluoromethylpyridin-2-yl group and a 5-trifluoromethoxypyridin-2-yl group.

Examples of the pyridyl group and pyrimidinyl group (wherein the pyridyl group and pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms) in this invention include a 2-pyridyl group, a 3-pyridyl group, a 4-pyridyl group, a 5-trifluoromethyl-2-pyridyl group, a 3-chloro-5-trifluoromethyl-2-pyridyl group, a 2-pyrimidinyl group, a 4-pyrimidinyl group, a 5-pyrimidinyl group, and a 2-chloro-4-pyrimidinyl group.

In the compound of the present invention, an N-oxide is a compound in which the nitrogen atom constituting the ring on the heterocyclic group is oxidized. Examples of the heterocyclic group that may form an N-oxide include a pyridine ring.

In the compound of the present invention, examples of the N-oxide include the compounds represented by the formulae (1A) to (1F) set forth below.

Examples of the compound of the present invention include the following compounds.

In the formula (1), compounds wherein $R^1$ is a C1 to C6 alkyl group optionally having one or more halogen atoms or one or more cyclopropyl groups (wherein the cyclopropyl group optionally has one or more halogen atoms or one or more C1 to C3 alkyl groups);

In the formula (1), compounds wherein $R^1$ is a C1 to C6 alkyl group optionally having one or more halogen atoms;

In the formula (1), compounds wherein $R^1$ is a C1 to C6 alkyl group having one or more halogen atoms;

In the formula (1), compounds wherein $R^1$ is a C1 to C6 alkyl group;

In the formula (1), compounds wherein $R^1$ is a C1 to C3 alkyl group;

In the formula (1), compounds wherein $R^1$ is a C1 to C6 alkyl group optionally having one or more cyclopropyl groups (wherein the cyclopropyl group optionally has one or more halogen atoms or one or more C1 to C3 alkyl groups);

In the formula (1), compounds wherein $R^1$ is a C1 to C6 alkyl group optionally having one or more cyclopropyl groups;

In the formula (1), compounds wherein $R^1$ is a C1 to C6 alkyl group having one or more cyclopropyl groups;

In the formula (1), compounds wherein $R^1$ is a methyl group, an ethyl group, a propyl group, a cyclopropyl group or a cyclopropylmethyl group;

In the formula (1), compounds wherein $R^1$ is a methyl group;

In the formula (1), compounds wherein $R^1$ is an ethyl group;

In the formula (1), compounds wherein $R^1$ is a propyl group;

In the formula (1), compounds wherein $R^1$ is a cyclopropyl group;

In the formula (1), compounds wherein $R^1$ is a cyclopropylmethyl group;

In the formula (1), compounds wherein both $R^2$ and $R^4$ are a hydrogen atom;

In the formula (1), compounds wherein both $R^2$ and $R^4$ are a hydrogen atom, and $R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a halogen atom or a hydrogen atom;

In the formula (1), compounds wherein both $R^2$ and $R^4$ are a hydrogen atom, and $R^3$ is a C1 to C6 alkyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a halogen atom or a hydrogen atom;

In the formula (1), compounds wherein both $R^2$ and $R^4$ are a hydrogen atom, and $R^3$ is a C1 to C6 alkyl group optionally having one or more halogen atoms, a halogen atom or a hydrogen atom; In the formula (1), compounds wherein both $R^2$ and $R^4$ are a hydrogen atom, and $R^3$ is a C1 to C6 alkyl group optionally having one or more halogen atoms or a hydrogen atom;
In the formula (1), compounds wherein both $R^2$ and $R^4$ are a hydrogen atom, and $R^3$ is a trifluoromethyl group, a halogen atom or a hydrogen atom;
In the formula (1), compounds wherein both $R^2$ and $R^4$ are a hydrogen atom, and $R^3$ is a trifluoromethyl group or a hydrogen atom;
In the formula (1), compounds wherein both $R^2$ and $R^4$ are a hydrogen atom, and $R^3$ is a trifluoromethyl group;
In the formula (1), compounds wherein both $R^2$ and $R^4$ are a hydrogen atom, and $R^3$ is a hydrogen atom;
In the formula (1), compounds wherein $R^1$ is a methyl group, an ethyl group, a propyl group, a cyclopropyl group or a cyclopropylmethyl group, both $R^2$ and $R^4$ are a hydrogen atom, and $R^3$ is a trifluoromethyl group, a halogen atom or a hydrogen atom;
In the formula (1), compounds wherein $R^1$ is an ethyl group, both $R^2$ and $R^4$ are a hydrogen atom, and $R^3$ is a trifluoromethyl group or a hydrogen atom;
In the formula (1), compounds wherein $R^1$ is an ethyl group, both $R^2$ and $R^4$ are a hydrogen atom, and $R^3$ is a trifluoromethyl group; In the formula (1), compounds wherein $R^1$ is an ethyl group, both $R^2$ and $R^4$ are a hydrogen atom, and $R^3$ is a hydrogen atom;
In the formula (1), compounds wherein $R^1$ is a C1 to C6 alkyl group optionally having one or more halogen atoms or one or more cyclopropyl groups (wherein the cyclopropyl group optionally has one or more halogen atoms or one or more C1 to C3 alkyl groups), all of $R^2$, $R^3$ and $R^4$ are a hydrogen atom, $A^1$ is CH or $CR^6$, $A^2$ is CH, G is a C1 to C3 perfluoroalkyl group or $S(O)_mR^{11}$ (wherein $R^{11}$ represents a C1 to C3 perfluoroalkyl group), $R^6$ is a halogen atom, $R^7$ is a hydrogen atom, and p is 0 or 1 (wherein, when p is 0, $A^1$ is $CR^6$) ;
In the formula (1), compounds wherein the ring J is ring J1; In the formula (1), compounds wherein the ring J is ring J1, and $A^1$ is N;
In the formula (1), compounds wherein the ring J is ring J1, and $A^1$ is CH;
In the formula (1), compounds wherein the ring J is ring J1, and $A^1$ is $CR^6$;
In the formula (1), compounds wherein the ring J is ring J1, $A^1$ is N, and p is 1;
In the formula (1), compounds wherein the ring J is ring J1, $A^1$ is N, and p is 2;
In the formula (1), compounds wherein the ring J is ring J1, $A^1$ is CH, and p is 1;
In the formula (1), compounds wherein the ring J is ring J1, $A^1$ is CH, and p is 2;
In the formula (1), compounds wherein the ring J is ring J1, $A^1$ is $GR^6$, and p is 0;
In the formula (1), compounds wherein the ring J is ring J1, $A^1$ is $CR^6$, and p is l;
In the formula (1), compounds wherein the ring J is ring J1, $A^1$ is $CR^6$, and p is 2;
In the formula (1), compounds wherein the ring J is ring J1, and G is a C1 to C3 perfluoroalkyl group;
In the formula (1), compounds wherein the ring J is ring J1, and G is $OR^{11}$;
In the formula (1), compounds wherein the ring J is ring J1, and G is $S(O)_mR^{11}$;
In the formula (1), compounds wherein the ring J is ring J1, and G is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group;
In the formula (1), compounds wherein the ring J is ring J1, and G is a trifluoromethyl group;
In the formula (1), compounds wherein the ring J is ring J1, and G is a pentafluoroethyl group;
In the formula (1), compounds wherein the ring J is ring J1, and G is a heptafluoropropyl group;
In the formula (1), compounds wherein the ring J is ring J1, and G is a heptafluoroisopropyl group;
In the formula (1), compounds wherein the ring J is ring J1, and G is a trifluoromethoxy group;
In the formula (1), compounds wherein the ring J is ring J1, and G is a trifluoromethylsulfanyl group;
In the formula (1), compounds wherein the ring J is ring J1, and G is a trifluoronethylsulfinyl group;
In the formula (1), compounds wherein the ring J is ring J1, and G is a trifluoromethylsulfonyl group;
In the formula (1), compounds wherein the ring J is ring J1, $R^1$ is a C1 to C6 alkyl group optionally having one or more halogen atoms or one or more cyclopropyl groups (wherein the cyclopropyl group optionally has one or more halogen atoms or one or more C1 to C3 alkyl groups), both $R^2$ and $R^4$ are a hydrogen atom, $R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a halogen atom or a hydrogen atom, $R^5$ is a C1 to C6 alkyl group optionally having one or more halogen atoms, $A^1$ is CH or $CR^6$, G is a C1 to C3 perfluoroalkyl group, $OR^{11}$, or $S(O)_mR^{11}$ (wherein $R^{11}$ represents a C1 to C3 perfluoroalkyl group), $R^6$ is a C1 to C6 alkyl group optionally having

one or more halogen atoms, $OR^5$, $5(O)_mP^5$, $NR^5R^{10}$ or a halogen atom, and p is 0 or 1 (wherein, when p is 0, $A^1$ is $CR^6$); In the formula (1), compounds wherein the ring J is ring J1, $R^1$ is a C1 to C6 alkyl group optionally having one or more halogen atoms or one or more cyclopropyl groups (wherein the cyclopropyl group optionally has one or more halogen atoms or one or more C1 to C3 alkyl groups), all of $R^2$, $R^7$ and $R^4$ are a hydrogen atoms, $A^1$ is CH or $CR^6$, G is a C1 to C3 perfluoroalkyl group or $S(O)_mR^{11}$ (wherein $R^{11}$ represents a C1 to C3 perfluoroalkyl group), $R^6$ is a halogen atom, and p is 0 or 1 (wherein, when p is 0, $A^1$ is $CR^6$) ;
In the formula (1), compounds wherein the ring J is ring J1, and the ring J1 is ring J3, ring J4, ring J5, ring J6 or ring J7 of the following formulae:

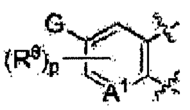 = 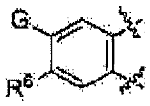 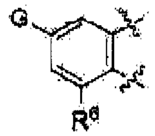 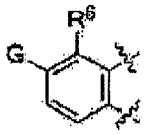 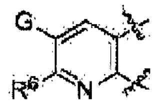 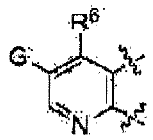

(J1) (J3) (J4) (J5) (J6) (J7)

wherein symbols represent the same meaning as in the formula (1);
In the formula (1), compounds wherein the ring J is ring J1, and the ring J1 is ring J3;
In the formula (1), compounds wherein the ring J is ring J1, and the ring J1 is ring J4;
In the formula (1), compounds wherein the ring J is ring J1, and the ring J1 is ring J5;
In the formula (1), compounds wherein the ring J is ring J1, and the ring J1 is ring J6;
In the formula (1), compounds wherein the ring J is ring J1, and the ring J1 is ring J7;
In the formula (1), compounds wherein the ring J is ring J2; In the formula (1), compounds wherein the ring J is ring J2, and $A^2$ is N;
In the formula (1), compounds wherein the ring J is ring J2, and $A^2$ is CH;
In the formula (1), compounds wherein the ring J is ring J2, and $A^2$ is $CR^8$;
In the formula (1), compounds wherein the ring J is ring J2, $A^2$ is N, and $R^7$ is a hydrogen atom; In the formula (1), compounds wherein the ring J is ring J2, $A^2$ is N, and $R^7$ is a C1 to C6 alkyl group optionally having one or more halogen atoms, a C2 to C6 alkenyl group optionally having one or more halogen atoms, a C2 to C6 alkynyl group optionally having one or more halogen atoms, $OR^5$, $S(O)mR^5$, $S(O)_2NR^5R^{10}$, $NR^5R^{10}$, $NR^5C(O)R^{10}$, $NR^5CO_2R^{10}$, $C(O)R^5$, $CO_2R^5$, $C(O)NR^5R^{10}$, a cyano group or a halogen atom; In the formula (1), compounds wherein the ring J is ring J2, $A^2$ is CH, and $R^7$ is a hydrogen atom;
In the formula (1), compounds wherein the ring J is ring J2, $A^2$ is CH, and $R^7$ is a C1 to C6 alkyl group optionally having one or more halogen atoms, a C2 to C6 alkenyl group optionally having one or more halogen atoms, a C2 to C6 alkynyl group optionally having one or more halogen atoms, $OR^5$, $S(O)_mR^5$, $S(O)_2NR^5R^{10}$, $NR^5R^{10}$, $NR^5C(O)R^{10}$, $NR^5CO_2R^{10}$, $C(O)R^5$, $CO_2R^5$, $C(O)NR^5R^{10}$, a cyano group or a halogen atom;
In the formula (1), compounds wherein the ring J is ring J2, $A^2$ is $CR^8$, $R^8$ is a C1 to C6 alkyl group optionally having one or more halogen atoms, a C2 to C6 alkenyl group optionally having one or more halogen atoms, a C2 to C6 alkynyl group optionally having one or more halogen atoms, $OR^5$, $S(O)_mR^5$, $S(O)_2NR^5R^{10}$, $NR^5R^{10}$, $NR^5C(O)R^{10}$, $NR^5CO_2R^{10}$, $C(O)R^5$, $CO_2R^5$, $C(O)NR^5R^{10}$, a cyano group or a halogen atom, and $R^7$ is a hydrogen atom;
In the formula (1), compounds wherein the ring J is ring J2, and G is a C1 to C3 perfluoroalkyl group;
In the formula (1), compounds wherein the ring J is ring J2, and G is $OR^{11}$;
In the formula (1), compounds wherein the ring J is ring J2, and G is $S(O)_mR^{11}$;
In the formula (1), compounds wherein the ring J is ring J2, and G is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group;
In the formula (1), compounds wherein the ring J is ring J2, and G is a trifluoromethyl group;
In the formula (1), compounds wherein the ring J is ring J2, and G is a pentafluoroethyl group;
In the formula (1), compounds wherein the ring J is ring J2, and G is a heptafluoropropyl group;
In the formula (1), compounds wherein the ring J is ring J2, and G is a heptafluoroisopropyl group;
In the formula (1), compounds wherein the ring J is ring J2, and G is a trifluoromethoxy group;
In the formula (1), compounds wherein the ring J is ring J2, and G is a trifluoromethylsulfanyl group;
In the formula (1), compounds wherein the ring J is ring J2, and G is a trifluoromethylsulfinyl group;
In the formula (1), compounds wherein the ring J is ring J2, and G is a trifluoromethylsulfonyl group;
In the formula (1), compounds wherein the ring J is ring J2, $R^1$ is a C1 to C6 alkyl group optionally having one or more halogen atoms or one or more cyclopropyl groups (wherein the cyclopropyl group optionally has one or more halogen atoms or one or more C1 to C3 alkyl groups), both $R^2$ and $R^4$ are a hydrogen atom, $R^3$ is a C1 to C6 alkyl group optionally

having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a halogen atom or a hydrogen atom, $R^5$ is a C1 to C6 alkyl group optionally having one or more halogen atoms, $A^2$ is CH, G is a C1 to C3 perfluoroalkyl group, $OR^{11}$, or $S(O)_mR^{11}$ (wherein $R^{11}$ represents a C1 to C3 perfluoroalkyl group), and $R^7$ is a C1 to C6 alkyl group optionally having one or more halogen atoms, $OR^5$, $S(O)_mR^5$, $NR^5R^{10}$, a halogen atom or a hydrogen atom;

In the formula (1), compounds wherein the ring J is ring J2, $R^1$ is a C1 to C6 alkyl group optionally having one or more halogen atoms or one or more cyclopropyl groups (wherein the cyclopropyl group optionally has one or more halogen atoms or one or more C1 to C3 alkyl groups), all of $R^2$, $R^3$, $R^4$ and $R^7$ are a hydrogen atom, $A^2$ is CH, and G is a C1 to C3 perfluoroalkyl group;

In the formula (1), compounds wherein the ring J is ring J2, and the ring J2 is ring J8 or ring J9 of the following formulae:

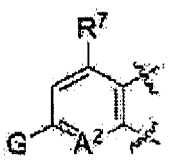 = 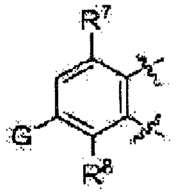 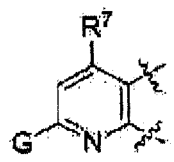

(J2) (J8) (J9)

wherein symbols represent the same meaning as in the formula (1) ;

In the formula (1), compounds wherein the ring J is ring J2, and the ring J2 is ring J8;

In the formula (1), compounds wherein the ring J is ring J2, the ring J2 is ring J8, and $R^7$ is a hydrogen atom;

In the formula (1), compounds wherein the ring J is ring J2, the ring J2 is ring J8, and $R^8$ is a hydrogen atom;

In the formula (1), compounds wherein the ring J is ring J2, the ring J2 is ring J8, and both $R^7$ and $R^8$ are a hydrogen atom; In the formula (1), compounds wherein the ring J is ring J2, and the ring J2 is ring J9;

In the formula (1), compounds wherein the ring J is ring J2, the ring J2 is ring J9, and $R^7$ is a hydrogen atom; Compounds represented by formula (1-1),

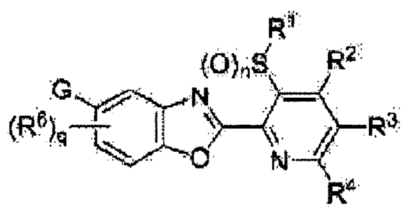

(1-1)

wherein q represents 1, 2 or 3, and other symbols represent the same meaning as in the formula (1);

Compounds represented by formula (1-1a),

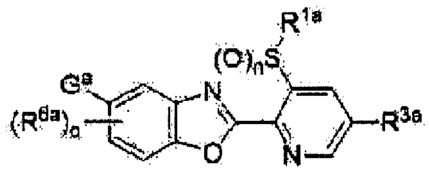

(1-1a)

wherein

$R^{1a}$ represents a C1 to C6 alkyl group optionally having one or more halogen atoms or one or more cyclopropyl groups (wherein the cyclopropyl group optionally has one or more halogen atoms or one or more C1 to C3 alkyl groups),

$R^{3a}$ represents a C1 to C6 alkyl group optionally having one or more halogen atoms, a C2 to C6 alkenyl group optionally having one or more halogen atoms, a C2 to C6 alkynyl group optionally having one or more halogen

atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^{5a}$, $S(O)_mR^{5a}$, a halogen atom or a hydrogen atom,
$R^{5a}$ represents a C1 to C6 alkyl group optionally having one or more halogen atoms,
$G^a$ represents a C1 to C3 perfluoroalkyl group, $OR^{11}$ or $S(O)_mR^{11}$,
$R^{6a}$ represents a C1 to C6 alkyl group optionally having one or more halogen atoms, $OR^{5a}$, $S(O)_mR^{5a}$, $NR^{5a}R^{10a}$ or a halogen atom,
q represents 1, 2 or 3,
m represents 0, 1 or 2, and
n represents 0, 1 or 2;

In the formula (1-1a), compounds wherein q is 1;
In the formula (1-1a), compounds wherein q is 2;
In the formula (1-1a), compounds wherein q is 3;
In the formula (1-1a), compounds wherein $R^{1a}$ is a C1 to C6 alkyl group optionally having one or more halogen atoms;
In the formula (1-1a), compounds wherein $R^{1a}$ is a C1 to C6 alkyl group;
In the formula (1-1a), compounds wherein $R^{1A}$ is a C1 to C3 alkyl group;
In the formula (1-1a), compounds wherein $R^{1a}$ is a methyl group, an ethyl group, a propyl group, a cyclopropyl group or a cyclopropylmethyl group;
In the formula (1-1a), compounds wherein $R^{1a}$ is a methyl group, an ethyl group or a propyl group;
In the formula (1-1a), compounds wherein $R^{3a}$ is a C1 to C6 alkyl group optionally having one or more halogen atoms or a hydrogen atom;
In the formula (1-1a), compounds wherein $R^{3a}$ is a trifluoromethyl group, a halogen atom or a hydrogen atom; In the formula (1-1a), compounds wherein $R^{1a}$ is an ethyl group, and $R^{3a}$ is a trifluoromethyl group or a hydrogen atom;
In the formula (1-1a), compounds wherein $R^{1a}$ is an ethyl group, and $R^{3a}$ is a trifluoromethyl group;
In the formula (1-1a), compounds wherein $R^{1a}$ is an ethyl group, and $R^{3a}$ is a hydrogen atom;
In the formula (1-1a), compounds wherein $G^a$ is a C1 to C3 perfluoroalkyl group;
In the formula (1-1a), compounds wherein $G^a$ is $OR^{11}$;
In the formula (1-1a), compounds wherein $G^a$ is $S(O)_mR^{11}$;
In the formula (1-1a), compounds wherein $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group;
In the formula (1-1a), compounds wherein $G^a$ is a trifluoromethyl group;
In the formula (1-1a), compounds wherein $G^a$ is a pentafluoroethyl group;
In the formula (1-1a), compounds wherein $G^a$ is a heptafluoropropyl group;
In the formula (1-1a), compounds wherein $G^a$ is a heptafluoroisopropyl group;
In the formula (1-1a), compounds wherein $G^a$ is a trifluoromethoxy group;
In the formula (1-1a), compounds wherein $G^a$ is a trifluoromethylsulfanyl group;
In the formula (1-1a), compounds wherein $G^a$ is a trifluoromethylsulfinyl group;
In the formula (1-1a), compounds wherein $G^a$ is a trifluoromethylsulfonyl group;
In the formula (1-1a), compounds wherein q is 1, and $R^{6a}$ is $OR^{5a}$, $S(O)_mR^{5a}$, $NR^{5a}R^{10a}$ or a halogen atom;
In the formula (1-1a), compounds wherein q is 1, and $R^{6a}$ is a methoxy group, an ethoxy group, a methylsulfanyl group, a methylsulfonyl group, an ethylsulfanyl group, an ethylsulfonyl group, a methylamino group, a dimethylamino group, a diethylamino group or a halogen atom;
In the formula (1-1a), compounds wherein q is 1, and $R^{6a}$ is a halogen atom;
In the formula (1-1a), compounds wherein q is 1, and $R^{6a}$ is a fluorine atom;
In the formula (1-1a), compounds wherein q is 1, and $R^{6a}$ is a chlorine atom;
In the formula (1-1a), compounds wherein q is 1, and $R^{6a}$ is a bromine atom;
In the formula (1-1a), compounds wherein q is 1, and $R^{6a}$ is an iodine atom;
In the formula (1-1a), compounds wherein $R^{1a}$ is a C1 to C3 alkyl group, $R^{3a}$ is a C1 to C6 alkyl group optionally having one or more halogen atoms, or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, q is 1, and $R^{6a}$ is $OR^{5a}$, $S(O)_mR^{5a}$, $NR^{5a}R^{10a}$ or a halogen atom;
In the formula (1-1a), compounds wherein $R^{1a}$ is an ethyl group, $R^{3a}$ is a trifluoromethyl group or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, q is 1, and $R^{6a}$ is a methoxy group, an ethoxy group, a methylsulfanyl group, a methylsulfonyl group, an ethylsulfanyl group,

an ethylsulfonyl group, a methylamino group, a dimethylamino group, a diethylamino group or a halogen atom; Compounds represented by formula (1-2),

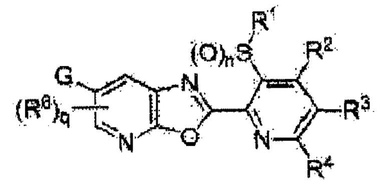

(1-2)

wherein q represents 1, 2 or 3, and other symbols represent the same meaning as in the formula (1);
compounds represented by formula (1-2a),

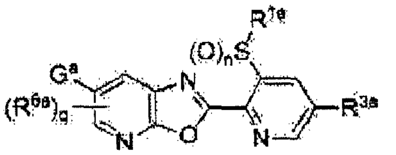

(1-2a)

wherein

$R^{1a}$ represents a C1 to C6 alkyl group optionally having one or more halogen atoms or one or more cyclopropyl groups (wherein the cyclopropyl group optionally has one or more halogen atoms or one or more C1 to C3 alkyl groups),
$R^{3a}$ represents a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^{5a}$, $S(O)_mR^{5a}$, a halogen atom or a hydrogen atom,
$R^{5a}$ represents a C1 to C6 alkyl group optionally having one or more halogen atoms,
$G^a$ represents a C1 to C3 perfluoroalkyl group, $OR^{11}$ or $S(O)_mR^{11}$,
$R^{6a}$ represents a C1 to C6 alkyl group optionally having one or more halogen atoms, $OR^{5a}$, $S(O)_mR^{5a}$, $NR^{5a}R^{10a}$ or a halogen atom,
q represents 1 or 2, m represents 0, 1 or 2, and
n represents 0, 1 or 2;

In the formula (1-2a), compounds wherein q is 1;
In the formula (1-2a), compounds wherein q is 2;
In the formula (1-2a), compounds wherein $R^{1a}$ is a C1 to C6 alkyl group optionally having one or more halogen atoms;
In the formula (1-2a), compounds wherein $R^{1a}$ is a C1 to C6 alkyl group;
In the formula (1-2a), compounds wherein $R^{1a}$ is a C1 to C3 alkyl group;
In the formula (1-2a), compounds wherein $R^{1a}$ is a methyl group, an ethyl group, a propyl group, a cyclopropyl group or a cyclopropylmethyl group;
In the formula (1-2a), compounds wherein $R^{1a}$ is a methyl group, an ethyl group or a propyl group;
In the formula (1-2a), compounds wherein $R^{3a}$ is a C1 to C6 alkyl group optionally having one or more halogen atoms or a hydrogen atom;
In the formula (1-2a), compounds wherein $R^{3a}$ is a trifluoromethyl group, a halogen atom or a hydrogen atom; In the formula (1-2a), compounds wherein $R^{1a}$ is an ethyl group, and $R^{3a}$ is a trifluoromethyl group or a hydrogen atom;
In the formula (1-2a), compounds wherein $R^{1a}$ is an ethyl group, and $R^{3a}$ is a trifluoromethyl group;
In the formula (1-2a), compounds wherein $R^{1a}$ is an ethyl group, and $R^{3a}$ is a hydrogen atom;
In the formula (1-2a), compounds wherein $G^a$ is a C1 to C3 perfluoroalkyl group;
In the formula (1-2a), compounds wherein $G^a$ is $OR^{11}$;
In the formula (1-2a), compounds wherein $G^a$ is $S(O)_mR^{11}$;
In the formula (1-2a), compounds wherein $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group;
In the formula (1-2a), compounds wherein $G^a$ is a trifluoromethyl group;

In the formula (1-2a), compounds wherein $G^a$ is a pentafluoroethyl group;
In the formula (1-2a), compounds wherein $G^a$ is a heptafluoropropyl group;
In the formula (1-2a), compounds wherein $G^a$ is a heptafluoroisopropyl group;
In the formula (1-2a), compounds wherein $G^a$ is a trifluoromethoxy group;
In the formula (1-2a), compounds wherein $G^a$ is a trifluoromethylsulfanyl group;
In the formula (1-2a), compounds wherein $G^a$ is a trifluoromethylsulfinyl group;
In the formula (1-2a), compounds wherein $G^a$ is a trifluoromethylsulfonyl group;
In the formula (1-2a), compounds wherein q is 1, and $R^{6a}$ is $OR^{5a}$, $S(O)_mR^{5a}$, $NR^{5a}R^{10a}$ or a halogen atom;
In the formula (1-2a), compounds wherein q is 1, and $R^{6a}$ is a methoxy group, an ethoxy group, a methylsulfanyl group, a methylsulfonyl group, an ethylsulfanyl group, an ethylsulfonyl group, a methylamino group, a dimethylamino group, a diethylamino group or a halogen atom;
In the formula (1-2a), compounds wherein q is 1, and $R^{6a}$ is a halogen atom;
In the formula (1-2a), compounds wherein q is 1, and $R^{6a}$ is a fluorine atom;
In the formula (1-2a), compounds wherein q is 1, and $R^{6a}$ is a chlorine atom;
In the formula (1-2a), compounds wherein q is 1, and $R^{6a}$ is a bromine atom;
In the formula (1-2a), compounds wherein q is l, and $R^{6a}$ is an iodine atom;
In the formula (1-2a), compounds wherein $R^{1a}$ is a C1 to C3 alkyl group, $R^{3a}$ is a C1 to C6 alkyl group optionally having one or more halogen atoms, or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, q is 1, and $R^{6a}$ is $OR^{5a}$, $S(O)_mR^{5a}$, $NR^{5a}R^{10a}$ or a halogen atom;
In the formula (1-2a), compounds wherein $R^{1a}$ is an ethyl group, $R^{3a}$ is a trifluoromethyl group or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, q is 1, and $R^{6a}$ is a methoxy group, an ethoxy group, a methylsulfanyl group, a methylsulfonyl group, an ethylsulfanyl group, an ethylsulfonyl group, a methylamino group, a dimethylamino group, a diethylamino group or a halogen atom; Compounds represented by formula (1-3),

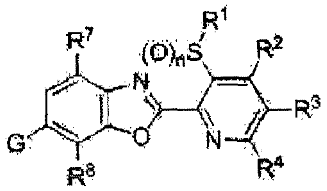
(1-3)

wherein symbols represent the same meaning as in the formula (1) ;
Compounds represented by formula (1-3a),

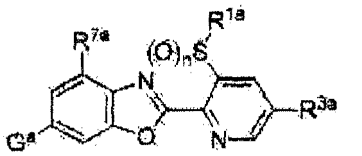
(1-3a)

wherein

$R^{1a}$ represents a C1 to C6 alkyl group optionally having one or more halogen atoms or one or more cyclopropyl groups (wherein the cyclopropyl group optionally has one or more halogen atoms or one or more C1 to C3 alkyl groups),
$R^{3a}$ represents a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^{5a}$, $S(O)_mR^{5a}$, a halogen atom or a hydrogen atom,
$R^{5a}$ represents a C1 to C6 alkyl group optionally having one or more halogen atoms,
$G^a$ represents a C1 to C3 perfluoroalkyl group, $OR^{11}$ or $S(O)_mR^{11}$,
$R^{7a}$ represents a C1 to C6 alkyl group optionally having one or more halogen atoms, $OR^{5a}$, $S(O)_mR^{5a}$, $NR^{5a}R^{10a}$,

a halogen atom or a hydrogen atom,
m represents 0, 1 or 2, and
n represents 0, 1 or 2;

In the formula (1-3a), compounds wherein $R^{1a}$ is a C1 to C6 alkyl group optionally having one or more halogen atoms;
In the formula (1-3a), compounds wherein $R^{1a}$ is a C1 to C6 alkyl group;
In the formula (1-3a), compounds wherein $R^{1a}$ is a C1 to C3 alkyl group;
In the formula (1-3a), compounds wherein $R^{1a}$ is a methyl group, an ethyl group, a propyl group, a cyclopropyl group or a cyclopropylmethyl group;
In the formula (1-3a), compounds wherein $R^{1a}$ is a methyl group, an ethyl group or a propyl group;
In the formula (1-3a), compounds wherein $R^{3a}$ is a C1 to C6 alkyl group optionally having one or more halogen atoms or a hydrogen atom;
In the formula (1-3a), compounds wherein $R^{3a}$ is a trifluoromethyl group, a halogen atom or a hydrogen atom;
In the formula (1-3a), compounds wherein $R^{1a}$ is an ethyl group, and $R^{3a}$ is a trifluoromethyl group or a hydrogen atom;
In the formula (1-3a), compounds wherein $R^{1a}$ is an ethyl group, and $R^{3a}$ is a trifluoromethyl group;
In the formula (1-3a), compounds wherein $R^{1a}$ is an ethyl group, and $R^{3a}$ is a hydrogen atom;
In the formula (1-3a), compounds wherein $G^a$ is a C1 to C3 perfluoroalkyl group;
In the formula (1-3a), compounds wherein $G^a$ is $OR^{11}$;
In the formula (1-3a), compounds wherein $G^a$ is $S(O)_mR^{11}$;
In the formula (1-3a), compounds wherein $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group;
in the formula (1-3a), compounds wherein $G^a$ is a trifluoromethyl group;
In the formula (1-3a), compounds wherein $G^a$ is a pentafluoroethyl group;
In the formula (1-3a), compounds wherein $G^a$ is a heptafluoropropyl group;
In the formula (1-3a), compounds wherein $G^a$ is a heptafluoroisopropyl group;
In the formula (1-3a), compounds wherein $G^a$ is a trifluoromethoxy group;
In the formula (1-3a), compounds wherein $G^a$ is a trifluoromethylsulfanyl group;
In the formula (1-3a), compounds wherein $G^a$ is a trifluoromethylsulfinyl group;
In the formula (1-3a), compounds wherein $G^a$ is a trifluoromethylsulfonyl group;
In the formula (1-3a), compounds wherein $R^{7a}$ is a C1 to C3 alkyl group optionally having one or more halogen atoms, $GR^{5a}$, $S(O)_mR^{5a}$, $NR^{5a}R^{10a}$, a halogen atom or a hydrogen atom;
In the formula (1-3a), compounds wherein $R^{7a}$ is a trifluoromethyl group, a methoxy group, an ethoxy group, a methylsulfanyl group, a methylsulfonyl group, an ethylsulfanyl group, an ethylsulfonyl group, a methylamino group, a dimethylamino group, a diethylamino group, a halogen atom or a hydrogen atom;
In the formula (1-3a), compounds wherein $R^{7a}$ is a trifluoromethyl group;
In the formula (1-3a), compounds wherein $R^{7a}$ is a halogen atom;
In the formula (1-3a), compounds wherein $R^{7a}$ is a fluorine atom;
In the formula (1-3a), compounds wherein $R^{7a}$ is a chlorine atom;
In the formula (1-3a), compounds wherein $R^{7a}$ is a bromine atom;
In the formula (1-3a), compounds wherein $R^{7a}$ is an iodine atom;
In the formula (1-3a), compounds wherein $R^{7a}$ is a hydrogen atom;
In the formula (1-3a), compounds wherein $R^{1a}$ is a C1 to C3 alkyl group, $R^{3a}$ is a C1 to C6 alkyl group optionally having one or more halogen atoms or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, and $R^{7a}$ is a trifluoromethyl group, a methoxy group, an ethoxy group, a methylsulfanyl group, a methylsulfonyl group, an ethylsulfanyl group, an ethylsulfonyl group, a methylamino group, a dimethylamino group, a diethylamino group, a halogen atom or a hydrogen atom;
In the formula (1-3a), compounds wherein $R^{1a}$ is an ethyl group, $R^{3a}$ is a trifluoromethyl group or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, and $R^{7a}$ is a hydrogen atom;
Compounds represented by formula (1-4),

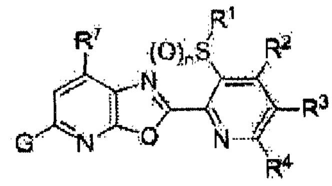

(1-4)

wherein symbols represent the same meaning as in the formula (1) ;
Compounds represented by formula (1-4a),

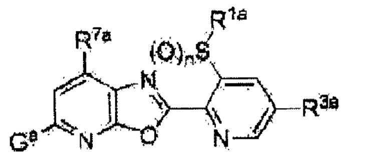

(1-4a)

wherein

$R^{1a}$ represents a C1 to C6 alkyl group optionally having one or more halogen atoms or one or more cyclopropyl groups (wherein the cyclopropyl group optionally has one or more halogen atoms or one or more C1 to C3 alkyl groups),
$R^{3a}$ represents a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^{5a}$, $S(O)_mR^{5a}$, a halogen atom or a hydrogen atom,
$R^{5a}$ represents a C1 to C6 alkyl group optionally having one or more halogen atoms,
$G^a$ represents a C1 to C3 perfluoroalkyl group, $OR^{11}$ or $S(O)_mR^{11}$,
$R^{7a}$ represents a C1 to C6 alkyl group optionally having one or more halogen atoms, $OR^{5a}$, $S(O)_mR^{5a}$, $NR^{5a}R^{10a}$, a halogen atom or a hydrogen atom,
m represents 0, 1 or 2, and n represents 0, 1 or 2; In the formula (1-4a), compounds wherein $R^{1a}$ is a C1 to C6 alkyl group optionally having one or more halogen atoms;

In the formula (1-4a), compounds wherein $R^{1a}$ is a C1 to C6 alkyl group;
In the formula (1-4a), compounds wherein $R^{1a}$ is a C1 to C3 alkyl group;
In the formula (1-4a), compounds wherein $R^{1a}$ is a methyl group, an ethyl group, a propyl group, a cyclopropyl group or a cyclopropylmethyl group;
In the formula (1-4a), compounds wherein $R^{1a}$ is a methyl group, an ethyl group or a propyl group;
In the formula (1-4a), compounds wherein $R^{3a}$ is a C1 to C6 alkyl group optionally having one or more halogen atoms or a hydrogen atom;
In the formula (1-4a), compounds wherein $R^{3a}$ is a trifluoromethyl group, a halogen atom or a hydrogen atom;
In the formula (1-4a), compounds wherein $R^{1a}$ is an ethyl group, and $R^{3a}$ is a trifluoromethyl group or a hydrogen atom;
In the formula (1-4a), compounds wherein $R^{1a}$ is an ethyl group, and $R^{3a}$ is a trifluoromethyl group;
In the formula (1-4a), compounds wherein $R^{1a}$ is an ethyl group, and $R^{3a}$ is a hydrogen atom;
In the formula (1-4a), compounds wherein $G^a$ is a C1 to C3 perfluoroalkyl group;
In the formula (1-4a), compounds wherein $G^a$ is $OR^{11}$;
In the formula (1-4a), compounds wherein $G^a$ is $S(O)_mR^{11}$;
In the formula (1-4a), compounds wherein $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group;
In the formula (1-4a), compounds wherein $G^a$ is a trifluoromethyl group;
In the formula (1-4a), compounds wherein $G^a$ is a pentafluoroethyl group;
In the formula (1-4a), compounds wherein $G^a$ is a heptafluoropropyl group;
In the formula (1-4a), compounds wherein $G^a$ is a heptafluoroisopropyl group;
In the formula (1-4a), compounds wherein $G^a$ is a trifluoromethoxy group;
In the formula (1-4a), compounds wherein $G^a$ is a trifluoromethylsulfanyl group;
In the formula (1-4a), compounds wherein $G^a$ is a trifluoromethylsulfinyl group;

In the formula (1-4a), compounds wherein $G^a$ is a trifluoromethylsulfonyl group;
In the formula (1-4a), compounds wherein $R^{7a}$ is a C1 to C3 alkyl group optionally having one or more halogen atoms, $OR^{5a}$, $S(O)_mR^{5a}$, $NR^{5a}R^{10a}$, a halogen atom or a hydrogen atom;
In the formula (1-4a), compounds wherein $R^{7a}$ is a trifluoromethoxy group, a methoxy group, an ethoxy group, a methylsulfanyl group, a methylsulfonyl group, an ethylsulfanyl group, an ethylsulfonyl group, a methylamino group, a dimethylamino group, a diethylamino group, a halogen atom or a hydrogen atom;
In the formula (1-4a), compounds wherein $R^{7a}$ is a trifluoromethyl group;
In the formula (1-4a), compounds wherein $R^{7a}$ is a halogen atom; In the formula (1-4a), compounds wherein $R^{7a}$ is a fluorine atom;
In the formula (1-4a), compounds wherein $R^{7a}$ is a chlorine atom;
In the formula (1-4a), compounds wherein $R^{7a}$ is a bromine atom;
In the formula (1-4a), compounds wherein $R^{7a}$ is an iodine atom;
In the formula (1-4a), compounds wherein $R^{7a}$ is a hydrogen atom;
In the formula (1-4a), compounds wherein $R^{1a}$ is a C1 to C3 alkyl group, $R^{3a}$ is a C1 to C6 alkyl group optionally having one or more halogen atoms, or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, and $R^{7a}$ is a halogen atom or a hydrogen atom;
In the formula (1-4a), compounds wherein $R^{1a}$ is an ethyl group, $R^{3a}$ is a trifluoromethyl group or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, and $R^{7a}$ is a hydrogen atom;
Compounds represented by formula (1-5),

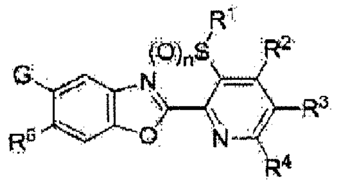
(1-5)

wherein symbols represent the same meaning as in the formula (1) ;
Compounds represented by formula (1-5a),

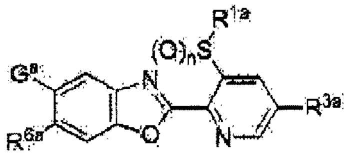
(1-5a)

wherein

$R^{1a}$ represents a C1 to C6 alkyl group optionally having one or more halogen atoms or one or more cyclopropyl groups (wherein the cyclopropyl group optionally has one or more halogen atoms or one or more C1 to C3 alkyl groups),
$R^{3a}$ represents a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^{5a}$, $S(O)_mR^{5a}$, a halogen atom or a hydrogen atom,
$R^{5a}$ represents a C1 to C6 alkyl group optionally having one or more halogen atoms,
$G^a$ represents a C1 to C3 perfluoroalkyl group, $OR^{11}$ or $S(O)_mR^{11}$,
$R^{6a}$ represents a C1 to C6 alkyl group optionally having one or more halogen atoms, $OR^{5a}$, $S(O)_mR^{5a}$, $NR^{5a}R^{10a}$ or a halogen atom,
m represents 0, 1 or 2, and
n represents 0, 1 or 2;

In the formula (1-5a), compounds wherein $R^{1a}$ is a C1 to C6 alkyl group optionally having one or more halogen atoms;

In the formula (1-5a), compounds wherein $R^{1a}$ is a C1 to C6 alkyl group;
In the formula (1-5a), compounds wherein $R^{1a}$ is a C1 to C3 alkyl group;
In the formula (1-5a), compounds wherein $R^{1a}$ is a methyl group, an ethyl group, a propyl group, a cyclopropyl group or a cyclopropylmethyl group;
In the formula (1-5a), compounds wherein $R^{1a}$ is a methyl group, an ethyl group or a propyl group;
In the formula (1-5a), compounds wherein $R^{3a}$ is a C1 to C6 alkyl group optionally having one or more halogen atoms or a hydrogen atom;
In the formula (1-5a), compounds wherein $R^{3a}$ is a trifluoromethyl group, a halogen atom or a hydrogen atom;
In the formula (1-5a), compounds wherein $R^{1a}$ is an ethyl group, and $R^{3a}$ is a trifluoromethyl group or a hydrogen atom;
In the formula (1-5a), compounds wherein $R^{1a}$ is an ethyl group, and $R^{3a}$ is a trifluoromethyl group;
In the formula (1-5a), compounds wherein $R^{1a}$ is an ethyl group, and $R^{3a}$ is a hydrogen atom;
In the formula (1-5a), compounds wherein $G^a$ is a C1 to C3 perfluoroalkyl group;
In the formula (1-5a), compounds wherein $G^a$ is $OR^{11}$;
In the formula (1-5a), compounds wherein $G^a$ is $S(O)_mR^{11}$;
In the formula (1-5a), compounds wherein $G^a$ is a trifluoromethyl group, a pentafluoroethyl group, a heptafluoropropyl group, a heptafluoroisopropyl group, a trifluoromethoxy group, a trifluoromethylsufanyl group, a trifluoromethylsulfinyl group or a trifluoromethylsulfonyl group;
In the formula (1-5a), compounds wherein $G^a$ is a trifluoromethyl group;
In the formula (1-5a), compounds wherein $G^a$ is a pentafluoroethyl group;
In the formula (1-5a), compounds wherein $G^a$ is a heptafluoropropyl group;
In the formula (1-5a), compounds wherein $G^a$ is a heptafluoroisopropyl group;
In the formula (1-5a), compounds wherein $G^a$ is a trifluoromethoxy group;
In the formula (1-5a), compounds wherein $G^a$ is a trifluoromethylsufanyl group;
In the formula (1-5a), compounds wherein $G^a$ is a trifluoromethylsulfinyl group;
In the formula (1-5a), compounds wherein $G^a$ is a trifluoromethylsulfonyl group;
In the formula (1-5a), compounds wherein $R^{6a}$ is $OR^{5a}$, $S(O)_mR^{5a}$, $NR^{5a}R^{10a}$ or a halogen atom;
In the formula (1-5a), compounds wherein $R^{6a}$ is a methoxy group, an ethoxy group, a methylsulfanyl group, a methylsulfonyl group, an ethylsulfanyl group, an ethylsulfonyl group, a methylamino group, a dimethylamino group, a dimethylamino group or a halogen atom;
In the formula (1-5a), compounds wherein $R^{6a}$ is a halogen atom;
In the formula (1-5a), compounds wherein $R^{6a}$ is a fluorine atom;
In the formula (1-5a), compounds wherein $R^{6a}$ is a chlorine atom;
In the formula (1-5a), compounds wherein $R^{6a}$ is a bromine atom;
In the formula (1-5a), compounds wherein $R^{6a}$ is an iodine atom;
In the formula (1-5a), compounds wherein $R^{1a}$ is a C1 to C3 alkyl group, $R^{3a}$ is a C1 to C6 alkyl group optionally having one or more halogen atoms, or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, and $R^{6a}$ is $OR^{5a}$, $S(O)_mR^{5a}$, $NR^{5a}R^{10a}$ or a halogen atom;
In the formula (1-5a), compounds wherein $R^{1a}$ is an ethyl group, $R^{3a}$ is a trifluoromethyl group or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, and $R^{6a}$ is a methoxy group, an ethoxy group, a methylsulfanyl group, a methylsulfonyl group, an ethylsulfanyl group, an ethylsulfonyl group, a methylamino group, a dimethylamino group, a dimethylamino group or a halogen atom; Compounds represented by formula (1-6),

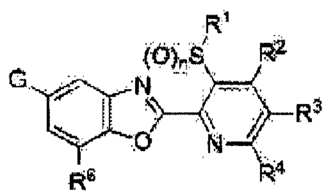
(1-6)

wherein symbols represent the same meaning as in the formula (1) ;
Compounds represented by formula (1-5a),

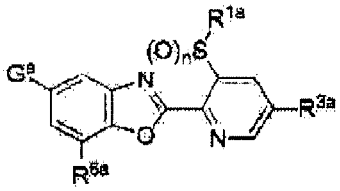

(1-6a)

wherein

$R^{1a}$ represents a C1 to C6 alkyl group optionally having one or more halogen atoms or one or more cyclopropyl groups (wherein the cyclopropyl group optionally has one or more halogen atoms or one or more C1 to C3 alkyl groups),
$R^{3a}$ represents a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^{5a}$, $S(O)_mR^{5a}$, a halogen atom or a hydrogen atom,
$P^{5a}$ represents a C1 to C6 alkyl group optionally having one or more halogen atoms,
$G^a$ represents a C1 to C3 perfluoroalkyl group, $OR^{11}$ or $S(O)_mR^{11}$,
$R^{6a}$ represents a C1 to C6 alkyl group optionally having one or more halogen atoms, $OR^{5a}$, $S(O)_mR^{5a}$, $NR^{5a}R^{10a}$ or a halogen atom,
m represents 0, 1 or 2, and
n represents 0, 1 or 2;

In the formula (1-6a), compounds wherein $R^{1a}$ is a C1 to C6 alkyl group optionally having one or more halogen atoms;
In the formula (1-6a), compounds wherein $R^{1a}$ is a C1 to C6 alkyl group;
In the formula (1-6a), compounds wherein $R^{1a}$ is a C1 to C3 alkyl group;
In the formula (1-6a), compounds wherein $R^{1a}$ is a methyl group, an ethyl group, a propyl group, a cyclopropyl group or a cyclopropylmethyl group;
In the formula (1-6a), compounds wherein $R^{1a}$ is a methyl group, an ethyl group or a propyl group;
In the formula (1-6a), compounds wherein $R^{3a}$ is a C1 to C6 alkyl group optionally having one or more halogen atoms or a hydrogen atom;
In the formula (1-6a), compounds wherein $R^{3a}$ is a trifluoromethyl group, a halogen atom or a hydrogen atom;
In the formula (1-6a), compounds wherein $R^{1a}$ is an ethyl group, and $R^{3a}$ is a trifluoromethyl group or a hydrogen atom;
In the formula (1-6a), compounds wherein $R^{1a}$ is an ethyl group, and $R^{3a}$ is a trifluoromethyl group;
In the formula (1-6a), compounds wherein $R^{1a}$ is an ethyl group, and $R^{3a}$ is a hydrogen atom;
In the formula (1-6a), compounds wherein $G^a$ is a C1 to C3 perfluoroalkyl group;
In the formula (1-6a), compounds wherein $G^a$ is $OR^{11}$;
In the formula (1-6a), compounds wherein $G^a$ is $S(O)_mR^{11}$;
In the formula (1-6a), compounds wherein $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group;
In the formula (1-6a), compounds wherein $G^a$ is a trifluoromethyl group;
In the formula (1-6a), compounds wherein $G^a$ is a pentafluoroethyl group;
In the formula (1-6a), compounds wherein $G^a$ is a heptafluoropropyl group;
In the formula (1-6a), compounds wherein $G^a$ is a heptafluoroisopropyl group;
In the formula (1-6a), compounds wherein $G^a$ is a trifluoromethoxy group;
In the formula (1-6a), compounds wherein $G^a$ is a trifluoromethylsulfanyl group;
In the formula (1-6a), compounds wherein $G^a$ is a trifluoromethylsufinyl group;
In the formula (1-6a), compounds wherein $G^a$ is a trifluoromethylsulfonyl group;
In the formula (1-6a), compounds wherein $R^{6a}$ is $OR^{5a}$, $S(O)_mR^{5a}$, $NR^{5a}R^{10a}$ of a halogen atom;
In the formula (1-6a), compounds wherein $R^{6a}$ is a methoxy group, an ethoxy group, a methylsulfanyl group, a methylsulfonyl group, an ethylsulfanyl group, an ethylsulfonyl group, a methylamino group, a dimethylamino group, a dimethylamino group or a halogen atom;
In the formula (1-6a), compounds wherein $R^{6a}$ is a halogen atom;
In the formula (1-6a), compounds wherein $R^{6a}$ is a fluorine atom;

In the formula (1-6a), compounds wherein $R^{6a}$ is a chlorine atom;
In the formula (1-6a), compounds wherein $R^{6a}$ is a bromine atom;
In the formula (1-6a), compounds wherein $R^{6a}$ is an iodine atom;
In the formula (1-6a), compounds wherein $R^{1a}$ is a C1 to C3 alkyl group, $R^{3a}$ is a C1 to C6 alkyl group optionally having one or more halogen atoms, or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, and $R^{6a}$ is $OR^{5a}$, $S(O)_mR^{5a}$, $NR^{5a}R^{10a}$ or a halogen atom;
In the formula (1-6a), compounds wherein $R^{1a}$ is an ethyl group, $R^{3a}$ is a trifluoromethyl group or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, and $R^{6a}$ is a methoxy group, an ethoxy group, a methylsulfanyl group, a methylsulfonyl group, an ethylsulfanyl group, an ethylsulfonyl group, a methylamino group, a dimethylamino group, a dimethylamino group or a halogen atom; Compounds represented by formula (1-7),

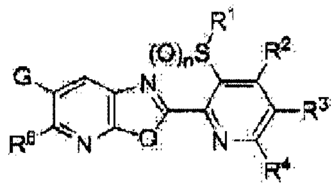
(1-7)

wherein symbols represent the same meaning as in the formula (1) ;
Compounds represented by formula (1-7a),

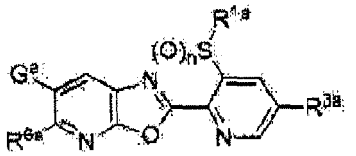
(1-7a)

wherein

$R^{1a}$ represents a C1 to C6 alkyl group optionally having one or more halogen atoms or one or more cyclopropyl groups (wherein the cyclopropyl group optionally has one or more halogen atoms or one or more C1 to C3 alkyl groups),
$R^{3a}$ represents a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^{5a}$, $S(O)_mR^{5a}$, a halogen atom or a hydrogen atom,
$R^{5a}$ represents a C1 to C6 alkyl group optionally having one or more halogen atoms,
$G^a$ represents a C1 to C3 perfluoroalkyl group, $OR^{11}$ or $S(O)_mR^{11}$,
$R^{6a}$ represents a C1 to C6 alkyl group optionally having one or more halogen atoms, $OR^{5a}$, $S(O)_mR^{5a}$, $NR^{5a}R^{10a}$ or a halogen atom,
m represents 0, 1 or 2, and
n represents 0, 1 or 2;

In the formula (1-7a), compounds wherein $R^{1a}$ is a C1 to C6 alkyl group optionally having one or more halogen atoms;
In the formula (1-7a), compounds wherein $R^{1a}$ is a C1 to C6 alkyl group;
In the formula (1-7a), compounds wherein $R^{1a}$ is a C1 to C3 alkyl group;
In the formula (1-7a), compounds wherein $R^{1a}$ is a methyl group, an ethyl group, a propyl group, a cyclopropyl group or a cyclopropylmethyl group;
In the formula (1-7a), compounds wherein $R^{1a}$ is a methyl group, an ethyl group or a propyl group;
In the formula (1-7a), compounds wherein $R^{3a}$ is a C1 to C6 alkyl group optionally having one or more halogen atoms or a hydrogen atom;
In the formula (1-7a), compounds wherein $R^{3a}$ is a trifluoromethyl group, a halogen atom or a hydrogen atom; +In the formula (1-7a), compounds wherein $R^{1a}$ is an ethyl group, and $R^{3a}$ is a trifluoromethyl group or a hydrogen atom;

In the formula (1-7a), compounds wherein $R^{1a}$ is an ethyl group, and $R^{3a}$ is a trifluoromethyl group;
In the formula (1-7a), compounds wherein $R^{1a}$ is an ethyl group, and $R^{3a}$ is a hydrogen atom;
In the formula (1-7a), compounds wherein $G^a$ is a C1 to C3 perfluoroalkyl group;
In the formula (1-7a), compounds wherein $G^a$ is $OR^{11}$;
In the formula (1-7a), compounds wherein $G^a$ is $S(O)_mR^{11}$;
In the formula (1-7a), compounds wherein $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group;
In the formula (1-7a), compounds wherein $G^a$ is a trifluoromethyl group;
In the formula (1-7a), compounds wherein $G^a$ is a pentafluoroethyl group;
In the formula (1-7a), compounds wherein $G^a$ is a heptafluoropropyl group;
In the formula (1-7a), compounds wherein $G^a$ is a heptafluoroisopropyl group;
In the formula (1-7a), compounds wherein $G^a$ is a trifluoromethoxy group;
In the formula (1-7a), compounds wherein $G^a$ is a trifluoromethylsulfanyl group;
In the formula (1-7a), compounds wherein $G^a$ is a trifluoromethylsulfinyl group;
In the formula (1-7a), compounds wherein $G^a$ is a trifluoromethylsulfonyl group;
In the formula (1-7a), compounds wherein $R^{6a}$ is $OR^{5a}$, $S(O)_mR^{5a}$, $NR^{5a}R^{10a}$ or a halogen atom;
In the formula (1-7a), compounds wherein $R^{6a}$ is a methoxy group, an ethoxy group, a methylsulfanyl group, a methylsulfonyl group, an ethylsulfanyl group, an ethylsulfonyl group, a methylamino group, a dimethylamino group, a dimethylamino group or a halogen atom;
In the formula (1-7a), compounds wherein $R^{6a}$ is a halogen atom;
In the formula (1-7a), compounds wherein $R^{6a}$ is a fluorine atom;
In the formula (1-7a), compounds wherein $R^{6a}$ is a chlorine atom;
In the formula (1-7a), compounds wherein $R^{6a}$ is a bromine atom;
In the formula (1-7a), compounds wherein $R^{6a}$ is an iodine atom,
In the formula (1-7a), compounds wherein $R^{1a}$ is a C1 to C3 alkyl group, $R^{3a}$ is a C1 to C6 alkyl group optionally having one or more halogen atoms, or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, and $R^{6a}$ is $OR^{5a}$, $S(O)_mR^{5a}$, $NR^{5a}R^{10a}$ or a halogen atom;
In the formula (1-7a), compounds wherein $R^{1a}$ is an ethyl group, $R^{3a}$ is a trifluoromethyl group or a hydrogen atom, $G^a$ is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, and $R^{6a}$ is a methoxy group, an ethoxy group, a methylsulfanyl group, a methylsulfonyl group, an ethylsulfanyl group, an ethylsulfonyl group, a methylamino group, a dimethylamino group, a dimethylamino group or a halogen atom;
In the compound of the present invention, examples of the N-oxide include the compounds represented by, for example, the following formulae (1A) to (1F).
Compounds represented by formula (1A)

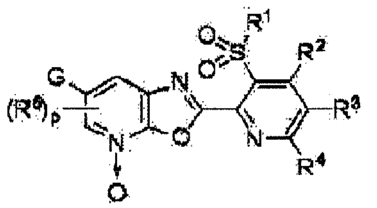 (1A)

wherein symbols represent the same meaning as in the formula (1);
In the formula (1A), compounds wherein

$R^1$ is a C1 to C6 alkyl group optionally having one or more halogen atoms or one or more cyclopropyl groups (wherein the cyclopropyl group optionally has one or more halogen atoms or one or more C1 to C3 alkyl groups),
both $R^2$ and $R^4$ are a hydrogen atom,
$R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a halogen atom or a hydrogen atom,

$R^5$ is a C1 to C6 alkyl group optionally having one or more halogen atoms,
G is a C1 to C3 perfluoroalkyl group, $OR^{11}$ or $S(O)_mR^{11}$ (wherein $R^{11}$ represents a C1 to C3 perfluoroalkyl group),
$R^6$ is a C1 to C6 alkyl group optionally having one or more halogen atoms, $OR^5$, $S(O)_mR^5$, $NR^5R^{10}$ or a halogen atom, and
p is 1;

In the formula (1A), compounds wherein $R^1$ is an ethyl group, $R^3$ is a trifluoromethyl group or a hydrogen atom, G is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, p is 1, and $R^6$ is a methoxy group, an ethoxy group, a methylsulfanyl group, a methylsulfonyl group, an ethylsulfanyl group, an ethylsulfonyl group, a methylamino group, a dimethylamino group, a dimethylamino group or a halogen atom; Compounds represented by formula (1B)

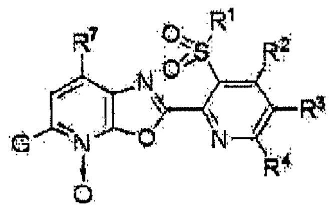
(1B)

wherein symbols represent the same meaning as in the formula (1);
In the formula (1B), compounds wherein

$R^1$ is a C1 to C6 alkyl group optionally having one or more halogen atoms or one or more cyclopropyl groups (wherein the cyclopropyl group optionally has one or more halogen atoms or one or more C1 to C3 alkyl groups),
both $R^2$ and $R^4$ are a hydrogen atom,
$R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a halogen atom or a hydrogen atom,
$R^5$ is a C1 to C6 alkyl group optionally having one or more halogen atoms,
G is a C1 to C3 perfluoroalkyl group, $OR^{11}$ or $S(O)_mR^{11}$ (wherein $R^{11}$ represents a C1 to C3 perfluoroalkyl group), and
$R^7$ is a C1 to C6 alkyl group optionally having one or more halogen atoms, $OR^5$, $S(O)_mR^5$, $NR^5R^{10}$, a halogen atom or a hydrogen atom;

In the formula (1B), compounds wherein $R^1$ is an ethyl group, $R^3$ is a trifluoromethyl group or a hydrogen atom, G is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, and $R^7$ is a hydrogen atom;
Compounds represented by formula (1C)

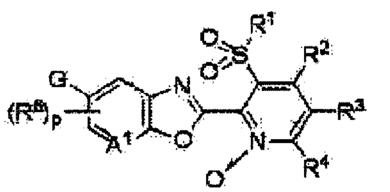
(1C)

wherein symbols represent the same meaning as in the formula (1);
In the formula (1C), compounds wherein $A^1$ is N;
In the formula (1C), compounds wherein $A^1$ is CH;
In the formula (1C), compounds wherein $A^1$ is $CR^6$;
In the formula (1C), compounds wherein $R^1$ is a C1 to C6 alkyl group optionally having one or more halogen atoms or one or more cyclopropyl groups (wherein the cyclopropyl group optionally has one or more halogen atoms or one or more C1 to C3 alkyl groups), both $R^2$ and $R^4$ are a hydrogen atom, $R^3$ is a C1 to C6 alkyl group optionally having one

or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a halogen atom or a hydrogen atom, $R^5$ is a C1 to C6 alkyl group optionally having one or more halogen atoms, $A^1$ is CH or $CR^6$, G is a C1 to C3 perfluoroalkyl group, $OR^{11}$ or $S(O)_mR^{11}$ (wherein $R^{11}$ represents a C1 to C3 perfluoroalkyl group), $R^6$ is a C1 to C6 alkyl group optionally having one or more halogen atom, $OR^5$, $S(O)_mR^5$, $NR^5R^{10}$ or a halogen atom, and p is 0 or 1 (wherein, when p is 0, $A^1$ is $CR^6$);

In the formula (1C), compounds wherein $R^1$ is an ethyl group, $R^3$ is a trifluoromethyl group or a hydrogen atom, G is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, p is 0 or 1 (wherein, when p is 0, $A^1$ is $CR^6$), $A^1$ is CH or $CR^6$, and $R^6$ is a methoxy group, an ethoxy group, a ethylsulfanyl group, a methylsulfonyl group, an ethylsulfanyl group, an ethylsulfonyl group, a methylamino group, a dimethylamino group, a diethylamino group or a halogen atom; Compounds represented by formula (1C-1)

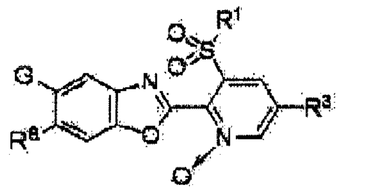
(1C-1)

wherein symbols represent the same meaning as in the formula (1);

Compounds represented by formula (1C-1a)

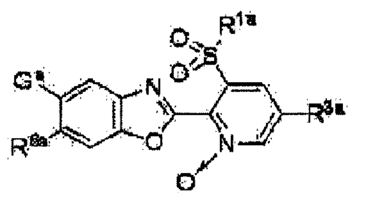
(1C-1a)

wherein symbols represent the same meaning as in the formula (1-5a);

Compounds represented by formula (1C-2)

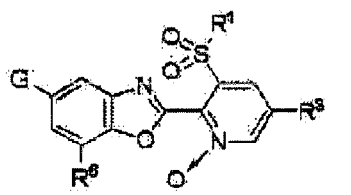
(1C-2)

wherein symbols represent the same meaning as in the formula (1);

Compounds represented by formula (1C-2a)

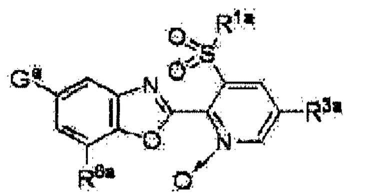
(1C-2a)

wherein symbols represent the same meaning as in the formula (1-6a);

Compounds represented by formula (1C-3)

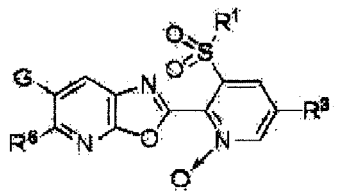

(1C-3)

wherein symbols represent the same meaning as in the formula (1);
Compounds represented by formula (1C-3a)

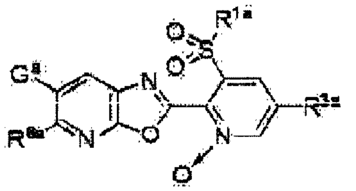

(1C-3a)

wherein symbols represent the same meaning as in the formula (1-7a);
Compounds represented by formula (1D)

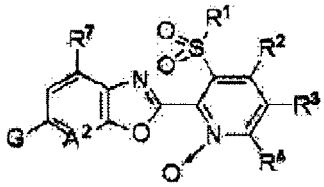

(1D)

wherein symbols represent the same meaning as in the formula (1);
In the formula (1D), compounds wherein $A^2$ is N;
In the formula (1D), compounds wherein $A^2$ is CH;
In the formula (1D), compounds wherein $A^2$ is $CR^8$;
In the formula (1D), compounds wherein

$R^1$ is a C1 to C6 alkyl group optionally having one or more halogen atoms or one or more cyclopropyl groups (wherein the cyclopropyl group optionally has one or more halogen atoms or one or more C1 to C3 alkyl groups),
both $R^2$ and $R^4$ are a hydrogen atom,
$R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a halogen atom or a hydrogen atom,
$R^5$ is a C1 to C6 alkyl group optionally having one or more halogen atoms,
$A^2$ is CH,
G is a C1 to C3 perfluoroalkyl group, $OR^{11}$ or $S(O)_mR^{11}$ (wherein $R^{11}$ represents a C1 to C3 perfluoroalkyl group), and
$R^7$ is a C1 to C6 alkyl group optionally having one or more halogen atoms, $OR^5$, $S(O)_mR^5$, $NR^5R^{10}$, a halogen atom or a hydrogen atom;

In the formula (1D), compounds wherein $R^1$ is an ethyl group, $R^3$ is a trifluoromethyl group or a hydrogen atom, G is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, $A^2$ is CH, and $R^7$ is a hydrogen atom;
Compounds represented by formula (1E)

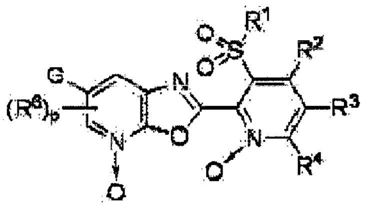

(1E)

wherein symbols represent the same meaning as in the formula (1);
In the formula (1E), compounds wherein $R^1$ is a C1 to C6 alkyl group optionally having one or more halogen atoms or one or more cyclopropyl groups (wherein the cyclopropyl group optionally has one or more halogen atoms or one or more C1 to C3 alkyl groups),

both $R^2$ and $R^4$ are a hydrogen atom, $R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a halogen atom or a hydrogen atom, $R^5$ is a C1 to C6 alkyl group optionally having one or more halogen atoms, G is a C1 to C3 perfluoroalkyl group, $OR^{11}$ or $S(O)_mR^{11}$ (wherein $R^{11}$ represents a C1 to C3 perfluoroalkyl group), $R^6$ is a C1 to C6 alkyl group optionally having one or more halogen atoms, $OR^5$, $S(O)_mR^5$, $NR^5R^{10}$ or a halogen atom, and p is 1; In the formula (1E), compounds wherein $R^1$ is an ethyl group, $R^3$ is a trifluoromethyl group or a hydrogen atom, G is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, p is 1, and $R^6$ is a methoxy group, an ethoxy group, a methylsulfanyl group, a methylsulfonyl group, an ethylsulfanyl group, an ethylsulfonyl group, a methylamino group, a dimethylamino group, a diethylamino group or a halogen atom; Compounds represented by formula (1F)

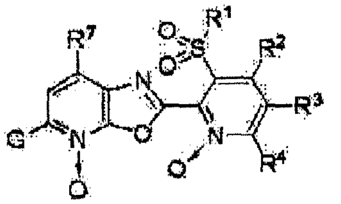

(1F)

wherein symbols represent the same meaning as in the formula (1);
In the formula (1F), compounds wherein

$R^1$ is a C1 to C6 alkyl group optionally having one or more halogen atoms or one or more cyclopropyl groups (wherein the cyclopropyl group optionally has one or more halogen atoms or one or more C1 to C3 alkyl groups),
both $R^2$ and $R^4$ are a hydrogen atom,
$R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a halogen atom or a hydrogen atom,
$R^5$ is a C1 to C6 alkyl group optionally having one or more halogen atoms,
G is a C1 to C3 perfluoroalkyl group, $OR^{11}$ or $S(C)_mR^{11}$ (wherein $R^{11}$ represents a C1 to C3 perfluoroalkyl group), and
$R^7$ is a C1 to C6 alkyl group optionally having one or more halogen atoms, $OR^5$, $S(O)_mR^5$, $NR^5R^{10}$, a halogen atom or a hydrogen atom;

In the formula (1F), compounds wherein $R^1$ is an ethyl group, $R^3$ is a trifluoromethyl group or a hydrogen atom, G is a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, and $R^7$ is a hydrogen atom.
Next, the method for producing the compound of the present invention will be described.

The compound of the present invention and the intermediate compound can be produced, for example, according to the following (Production Method 1) to (Production Method 8).

(Production Method 1)

The compound of the present invention in which n is 1 or 2 in the formula (1) can be produced by oxidizing the compound of the present invention in which n is 0.

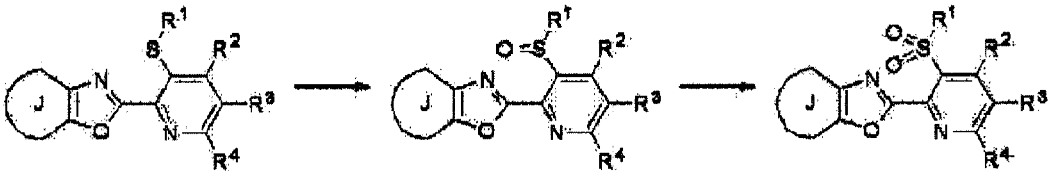

(1-n0) (1-n1) (1-n2)

In the formula, symbols represent the same meaning as in the formula (1).

The compound of the present invention (1-n1) in which n is 1 in the formula (1) can be produced by reacting the compound of the present invention (1-n0) in which n is 0 with an oxidizing agent. Examples of the oxidizing agent include sodium periodate and m-chloroperbenzoic acid.

The reaction is usually carried out in a solvent. Examples of the solvent include aliphatic halogenated hydrocarbons such as dichloromethane and chloroform, alcohols such as methanol and ethanol, acetic acid, water, and mixtures thereof.

In the reaction, the oxidizing agent is usually used in a ratio of 1 to 3 mol, based on 1 mol of the compound of the present invention (1-n0). Preferably, the oxidizing agent is used in a ratio of 1 to 1.2 mol, based on 1 mol of the compound of the present invention (1-n0).

The reaction temperature is usually within the range of -50 to 50°C, and the reaction time is usually within the range of 0.1 to 12 hours.

After completion of the reaction, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1-n1) can be isolated. The isolated compound of the present invention (1-n1) also can be further purified by chromatography, recrystallization, or the like.

The compound of the present invention (1-n2) in which n is 2 in the formula (1) can be produced by reacting the compound of the present invention (1-n1) in which n is 1 with an oxidizing agent. Examples of the oxidizing agent include m-chloroperbenzoic acid, aqueous hydrogen peroxide and oxone (registered trademark).

The reaction is usually carried out in a solvent. Examples of the solvent include aliphatic halogenated hydrocarbons such as dichloromethane and chloroform, alcohols such as methanol and ethanol, acetic acid, water, and mixtures thereof.

The reaction can be also carried out, in the presence of a catalyst, as necessary. Examples of the catalyst include tungstic acid, sodium tungstate and potassium tungstate.

In the reaction, the oxidizing agent is usually used in a ratio of 1 to 4 mol, and the catalyst is usually used in a ratio of 0.01 to 0.5 mol, based on 1 mol of the compound of the present invention (1-n1). Preferably, the oxidizing agent is used in a ratio of 1 to 2 mol, and the catalyst is used in a ratio of 0.05 to 0.2 mol, based on 1 mol of the compound of the present invention (1-n1).

The reaction temperature is usually within the range of -50 to 100°C. The reaction time is usually within the range of 0.1 to 12 hours.

After completion of the reaction, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1-n2) can be isolated. The compound of the present invention (1-n2) also can be further purified by chromatography, recrystallization, or the like.

Here, during the synthesis of the compound of the present invention (1-n2) in which n is 2, an N-oxide of the compound of the present invention is produced in some cases.

In addition, the compound of the present invention (1-n2) in which n is 2 in the formula (1) can be produced by a one-step reaction (one pot), by reacting the compound of the present invention (1-n0) in which n is C with an oxidizing agent. Examples of the oxidi zing agent include m-chloroperbenzoic acid, aqueous hydrogen peroxide and oxone (registered trademark).

The reaction is usually carried out in a solvent. Examples of the solvent include aliphatic halogenated hydrocarbons such as dichloromethane and chloroform, alcohols such as methanol and ethanol, acetic acid, water, and mixtures thereof.

The reaction can be also carried out, in the presence of a catalyst, as necessary. Examples of the catalyst include tungstic acid, sodium tungstate and potassium tungstate.

In the reaction, the oxidizing agent is usually used in a ratio of 2 to 5 mol, and the catalyst is usually used in a ratio of 0.01 to 0.5 mol, based on 1 mol of the compound of the present invention (1-n0). Preferably, the oxidizing agent is used in a ratio of 2 to 3 mol, and the catalyst is used in a ratio of 0.05 to 0.2 mol, based on 1 mol of the compound of the present invention (1-n0).

The reaction temperature is usually within the range of 0 to 120°C, and the reaction time is usually within the range of 0.1 to 12 hours.

After completion of the reaction, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1-n2) can be isolated. The isolated compound of the present invention (1-n2) also can be further purified by chromatography, recrystallization, or the like.

Here, during the synthesis of the compound of the present invention (1-n2) in which n is 2, an N-oxide of the compound of the present invention is produced in some cases.

(Production Method 2)

The compound of the present invention in which n is 2, G is $S(O)_mR^9$, and m is 1 or 2 in the formula (1) (the compound of the present invention in which G is $S(O)R^9$ or $S(O)_2R^9$) can be produced by oxidizing the compound of the present invention in which n is 2, G is $S(O)_mR^9$, and m is 0 (the compound of the present invention in which G is $SR^9$).

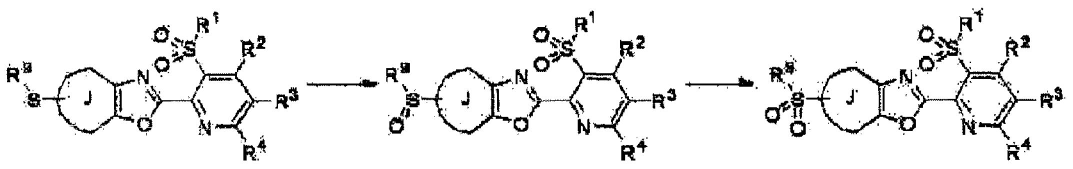

(1-n2m0) (1-n2m1) (1-n2m2)

In the formula, symbols represent the same meaning as in the formula (1).

The compound of the present invention (1-n2m1) in which n is 2, G is $S(O)_mR^9$, and m is 1 in the formula (1) can be produced by reacting the compound of the present invention (1-n2m0) in which n is 2, G is $S(O)_mR^9$, and m is O with an oxidizing agent. Examples of the oxidizing agent include sodium periodate, m-chloroperbenzoic acid, aqueous hydrogen peroxide and oxone (registered trademark).

The reaction is usually carried out in a solvent.

Examples of the solvent include aliphatic halogenated hydrocarbons such as dichloromethane and chloroform, alcohols such as methanol and ethanol, acetic acid, water, and mixtures thereof.

The reaction can be also carried out, in the presence of a catalyst, as necessary. Examples of the catalyst include tungstic acid, sodium tungstate and potassium tungstate.

In the reaction, the oxidizing agent is usually used in a ratio of 1 to 3 mol, and the catalyst is usually used in a ratio of 0.01 to 0.5 mol, based on 1 mol of the compound of the present invention (1-n2m0). Preferably, the oxidizing agent is used in a ratio of 1 to 1.2 mol, and the catalyst is used in a ratio of 0.05 to 0.2 mol, based on 1 mol of the compound of the present invention (1-n2m0).

The reaction temperature is usually within the range of -50 to 100°C. The reaction time is usually within the range of 0.1 to 12 hours.

After completion of the reaction, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1-n2m1) can be isolated. The isolated compound of the present invention (1-n2m1) also can be further purified by chromatography, recrystallization, or the like.

The compound of the present invention (1-n2m2) in which n is 2, G is $S(O)_mR^9$, and m is 2 in the formula (1) can be produced by reacting the compound of the present invention (1-n2m1) in which n is 2, G is $5(O)_mR^9$, and m is 1 with an oxidizing agent. Examples of the oxidizing agent include m-chloroperbenzoic acid, aqueous hydrogen peroxide and oxone (registered trademark).

The reaction is usually carried out in a solvent. Examples of the solvent include aliphatic halogenated hydrocarbons such as dichloromethane and chloroform, alcohols such as methanol and ethanol, acetic acid, water, and mixtures thereof.

The reaction can be also carried out, in the presence of a catalyst, as necessary. Examples of the catalyst include tungstic acid, sodium tungstate and potassium tungstate.

In the reaction, the oxidizing agent is usually used in a ratio of 1 to 4 mol, and the catalyst is usually used in a ratio of 0.01 to 0.5 mol, based on 1 mol of the compound of the present invention (1-n2m1). Preferably, the oxidizing agent is used in a ratio of 1 to 2 mol, and the catalyst is used in a ratio of 0.05 to 0.2 mol, based on 1 mol of the compound of the present invention (1-n2m1).

The reaction temperature is usually within the range of -50 to 100°C. The reaction time is usually within the range of 0.1 to 12 hours.

After completion of the reaction, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1-n2m2) can be isolated. The compound of the present invention (1-n2m2) also can be further purified by chromatography, recrystallization, or the like.

Here, during the synthesis of the compound of the present invention (1-n2m2), an N-oxide of the compound of the present invention is produced in some cases.

Also, the compound of the present invention (1-n2m2) in which n is 2, G is $S(O)_mR^9$, and m is 2 in the formula (1) can be produced by a one-step reaction (one pot), by reacting the compound of the present invention (1-n2m0) in which n is 2, G is $S(O)_mR^9$, and m is 0 with an oxidizing agent. Examples of the oxidizing agent include m-chloroperbenzoic acid, aqueous hydrogen peroxide and oxone (registered trademark).

The reaction is usually carried out in a solvent. Examples of the solvent include aliphatic halogenated hydrocarbons such as dichloromethane and chloroform, alcohols such as methanol and ethanol, acetic acid, water, and mixtures thereof.

The reaction can be also carried out, in the presence of a catalyst, as necessary. Examples of the catalyst include tungstic acid, sodium tungstate and potassium tungstate.

In the reaction, the oxidizing agent is usually used in a ratio of 2 to 5 mol, and the catalyst is usually used in a ratio of 0.01 to 0.5 mol, based on 1 mol of the compound of the present invention (1-n2m0). Preferably, the oxidizing agent is used in a ratio of 2 to 3 mol, and the catalyst is used in a ratio of 0.05 to 0.2 mol, based on 1 mol of the compound of the present invention (1-n2m0).

The reaction temperature is usually within the range of 0 to 120°C, and the reaction time is usually within the range of 0.1 to 12 hours.

After completion of the reaction, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1-n2m2) can be isolated. The isolated compound of the present invention (1-n2m2) also can be further purified by chromatography, recrystallization, or the like.

Here, during the synthesis of the compound of the present invention (1-n2m2), an N-oxide of the compound of the present invention is produced in some cases.

(Production Method 3)

The compound of the present invention in which n is 2, G is $S(O)_mR^9$, and m is 1 or 2 in the formula (1) (the compound of the present invention in which G is $S(O)R^9$ or $S(O)_2R^9$) can be produced by reacting the compound of the present invention in which n is 0, G is $S(O)_mR^9$, and m is 0 (the compound of the present invention in which G is $SR^9$) with an oxidizing agent.

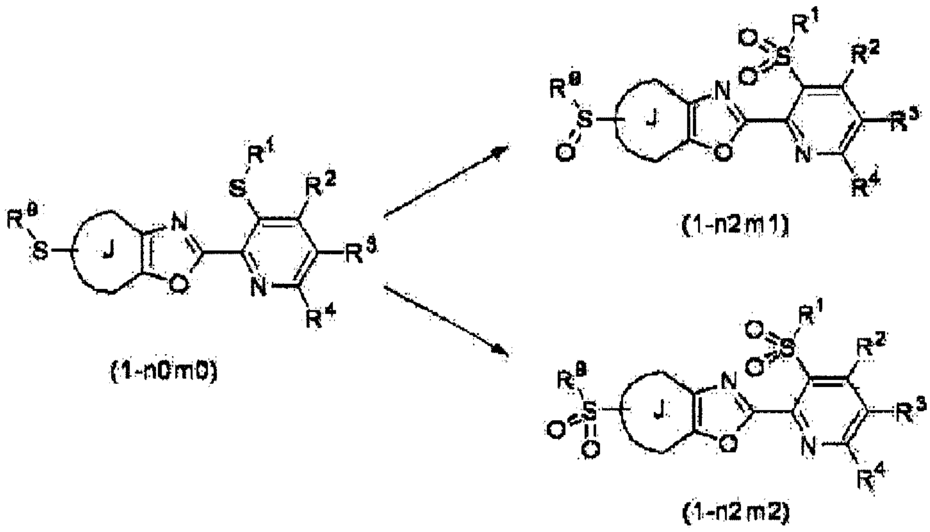

(1-n0m0) (1-n2m1) (1-n2m2)

In the formula symbols represent the same meaning as in the formula (1).

The compound of the present invention (1-n2m1) in which n is 2, G is S $(O)_mR^9$, and m is 1 in the formula (1) can be produced by a one-step reaction (one pot), by reacting the compound of the present invention (1-n0m0) in which n is 0, G is $S(O)_mR^9$, and m is 0 with an oxidizing agent. Examples of the oxidizing agent include m-chloroperbenzoic acid, aqueous hydrogen peroxide and oxone (registered trademark).

The reaction is usually carried out in a solvent. Examples of the solvent include aliphatic halogenated hydrocarbons such as dichloromethane and chloroform, alcohols such as methanol and ethanol, acetic acid, water, and mixtures thereof.

The reaction can be also carried out, in the presence of a catalyst, as necessary. Examples of the catalyst include tungstic acid, sodium tungstate and potassium tungstate.

In the reaction, the oxidizing agent is usually used in a ratio of 3 to 10 mol, and the catalyst is usually used in a ratio of 0.01 to 0.5 mol, based on 1 mol of the compound of the present invention (1-n0m0). Preferably, the oxidizing agent is used in a ratio of 3 to 5 mol, and the catalyst is used in a ratio of 0.05 to 0.2 mol, based on 1 mol of the compound of the present invention (1-n0m0).

The reaction temperature is usually within the range of 0 to 120°C, and the reaction tine is usually within the range of 0.1 to 12 hours.

After completion of the reaction, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1-n2m1) can be isolated. The isolated compound of the present invention (1-n2m1) also can be further purified by chromatography, recrystallization, or the like.

The compound of the present invention (1-n2m2) in which n is 2, G is $S(O)_mR^9$, and m is 2 in the formula (1) can be produced by a one-step reaction (one pot), by reacting the compound of the present invention (1-n0m0) in which n is 0, G is $S(O)_mR^9$, and m is 0 with an oxidizing agent. Examples of the oxidizing agent include m-chloroperbenzoic acid, aqueous hydrogen peroxide and oxone (registered trademark).

The reaction is usually carried out in a solvent. Examples of the solvent include aliphatic halogenated hydrocarbons such as dichloromethane and chloroform, alcohols such as methanol and ethanol, acetic acid, water, and mixtures thereof.

The reaction can be also carried out, in the presence of a catalyst, as necessary. Examples of the catalyst include tungstic acid, sodium tungstate and potassium tungstate.

In the reaction, the oxidizing agent is usually used in a ratio of 4 to 15 mol, and the catalyst is usually used in a ratio of 0.01 to 0.5 mol, based on 1 mol of the compound of the present invention (1-n0m0). Preferably, the oxidizing agent is used in a ratio of 4 to 5 mol, and the catalyst is used in a ratio of 0.05 to 0.2 mol, based on 1 mol of the compound of the present invention (1-n0m0).

The reaction temperature is usually within the range of 0 to 120°C, and the reaction time is usually within the range of 0.1 to 12 hours.

After completion of the reaction, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1-n2m2) can be isolated. The isolated

compound of the present invention (1-n2m2) also can be further purified by chromatography, recrystallization, or the like.

Here, during the synthesis of the compound of the present invention (1-n2m2), an N-oxide of the compound of the present invention is produced in some cases.

(Production Method 4-1)

The compound of the present invention (1A) can be produced by reacting the compound of the present invention (1-n2-J1-$A^1$=N) in which n is 2, the ring J is ring J1, and $A^1$ is N with an oxidizing agent, and the compound of the present invention (1B) can be produced by reacting the compound of the present invention (1-n2-J2-$A^2$=N) in which n is 2, the ring J is ring J2, and $A^2$ is N with an oxidizing agent.

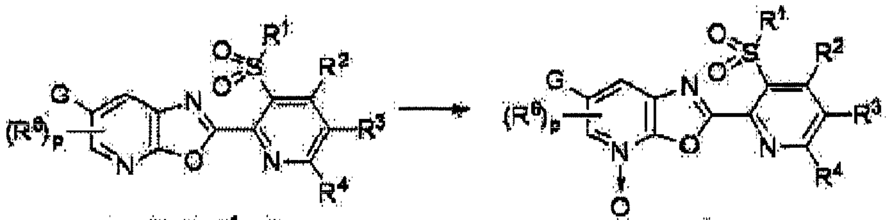

(1-n2-J1-$A^1$=N) (1A)

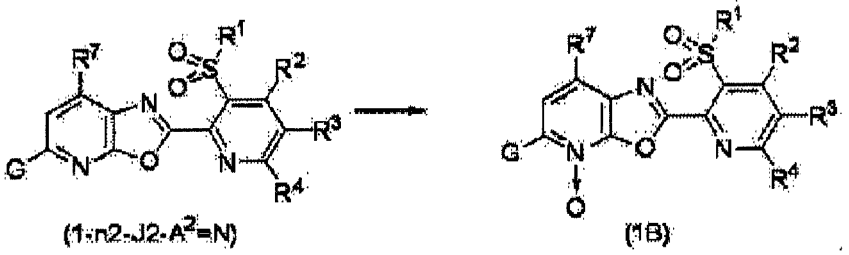

(1-n2-J2-$A^2$=N) (1B)

In the formula, p represents 1 or 2, and other symbols represent the same meaning as in the formula (1).

Examples of the oxidizing agent include m-chloroperbenzoic acid, aqueous hydrogen peroxide and oxone (registered trademark).

The reaction is usually carried out in a solvent. Examples of the solvent include aliphatic halogenated hydrocarbons such as dichloromethane and chloroform, alcohols such as methanol and athanol, acetic acid, water, and mixtures thereof.

The reaction can be also carried out, in the presence of a catalyst, as necessary. Examples of the catalyst include tungstic acid, sodium tungstate and potassium tungstate.

In the reaction, the oxidizing agent is usually used in a ratio of 1 to 10 mol, and the catalyst is usually used in a ratio of 0.01 to 0.5 mol, based on 1 mol of the compound of the present invention (1-n2-J1-$A^1$=N) or the compound of the present invention (1-n2-J2-$A^2$=N). Preferably, the oxidizing agent is used in a ratio of 2 to 5 mol, and the catalyst is used in a ratio of 0.05 to 0.2 mol, based on 1 mol of the compound of the present invention (1-n2-J1-$A^1$=N) or the compound of the present invention (1-n2-J2-$A^2$=N).

The reaction temperature is usually within the range of 20 to 120°C, and the reaction time is usually within the range of 0.1 to 48 hours.

After completion of the reaction, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1A) or the compound of the present invention (1B) can be isolated. The isolated compound of the present invention (1A) or compound of the present invention (1B) also can be further purified by chromatography, recrystallization, or the like.

Here, during the synthesis of the compound of the present invention (1A), the compound of the present invention wherein $A^1$ is N in the compound of the present invention (1C) and/or the compound of the present invention (1E) is simultaneously produced in some cases. Also, during the synthesis of the compound of the present invention (1B), the compound of the present invention wherein $A^2$ is N in the compound of the present invention (1D) and/or the compound of the present invention (1F) is simultaneously produced in some cases.

(Production Method 4-2)

The compound of the present invention (1C) can be produced by reacting the compound of the present invention (1-n2-J1) in which n is 2, and the ring J is ring J1, with an oxidizing agent, and the compound of the present invention (1D) an be produced by reacting the compound of the present invention (1-n2-J2) in which n is 2, and the ring J is ring J2, with an oxidizing agent.

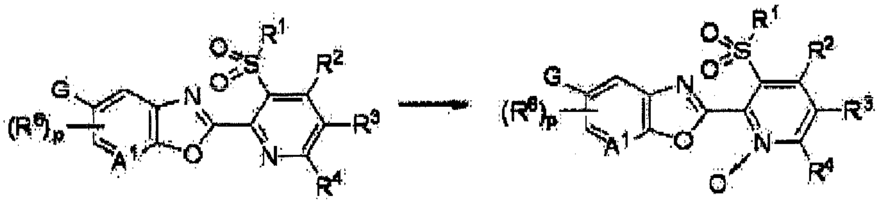

(1-n2-J1) (1C)

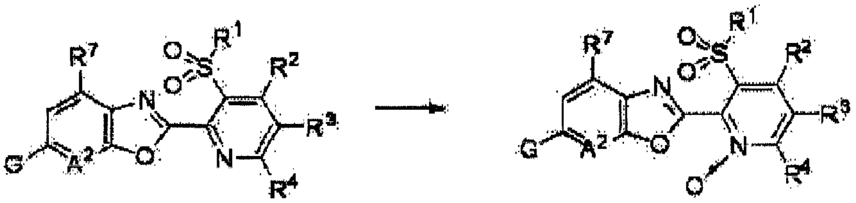

(1-n2-J2) (1D)

In the formula, symbols represent the same meaning as in the formula (1).

Examples of the oxidizing agent include m-chloroperbenzoic acid, aqueous hydrogen peroxide and oxone (registered trademark).

The reaction is usually carried out in a solvent. Examples of the solvent include aliphatic halogenated hydrocarbons such as dichloromethane and chloroform, alcohols such as methanol and ethanol, acetic acid, water, and mixtures thereof.

The reaction can be also carried out, in the presence of a catalyst, as necessary. Examples of the catalyst include tungstic acid, sodium tungstate and potassium tungstate.

In the reaction, the oxidizing agent is usually used in a ratio of 1 to 10 mol, and the catalyst is usually used in a ratio of 0.01 to 0.5 mol, based on 1 mol of the compound of the present invention (1-n2-J1) or the compound of the present invention (1-n2-J2). Preferably, the oxidizing agent is used in a ratio of 2 to 5 mol, and the catalyst is used in a ratio of 0.05 to 0.2 mol, based on 1 mol of the compound of the present invention (1-n2-J1) or the compound of the present invention (1-n2-J2).

The reaction temperature is usually within the range of 20 to 120°C, and the reaction time is usually within the range of 0.1 to 48 hours.

After completion of the reaction, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1C) or the compound of the present invention (1D) can be isolated. The isolated compound of the present invention (1C) or compound of the present invention (1D) also can be further purified by chromatography, recrystallization, or the like.

Here, during the synthesis of the compound of the present invention (1C), the compound of the present invention (1A) and/or the compound of the present invention (1E) is simultaneously produced in some cases. Also, during the synthesis of the compound of the present invention (1D), the compound of the present invention (1B) and/or the compound of the present invention (1F) is simultaneously produced in some cases.

(Production Method 4-3)

The compound of the present invention (1E) can be produced by reacting the compound of the present invention (1-n2-J1-$A^1$=N) in which n is 2, the ring J is ring J1, and $A^1$ is N with an oxidizing agent, and the compound of the present invention (1F) can be produced by reacting the compound of the present invention (1-n2-J2-$A^2$=N) in which n is 2, the ring J is ring J2, and $A^2$ is N with an oxidizing agent.

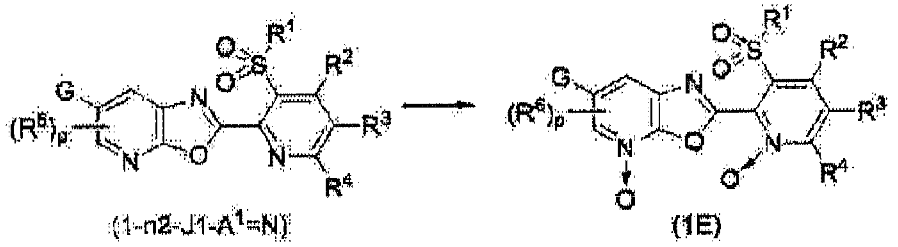

(1-n2-J1-$A^1$=N) (1E)

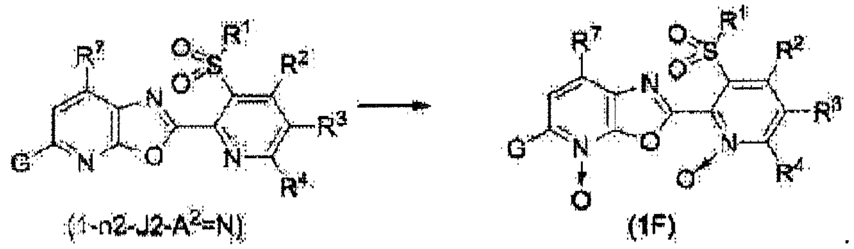

(1-n2-J2-$A^2$=N) (1F).

In the formula, symbols represent the same meaning as in the formula (1).

Examples of the oxidizing agent include m-chloroperbenzoic acid, aqueous hydrogen peroxide and oxone (registered trademark).

The reaction is usually carried out in a solvent. Examples of the solvent include aliphatic halogenated hydrocarbons such as dichloromethane and chloroform, alcohols such as methanol and ethanol, acetic acid, water, and mixtures thereof.

The reaction can be also carried out, in the presence of a catalyst, as necessary. Examples of the catalyst include tungstic acid, sodium tungstate and potassium tungstate.

In the reaction, the oxidizing agent is usually used in a ratio of 2 to 20 mol, and the catalyst is usually used in a ratio of 0.01 to 0.5 mol, based on 1 mol of the compound of the present invention (1-n2-J1-$A^1$=N) or the compound of the present invention (1-n2-J2-$A^2$=N), Preferably, the oxidizing agent is used in a ratio of 3 to 10 mol, and the catalyst is used in a ratio of 0.05 to 0.2 mol, based on 1 mol of the compound of the present invention (1-n2-J1-$A^1$=N) or the compound of the present invention (1-n2-J2-$A^2$=N).

The reaction temperature is usually within the range of 50 to 150°C, and the reaction time is usually within the range of 1 to 48 hours.

After completion of the reaction, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1E) or the compound of the present invention (1F) can be isolated. The isolated compound of the present invention (1E) or the compound of the present invention (1F) also can be further purified by chromatography, recrystallization, or the like.

Here, during the synthesis of the compound of the present invention (1E), the compound of the present invention (1A) and/or the compound of the present invention wherein $A^1$ is N in the compound of the present invention (1C) is simultaneously produced in some cases. Also, during the synthesis of the compound of the present invention (1F), the compound of the present invention (1B) and/or the compound of the present invention wherein $A^2$ is N in the compound of the present invention (1D) is simultaneously produced in some cases.

(Production Method 5-1)

The compound of the present invention can be produced by reacting intermediate compound (M1) with intermediate compound (M2) to produce intermediate compound (M3), and then intramolecularly condensing the obtained intermediate compound (M3), or produced by a one-step reaction (one pot), by reacting the intermediate compound (M1) with the intermediate compound (M2) in the presence of a condensing agent.

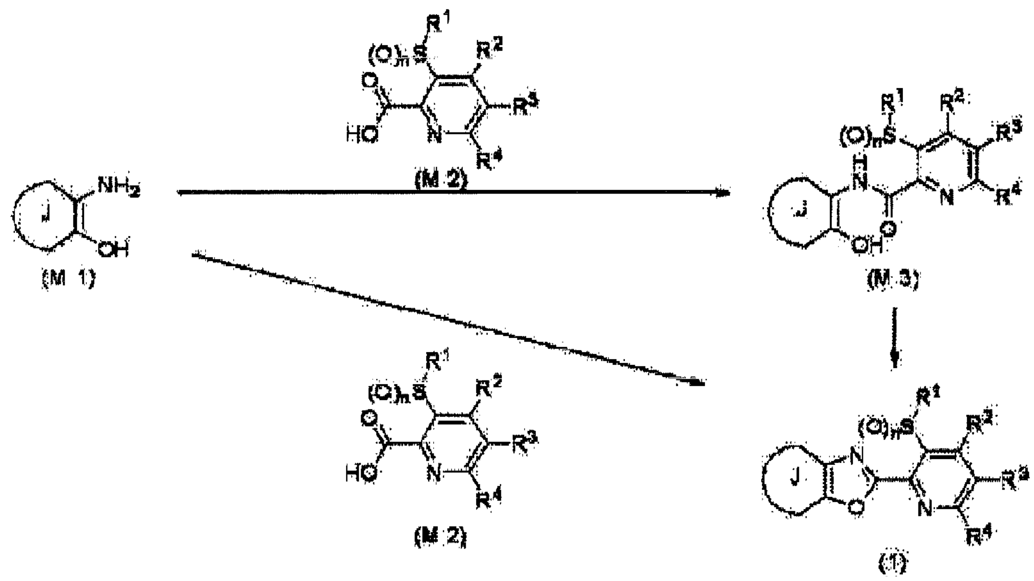

In the formula, symbols represent the same meaning as in the formula (1).

The intermediate compound (M3) can be produced by reacting the intermediate compound (M1) with the intermediate compound (M2), in the presence of a condensing agent. Examples of the condensing agent include carbodiimides such as 1-ethyl-3-(3-dimethylaminopropyl)carbodiimide hydrochloride (hereinafter, referred to as EDCI hydrochloride) and 1,3-dicyclohexylcarbodiimide.

The reaction is usually carried out in a solvent. Examples of the solvent include ethers such as 1,4-dioxane, diethyl ether, tetrahydrofuran (hereinafter, referred to as THF) and tert-butyl methyl ether, halogenated hydrocarbons such as dichloromethane, chloroform, carbon tetrachloride, 1,2-dichloroethane and chlorobenzene, aromatic hydrocarbons such as toluene, benzene and xylene, esters such as ethyl acetate and butyl acetate, nitriles such as acetonitrile, aprotic polar solvents such as N,N-dimethylformamide (hereinafter, referred to as DMF), N-methyl pyrrolidone (hereinafter, referred to as NMP), 1,3-dimethyl-2-imidazolidinone and dimethyl sulfoxide (hereinafter, referred to as DMSO), nitrogen-containing aromatic compounds such as pyridine and quinolone, and mixtures thereof.

The reaction can be also carried out by adding a catalyst, as necessary. Examples of the catalyst include 1-hydroxybenzotriazole (hereinafter, referred to as HOBt).

In the reaction, the intermediate compound (M2) is usually used in a ratio of 0.5 to 2 mol, the condensing agent is usually used in a ratio of 1 to 5 mol, and the catalyst is usually used in a ratio of 0.01 to 1 mol, based on 1 mol of the intermediate compound (M1).

The reaction temperature is usually within the range of 0 to 120°C, and the reaction time is usually within the range of 0.1 to 24 hours.

After completion of the reaction, the intermediate compound (M3) can be isolated by adding the reaction mixture to water, then extracting the mixture with an organic solvent, and concentrating the organic layer; collecting by filtration a solid generated by adding the reaction mixture to water; or collecting by filtration a solid generated in the reaction mixture. The isolated intermediate compound (M3) also can be further purified by recrystallization, chromatography, or the like.

The compound of the present invention (1) can be produced by intramolecular condensation of the intermediate compound (M3).

The reaction is usually carried out in a solvent. Examples of the solvent include ethers such as 1,4-dioxane, diethyl ether, THF and tert-butyl methyl ether, halogenated hydrocarbons such as dichloromethane, chloroform, carbon tetrachloride, 1,2-dichloroethane and chlorobenzene, aromatic hydrocarbons such as toluene, benzene and xylene, esters such as ethyl acetate and butyl acetate, nitriles such as acetonitrile, aprotic polar solvents such as DMF, NMP, 1,3-dimethyl-2-imidazolidinone and DMSO, nitrogen-containing aromatic compounds such as pyridine and quinoline, and mixtures thereof.

In the reaction, a condensing agent, an acid, a base or a chlorinating agent can be used, as necessary. Examples of the condensing agent include acetic anhydride, trifluoroacetic anhydride, EDCI hydrochloride, a mixture of triphenylphosphine, a base and carbon tetrachloride or carbon tetrabromide, a mixture of triphenylphosphine and azodiesters such as diethyl azodicarboxylate, and the like. Examples of the acid include sulfonic acids such as p-toluenesulfonic acid, carboxylic acids such as acetic acid, and polyphosphoric acid. Examples of the base include nitrogen-containing heterocyclic compounds such as pyridine, picoline, 2,6-lutidine, 1,8-diazabicyclo[5.4.0]-7-undecene (hereinafter, referred

to as DBU) and 1,5-diazabicyclo[4.3.0]-5-nonene, tertiary amines such as triethylamine and N,N'-diisopropylethylamine, and inorganic bases such as tripotassium phosphate, potassium carbonate and sodium hydride. Examples of the chlorinating agent include phosphorus oxychloride.

In the reaction, when a condensing agent is used, the condensing agent is usually used in a ratio of 1 to 5 mol, when an acid is used, the acid is usually used in a ratio of 0.1 to 5 mol, when a base is used, the base is usually used in a ratio of 1 to 5 mol, and when a chlorinating agent is used, the chlorinating agent is usually used in a ratio of 1 to 5 mol, based on 1 mol of the intermediate compound (M3).

The reaction temperature is usually within the range of 0 to 200°C, and the reaction time is usually within the range of 0.1 to 24 hours.

After completion of the reaction, the compound of the present invention (1) can be isolated by adding the reaction mixture to water, then extracting the mixture with an organic solvent, and concentrating the organic layer; collecting by filtration a solid generated by pouring the reaction mixture to water; or collecting by filtration a solid generated in the reaction mixture. The isolated compound of the present invention (1) also can be further purified by recrystallization, chromatography, or the like.

The compound of the present invention (1) can be produced by a one-step reaction (one pot) by reacting the intermediate compound (M1) with the intermediate compound (M2), in the presence of a condensing agent. Examples of the condensing agent include carbodiimides such as EDCI hydrochloride and 1,3-dicyclohexylcarbodiimide, and boric acid.

The reaction is usually carried out in a solvent. Examples of the solvent used in the reaction include ethers such as 1,4-dioxane, diethyl ether, THF and tert-butyl methyl ether, halogenated hydrocarbons such as dichloromethane, chloroform, carbon tetrachloride, 1,2-dichloroethane and chlorobenzene, aromatic hydrocarbons such as toluene, benzene and xylene, esters such as ethyl acetate and butyl acetate, nitriles such as acetonitrile, aprotic polar solvents such as DMF, NMP, 1,3-dimethyl-2-imidazolidinone and DMSO, nitrogen-containing aromatic compounds such as pyridine and quinoline, and mixtures thereof.

The reaction can be also carried out, in the presence of a catalyst, as necessary. Examples of the catalyst include 1-hydroxybenzotriazole.

In the reaction, the intermediate compound (M2) is usually used in a ratio of 0.5 to 2 mol, the condensing agent is usually used in a ratio of 1 to 5 mol, and the catalyst is usually used in a ratio of 0.01 to 1 mol, based on 1 mol of the intermediate compound (M1).

The reaction temperature is usually within the range of 0 to 200°C, and the reaction time is usually within the range of 0.1 to 24 hours.

After completion of the reaction, the compound of the present invention (1) can be isolated by adding the reaction mixture to water, then extracting the mixture with an organic solvent, and concentrating the organic layer; collecting by filtration a solid generated by pouring the reaction mixture to water; or collecting by filtration a solid generated in the reaction mixture. The isolated compound of the present invention (1) also can be further purified by recrystallization, chromatography, or the like.

(Production Method 5-2)

The compound of the present invention (1) can be produced by reacting the intermediate compound (M1) with intermediate compound (M4) to produce the intermediate compound (M3), and then intramolecularly condensing the obtained intermediate compound (M3), or produced by a one-step reaction (one pot), by reacting the intermediate compound (M1) with the intermediate compound (M4).

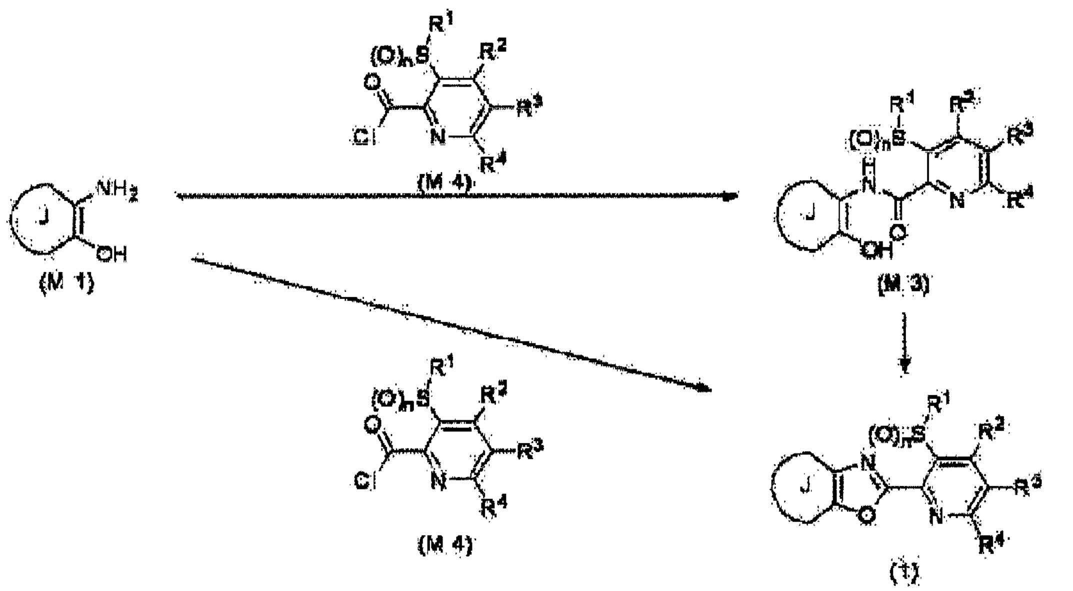

In the formula, symbols represent the same meaning as in the formula (1).

The intermediate compound (M3) can be produced by reacting the intermediate compound (M1) with the intermediate compound (M4).

When the solvent is used in the reaction, examples of the solvent include ethers such as THF, ethylene glycol dimethyl ether, tert-butyl methyl ether and 1,4-dioxane, aliphatic hydrocarbons such as hexane, heptane and octane, aromatic hydrocarbons such as toluene and xylene, halogenated hydrocarbons such as chlorobenzene, esters such as ethyl acetate and butyl acetate, nitriles such as acetonitrile, aprotic polar solvents such as DMF, NMP and DMSO, and mixtures thereof.

The reaction can be carried out, in the presence of a base, as necessary. Examples of the base include alkali metal carbonates such as sodium carbonate and potassium carbonate, tertiary amines such as triethylamine and N,N diisopropylethylamine, and nitrogen containing aromatic compounds such as pyridine and 4-dimethylaminopyridine.

In the reaction, the intermediate compound (M4) is usually used in a ratio of 1 to 3 mol, and the base is usually used in a ratio of 1 to 10 mol, based on 1 mol of the intermediate compound (M1).

The reaction temperature is usually within the range of -20 to 100°C. The reaction time is usually within the range of 0.1 to 24 hours.

After completion of the reaction, water is added to the reaction mixture, then the mixture is extracted with an organic solvent, and the organic layer is subjected to post-treatment operations such as drying and concentration, whereby the intermediate compound (M3) can be isolated. The isolated intermediate compound (M3) also can be further purified by chromatography, recrystallization, or the like.

The compound of the present invention (1) can be produced by intramolecular condensation of the intermediate compound (M3), according to the description of Production Method 5-1.

The compound of the present invention (1) can be produced by a one-step reaction (one pot) by reacting the intermediate compound (M1) with the intermediate compound (M4).

When the solvent is used in the reaction, examples of the solvent include ethers such as THF, ethylene glycol dimethyl ether, tert-butyl methyl ether and 1,4-dioxane, aliphatic hydrocarbons such as hexane, heptane and octane, aromatic hydrocarbons such as toluene and xylene, halogenated hydrocarbons such as chlorobenzene, esters such as ethyl acetate and butyl acetate, nitriles such as acetonitrile, aprotic polar solvents such as DMF, NMP and DMSO, and mixtures thereof.

The reaction can be carried out, in the presence of a base, as necessary. Examples of the base include alkali metal carbonates such as sodium carbonate and potassium carbonate, tertiary amines such as triethylamine and N,N-diisopropylethylamine, and nitrogen-containing aromatic compounds such as pyridine and 4-dimethylaminopyridine.

In the reaction, the intermediate compound (M4) is usually used in a ratio of 1 to 3 mol, and the base is usually used in a ratio of 1 to 10 mol, based on 1 mol of the intermediate compound (M1).

The reaction temperature is usually within the range of 20 to 200°C, and the reaction time is usually within the range of 0.1 to 24 hours.

After completion of the reaction, water is added to the reaction mixture, then the mixture is extracted with an organic solvent, and the organic layer is subjected to post-treatment operations such as drying and concentration, whereby the compound of the present invention (1) can be isolated. The isolated compound of the present invention (1)

also can be further purified by chromatography, recrystallization, or the like.

(Production Method 6)

The compound of the present invention (1) can be produced by reacting the intermediate compound (M1) with intermediate compound (M5), in the presence of an oxidizing agent.

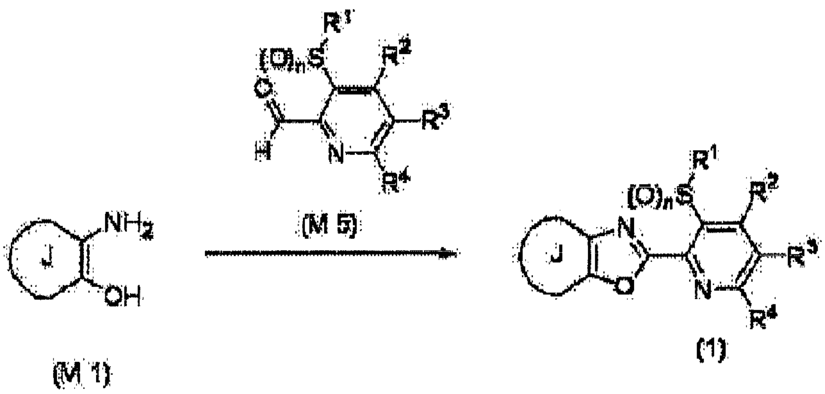

(M 5) (M 1) (1)

In the formula, symbols represent the same meaning as in the formula (1).

Examples of the oxidizing agent include oxygen, copper(II) chloride, and 2,3-dichloro-5,6-dicyano-p-benzoquinone (hereinafter, referred to as DDQ).

The reaction is usually carried out in a solvent. Examples of the solvent include alcohols such as methanol and ethanol, ethers such as 1,4-dioxane, diethyl ether, THF and tert-butyl methyl ether, halogenated hydrocarbons such as dichloromethane, chloroform, carbon tetrachloride, 1,2-dichloroethane and chlorobenzene, aromatic hydrocarbons such as toluene, benzene and xylene, esters such as ethyl acetate and butyl acetate, nitriles such as acetonitrile, aprotic polar solvents such as DMF, NMP, 1,3-dimethyl-2-imidazolidinone and DMSO, nitrogen-containing aromatic compounds such as pyridine and quinoline, and mixtures thereof.

The reaction can be carried out, in the presence of an acid, as necessary. Examples of the acid include sulfonic acids such as p-toluenesulfonic acid, carboxylic acids such as acetic acid, and polyphosphoric acid.

The reaction can be carried out, in the presence of a sulfite, as necessary. Examples of the sulfite include sulfites such as sodium bisulfite and sodium disulfite.

In the reaction, the intermediate compound (M5) is usually used in a ratio of 1 to 2 mol, the oxidizing agent is usually used in a ratio of 1 to 5 mol, the acid is usually used in a ratio of 0.1 to 2 mol, and the sulfite is usually used in a ratio of 1 to 5 mol, based on 1 mol of the intermediate compound [M1].

The reaction temperature is usually within the range of 0 to 200°C, and the reaction time is usually within the range of 0.1 to 24 hours.

After completion of the reaction, the compound of the present invention (1) can be isolated by adding the reaction mixture to water, then extracting the mixture with an organic solvent, and concentrating the organic layer; collecting by filtration a solid generated by pouring the reaction mixture to water; or collecting by filtration a solid generated in the reaction mixture. The isolated compound of the present invention (1) also can be further purified by recrystallization, chromatography, or the like.

(Production Method 7)

The compound of the present invention (1-n0) in which n is 0 in the formula (1) can be produced by reacting intermediate compound (M6) with compound (M7), in the presence of a base.

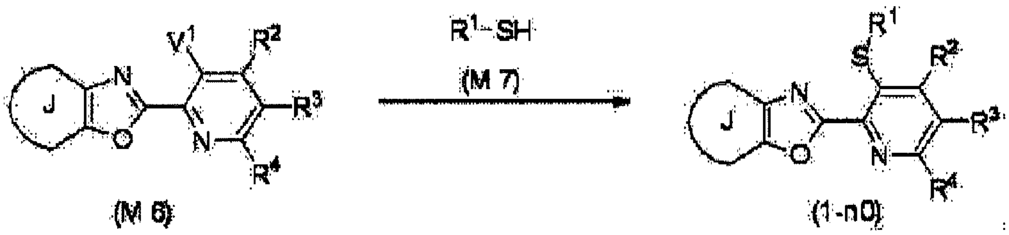


(M 6) (1-n0)

In the formula, $V^1$ represents a halogen atom, and other symbols represent the same meaning as in the formula (1).

The reaction is usually carried out in a solvent. Examples of the solvent include ethers such as THF, ethylene glycol dimethyl ether, tert-butyl methyl ether and 1, 4-dioxane, aromatic hydrocarbons such as toluene and xylene, nitriles such as acetonitrile, aprotic polar solvents such as DMF, NMP and DMSO, water, and mixtures thereof.

Examples of the base include alkali metal carbonates such as sodium carbonate and potassium carbonate, and alkali metal hydrides such as sodium hydride.

In the reaction, the compound (M7) is usually used in a ratio of 1 to 10 mol, and the base is usually used in a ratio of 1 to 10 mol, based on 1 mol of the intermediate compound (M6) .

The reaction temperature is usually within the range of -50 to 100°C. The reaction time is usually within the range of 0.1 to 12 hours.

After completion of the reaction, the reaction mixture is extracted with an organic solvent, and the organic layer is subjected to post-treatment operations such as drying and concentration, whereby the compound of the present invention (1-n0) in which n is 0 can be isolated. The isolated compound of the present invention (1-n0) in which n is 0 also can be further purified by chromatography, recrystallization, or the like.

$V^1$ is preferably a fluorine atom or a chlorine atom.

(Production Method 8-1)

The intermediate compound (M6) can be produced by reacting the intermediate compound (M1) with the intermediate compound (M8) to produce intermediate compound (M9), and then intramolecularly condensing the obtained intermediate compound (M9), or produced by a one-step reaction (one pot), by reacting the intermediate compound (M1) with the intermediate compound (M8).

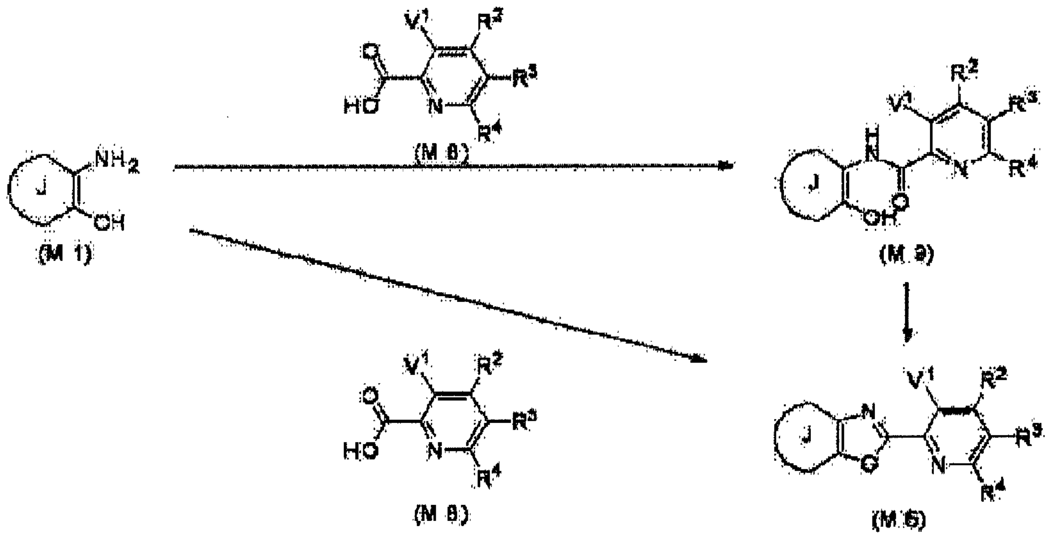

In the formula, $V^1$ represents a halogen atom, and other symbols represent the same meaning as in the formula (1).

The intermediate compound (M9) can be produced, using the intermediate compound (M8) in place of the intermediate compound (M2), in accordance with the method of Production Method 5-1.

The intermediate compound (M6) can be produced, using the intermediate compound (M9) in place of the intermediate compound (M3), in accordance with the method of Production Method 5-1.

Also, the intermediate compound (M6) can be produced by a one-step reaction (one pot), using the intermediate compound (M8) in place of the intermediate compound (M2), in accordance with the method of Production Method 5-1.

$V^1$ is preferably a fluorine atom or a chlorine atom.

(Production Method 8-2)

The intermediate compound (M6) can be produced by reacting the intermediate compound (M1) with intermediate compound (M10) to produce the intermediate compound (M9), and then intramolecularly condensing the obtained intermediate compound (M9), or produced by a one-step reaction (one pot), by reacting the intermediate compound (M1) with the intermediate compound (M10).

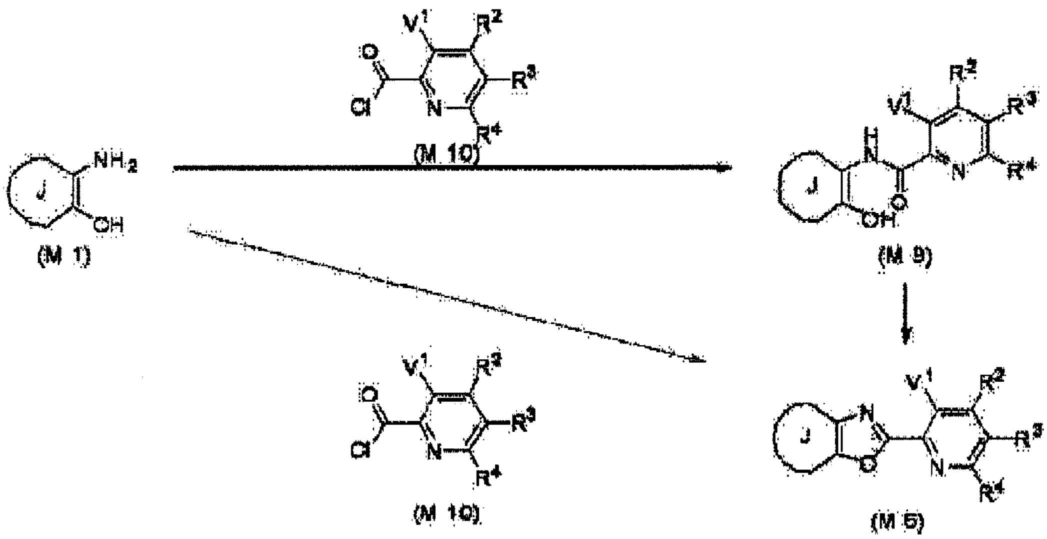

In the formula, $V^1$ represents a halogen atom, and other symbols represent the same meaning as in the formula (1).

The intermediate compound (M9) can be produced, using the intermediate compound (M10) in place of the intermediate compound (M4), in accordance with the method of Production Method 5-2.

The intermediate compound (M6) can be produced, using the intermediate compound (M9) in place of the intermediate compound (M3), in accordance with the method of Production

Method 5-1 or Production Method 5-2.

Also, the intermediate compound (M6) can be produced by a one-step reaction (one pot), using the intermediate compound (M10) in place of the intermediate compound (M4), in accordance with the method of Production Method 5-2.

$V^1$ is preferably a fluorine atom or a chlorine atom.

(Production Method 9)

The intermediate compound (M6) can be produced by reacting intermediate compound (M1) with the intermediate compound (M11).

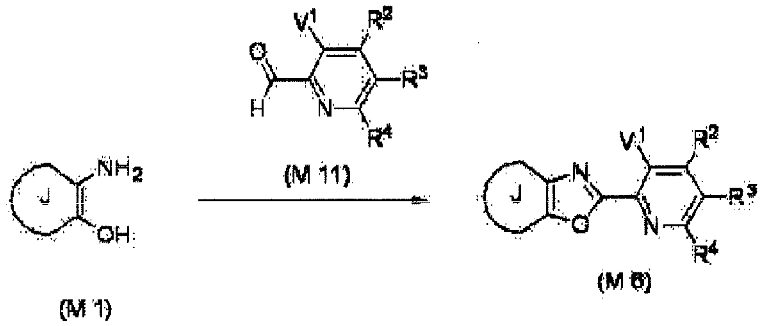

In the formula, symbols represent the same meaning as described above.

The intermediate compound (M6) can be produced, using the intermediate compound (M11) in place of the intermediate compound (M5), in accordance with the method of Production Method 6.

(Production Method 10)

The intermediate compound (M3-n0) in which n is 0 in the formula (M3) can be produced by reacting the intermediate compound (M9) with the compound (M7).

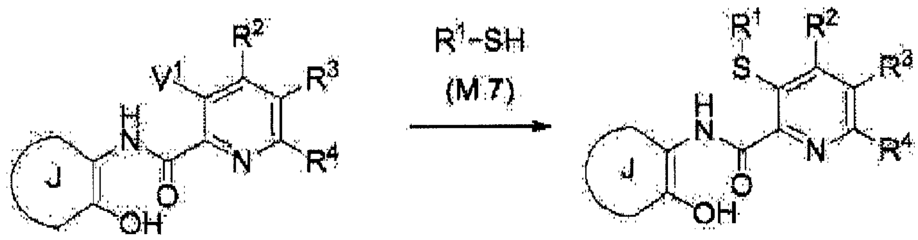


(M9) (M3-n0)

In the formula, symbols represent the same meaning as described above.

The intermediate compound (M3-n0) in which n is 0 can be produced, using the intermediate compound (M9) in place of the intermediate compound (M6), in accordance with the method of Production Method 7.

$V^1$ is preferably a fluorine atom or a chlorine atom.

(Production Method 11)

The intermediate compound (M2) can be produced by hydrolyzing intermediate compound (M12).

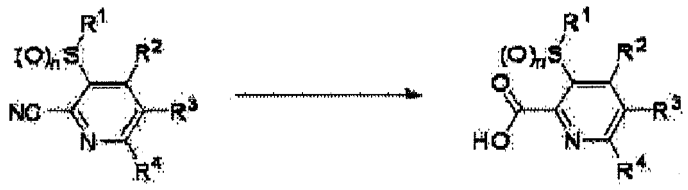

(M 12) (M 2)

In the formula, symbols represent the same meaning as in the formula (1).

When the hydrolysis reaction is carried out with an acid, an aqueous solution of the acid is usually used as a solvent.

Examples of the acid include mineral acids such as hydrochloric acid, nitric acid, phosphoric acid and sulfuric acid, and carboxylic acids such as acetic acid and trifluoroacetic acid.

In the reaction, the acid is usually used in a ratio of 1 mol or more, based on 1 mol, of the intermediate compound (M12).

The reaction temperature is usually within the range of 0 to 150°C, and the reaction time is usually within the range of 0.1 to 24 hours.

After completion of the reaction, the precipitated solid is filtered and collected, or the reaction mixture is extracted with an organic solvent, and the organic layer is subjected to post-treatment operations such as drying and concentration, whereby the intermediate compound (M2) can be isolated. The isolated intermediate compound (M2) also can be further purified by chromatography, recrystallization, or the like.

When the hydrolysis reaction is carried out with a base, the reaction is usually carried out in a solvent. Examples of the solvent include ethers such as THF, ethylene glycol dimethyl ether, tert-butyl methyl ether and 1,4-dioxane, alcohols such as methanol and ethanol, water, and mixtures thereof.

Examples of the base include alkali metal hydroxides such as sodium hydroxide and potassium hydroxide.

In the reaction, the base is usually used in a ratio of 1 to 10 mol, based on 1 mol of the intermediate compound (M12) .

The reaction temperature is usually within the range of 0 to 150°C, and the reaction time is usually within the range of 0.1 to 24 hours.

After completion of the reaction, the reaction liquid is acidified, then the reaction mixture is extracted with an organic solvent, and the organic layer is subjected to post-treatment operations such as drying and concentration, whereby the intermediate compound (M2) can be isolated. The isolated intermediate compound (M2) also can be further purified by chromatography, recrystallization, or the like.

(Production Method 12)

The intermediate compound (M4) can be produced by reacting the intermediate compound (M2) with a chlorinating agent.

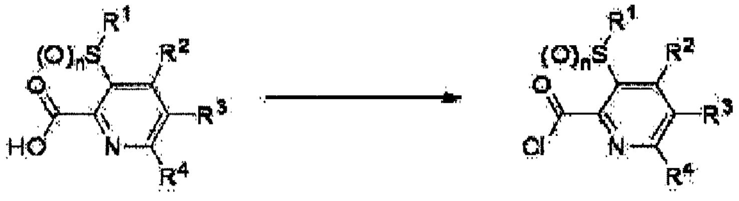

(M 2) (M 4)

In the formula, symbols represent the same meaning as in the formula (1).

Examples of the chlorinating agent include thionyl chloride, oxalyl dichloride and phosphorus oxychloride.

The reaction is usually carried out in a solvent. Examples of the solvent include ethers such as THF, ethylene glycol dimethyl ether, tert-butyl methyl ether and 1, 4-dioxane, aromatic hydrocarbons such as toluene and xylene, aliphatic halogenated hydrocarbons such as dichloromethane and chloroform, and mixtures thereof.

In the reaction, the chlorinating agent is usually used in a ratio of 1 to 5 mol, based on 1 mol of the intermediate compound (M2).

The reaction temperature is usually within the range of 0 to 100°C, and the reaction time is usually within the range of 0.1 to 24 hours.

After completion of the reaction, the intermediate compound (M4) can be isolated by distilling the solvent.

(Production Method 13)

The intermediate compound (M2), the intermediate compound (M5) or the intermediate compound (M12) can be produced by reacting the intermediate compound (M8), the intermediate compound (M11) or intermediate compound (M13), with the compound (M7), respectively, and oxidizing the obtained compound as necessary.

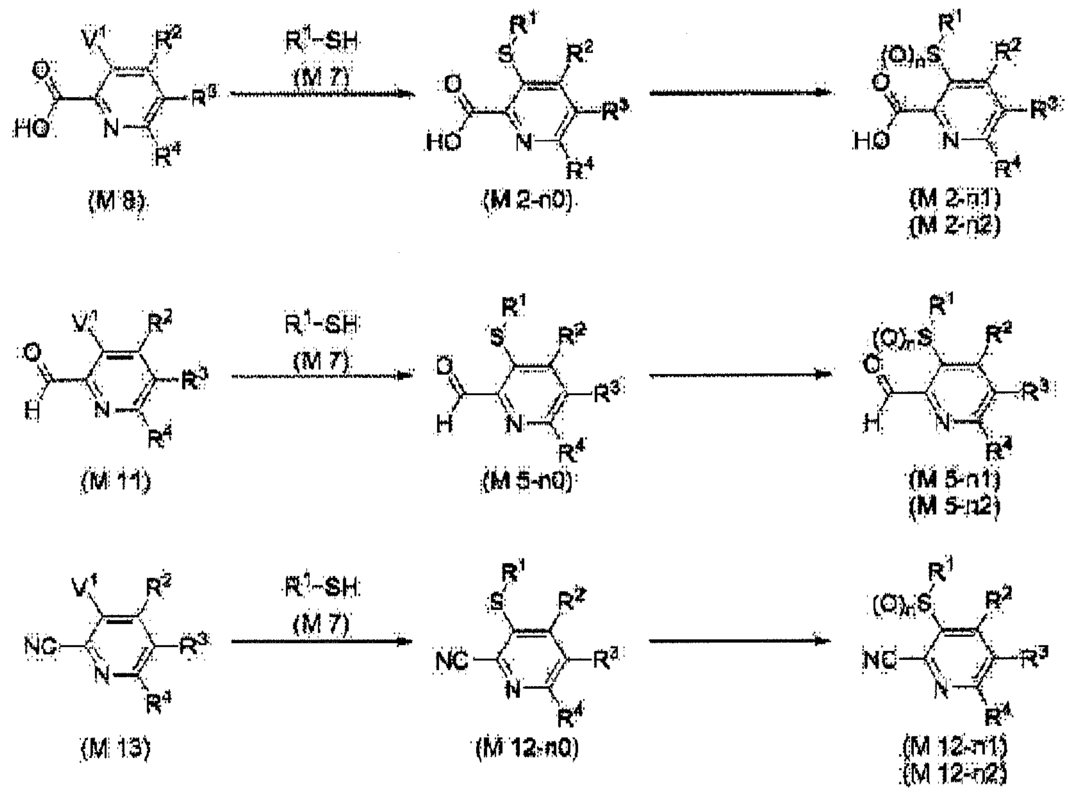


(M 8) (M 2-n0) (M 2-n1) (M 2-n2)

(M 11) (M 5-n0) (M 5-n1) (M 5-n2)

(M 13) (M 12-n0) (M 12-n1) (M 12-n2)

In the formula, symbols represent the same meaning as described above.

The intermediate compound (M2-n0) in which n is 0 can be produced, using the intermediate compound (M8) in place of the intermediate compound (M6), in accordance with the method of Production Method 7.

The intermediate compound (M5-n0) in which n is 0 can be produced, using the intermediate compound (M11) in place of the intermediate compound (M6), in accordance with the method of Production Method 7.

The intermediate compound (M12-n0) in which n is 0 can be produced, using the intermediate compound (M13) in place of the intermediate compound (M6), in accordance with the method of Production Method 7.

The intermediate compound (M2-n1) or (M2-n2) in which n is 1 or 2 can be produced, using the intermediate compound (M2-n0) in which n is 0 in place of the compound of the present invention (1-n0) in which n is 0, in accordance with the method of Production Method 1.

The intermediate compound (M5-n1) or (M5-n2) in which n is 1 or 2 can be produced, using the intermediate compound (M5-n0) in which n is 0 in place of the compound of the present invention (1-n0) in which n is 0, in accordance with the method of Production Method 1.

The intermediate compound (M12-n1) or (M12-n2) in which n is 1 or 2 can be produced, using the intermediate compound (M12-n0) in which n is 0 in place of the compound of the present invention (1-n0) in which n is 0, in accordance with the method of Production Method 1.

$V^1$ is preferably a fluorine atom or a chlorine atom.

(Production Method 14)

The intermediate compound (M1) can be produced by nitrating intermediate compound (M14) to produce intermediate compound (M15), then reducing the obtained intermediate compound (M15).

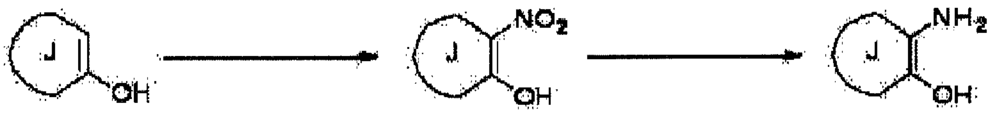

(M 14) (M 15) (M 1)

In the formula, symbols represent the same meaning as in the formula (1).

The intermediate compound (M15) can be produced by reacting the intermediate compound (M14) with a nitrating agent. Examples of the nitrating agent include concentrated nitric acid.

The reaction is usually carried out in a solvent. Examples of the solvent include aliphatic halogenated hydrocarbons such as dichloromethane and chloroform, acetic acid, concentrated sulfuric acid, concentrated nitric acid, water, and mixtures thereof.

In the reaction, the nitrating agent is usually used in a ratio of 1 to 3 mol, based on 1 mol of the intermediate compound (M14).

The reaction temperature is usually within the range of -10 to 100°C. The reaction time is usually within the range of 0.1 to 24 hours.

After completion of the reaction, the reaction mixture is added to water and extracted with an organic solvent, and the organic layer is subjected to post-treatment operations such as drying and concentration, whereby the intermediate compound (M15) can be isolated. The isolated intermediate compound (M15) also can be further purified by chromatography, recrystallization, or the like.

The intermediate compound (M1) can be produced by reacting the intermediate compound (M15) with hydrogen, in the presence of a hydrogenation catalyst. Examples of the hydrogenation catalyst include transition metal compounds such as palladium carbon, palladium hydroxide, Raney nickel, and platinum oxide.

The reaction is usually carried out in a solvent, usually in a hydrogen atmosphere of 1 to 100 atmospheric pressure. Examples of the solvent include ethers such as THF, ethylene glycol dimethyl ether, tert-butyl methyl ether and 1, 4-dioxane, esters such as ethyl acetate and butyl acetate, alcohols such as methanol and ethanol, water, and mixtures thereof.

In the reaction, the hydrogen is usually used in a ratio of 3 mol, and the hydrogenation catalyst is usually used in a ratio of 0.001 to 0.5 mol, based on 1 mol of the intermediate compound (M15).

The reaction temperature is usually within the range of -20 to 100°C. The reaction time is usually within the range of 0.1 to 24 hours.

After completion of the reaction, the reaction mixture is filtered, and extracted with an organic solvent as necessary, and the organic layer is subjected to post-treatment operations such as drying and concentration, whereby the intermediate compound (M1) can be isolated. The isolated intermediate compound (M1) also can be further purified

by chromatography, recrystallization, or the like.

Also, the intermediate compound (M1) can be produced by reacting the intermediate compound (M15) with a reducing agent. Examples of the reducing agent include metal powder such as iron powder, zinc powder, and tin dichloride.

The reaction can be carried out, for example, in the presence of a reducing agent, an acid such as hydrochloric acid and acetic acid, and water.

The reaction is usually carried out in a solvent. Examples of the solvent include ethers such as THF, ethylene glycol dimethyl ether, tert-butyl methyl ether and 1, 4-dioxane, esters such as ethyl acetate and butyl acetate, alcohols such as methanol and ethanol, aprotic polar solvents such as DMF, NMP and DMSO, and mixtures thereof.

In the reaction, the reducing agent is usually used in a ratio of 3 to 10 mol, the acid is usually used in a ratio of 0.01 to 0.5 mol, and water is usually used in a ratio of 3 mol or more, based on 1 mol of the intermediate compound (M15).

The reaction temperature is usually within the range of 0 to 100°C, and the reaction time is usually within the range of 0.1 to 24 hours.

After completion of the reaction, water is added to the reaction mixture, then the mixture is extracted with an organic solvent, and the organic layer is subjected to post-treatment operations such as drying and concentration, whereby the intermediate compound (M1) can be isolated. The isolated intermediate compound (M1) also can be further purified by chromatography, recrystallization, or the like.

(Production Method 15)

Intermediate compound (M17) in which G is SH in the formula (1) can be produced by reacting intermediate compound (M16) in which G is $V^1$ with a sulfurizing agent, in the presence of a catalyst. In addition, intermediate compound (M18) that is a disulfide body thereof can be produced by oxidizing two molecules of the intermediate compound (M17).

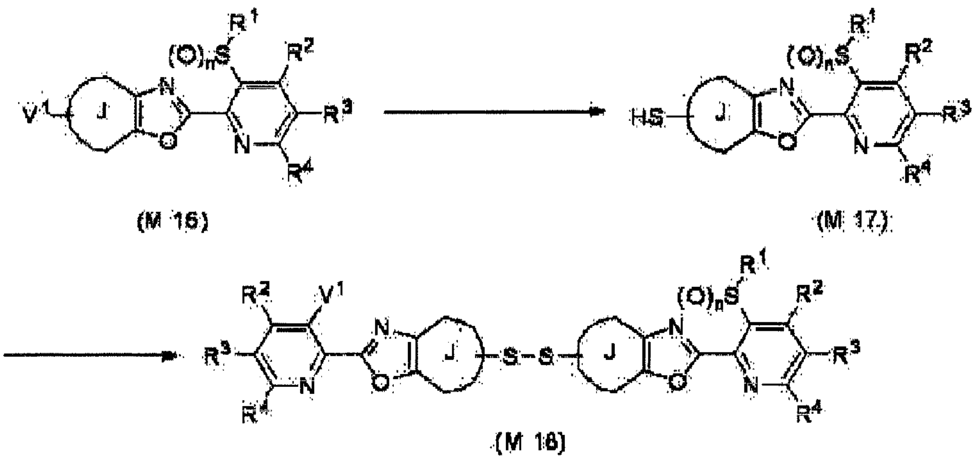

(M 16)

(M 17)

(M 18)

In the formula, symbols represent the same meaning as described above.

Examples of the sulfurizing agent include sodium sulfide, sodium sulfide nonahydrate and thiourea, and examples of the catalyst include copper(I) chloride, copper(I) bromide and copper(I) iodide.

The reaction is usually carried out in a solvent. Examples of the solvent include aromatic hydrocarbons such as toluene and xylene, aprotic polar solvents such as DMF, NMP and DMSO, and mixtures thereof.

The reaction can be also carried out by adding a ligand as necessary. Examples of the ligand include acetylacetone, salen, phenanthroline, and the like.

The reaction can be also carried out, in the presence of a base, as necessary. Examples of the base include inorganic bases such as potassium carbonate, cesium carbonate and tripotassium phosphate, and organic bases such as triethylamine.

In the reaction, the sulfurizing agent is usually used in a ratio of 1 to 10 mol, the catalyst is usually used in a ratio of 0.1 to 5 mol, the ligand is usually used in a ratio of 0.1 to 5 mol, and the base is usually used in a ratio of 1 to 2 mol, based on 1 mol of the intermediate compound (M16).

The reaction temperature is usually within the range of 50 to 200°C, and the reaction time is usually within the range of 0.5 to 24 hours.

After completion of the reaction, the reaction mixture is extracted with an organic solvent, and the organic layer is subjected to post-treatment operations such as drying and concentration, whereby the intermediate compound (M17) can be isolated. The isolated intermediate compound (M17) also can be further purified by chromatography, recrystallization, or the like.

In the reaction, $V^1$ is preferably a bromine atom and an iodide atom. Here, the reaction from the intermediate compound (M17) to the intermediate compound (M18) is likely to occur, and the intermediate compound (M18) is sometimes produced during the synthesis of the intermediate compound (M17).

The intermediate compound (M18) can be produced by reacting two molecules of the intermediate compound (M17) with an oxidizing agent. Examples of the oxidizing agent include oxygen, iodine, aqueous hydrogen peroxide, and potassium ferricyanide.

The reaction is usually carried out in a solvent. Examples of the solvent include water, alcohols such as methanol and ethanol, ethers such as THF, ethylene glycol dimethyl ether, tert-butyl methyl ether and 1,4-dioxane, aromatic hydrocarbons such as toluene and xylene, nitriles such as acetonitrile, aprotic polar solvents such as DMF, NMP and DMSO, carboxylic acids such as acetic acid, and mixtures thereof.

In the reaction, the oxidizing agent is usually used in a ratio of 0.5 to 10 mol, based on 1 mol of the intermediate compound (M17).

The reaction temperature is usually within the range of 0 to 100°C, and the reaction time is usually within the range of 0.1 to 24 hours.

After completion of the reaction, the reaction mixture is extracted with an organic solvent, and the organic layer is subjected to post-treatment operations such as drying and concentration, whereby the intermediate compound (M18) can be isolated. The isolated intermediate compound (M18) also can be further purified by chromatography, recrystallization, or the like.

(Production Method 16)

The intermediate compound (M17) in which G is SH in the formula (1) can be produced by reacting the intermediate compound (M16) in which G is $V^1$ with thiobenzoic acid to produce intermediate compound (M19), and then hydrolyzing the obtained intermediate compound (M19).

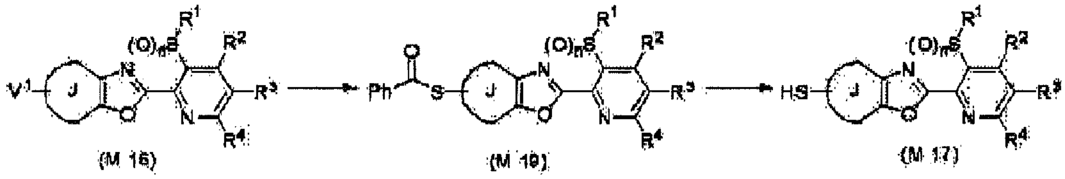

In the formula, symbols represent the same meaning as described above.

The intermediate compound (M19) can be produced by reacting the intermediate compound (M16) with thiobenzoic acid, in the presence of a base and a catalyst. Examples of the base include inorganic bases such as potassium carbonate, cesium carbonate and tripotassium phosphate, and organic bases such as triethylamine, and examples of the catalyst include copper(I) chloride, copper(I) bromide and copper(I) iodide.

The reaction is usually carried out in a solvent. Examples of the solvent include aromatic hydrocarbons such as toluene and xylene, aprotic polar solvents such as DMF, NMP and DMSO, and mixtures thereof,

The reaction can be also carried out, in the presence of a ligand, as necessary. Examples of the ligand include acetylacetone, salen, and phenanthroline.

In the reaction, the thiobenzoic acid is usually used in a ratio of 1 to 10 mol, the base is usually used in a ratio of 1 to 2 mol, the catalyst is usually used in a ratio of 0.1 to 5 mol, and the ligand is usually used in a ratio of 0.1 to 5 mol, based on 1 mol, of the intermediate compound (M16).

The reaction temperature is usually within the range of 50 to 200°C, and the reaction time is usually within the range of 0.5 to 24 hours.

After completion of the reaction, the reaction mixture is extracted with an organic solvent, and the organic layer is subjected to post-treatment operations such as drying and concentration, whereby the intermediate compound (M19) can be isolated. The isolated intermediate compound (M19) also can be further purified by chromatography, recrystallization, or the like.

In the reaction, $V^1$ is preferably a bromine atom and an iodide atom.

The intermediate compound (M17) can be produced by hydrolyzing the intermediate compound (M19).

When the hydrolysis reaction is carried out with an acid, an aqueous solution of the acid is usually used as a solvent. Examples of the acid include mineral acids such as hydrochloric acid, nitric acid, phosphoric acid and sulfuric acid, and carboxylic acids such as acetic acid and trifluoroacetic acid.

In the reaction, the acid is usually used in a ratio of 1 mol or more, based on 1 mol of the intermediate compound (M19).

The reaction temperature is usually within the range of 0 to 100°C, and the reaction time is usually within the

range of 0.1 to 24 hours.

After completion of the reaction, the reaction mixture is extracted with an organic solvent, and the organic layer is subjected to post-treatment operations such as drying and concentration, whereby the intermediate compound (M17) can be isolated. The isolated intermediate compound (M17) also can be further purified by chromatography, recrystallization, or the like.

When the hydrolysis reaction is carried out with a base, the reaction is usually carried out in a solvent. Examples of the solvent include ethers such as THF, ethylene glycol dimethyl ether, tert-butyl methyl ether and 1,4-dioxane, alcohols such as methanol and ethanol, water, and mixtures thereof.

Examples of the base include alkali metal hydroxides such as sodium hydroxide and potassium hydroxide.

In the reaction, the base is usually used in a ratio of 1 to 10 mol, based on 1 mol of the intermediate compound (M19).

The reaction temperature is usually within the range of 0 to 120°C, and the reaction time is usually within the range of 0.1 to 24 hours.

After completion of the reaction, the reaction liquid is acidified, then the reaction mixture is extracted with an organic solvent, and the organic layer is subjected to post-treatment operations such as drying and concentration, whereby the intermediate compound (M17) can be isolated. The isolated intermediate compound (M17) also can be further purified by chromatography, recrystallization, or the like. Here, the reaction from the intermediate compound (M17) to the intermediate compound (M18) is likely to occur, and the intermediate compound (M18) is sometimes produced during the synthesis of the intermediate compound (M17).

(Production Method 17)

The compound of the present invention (1-m0) in which G is $S(O)_mR^9$, and m is 0 (i.e., the compound of the present invention in which G is $SR^9$) can be produced by reacting the intermediate compound (M18) with compound (M20) in the presence of a reducing agent.

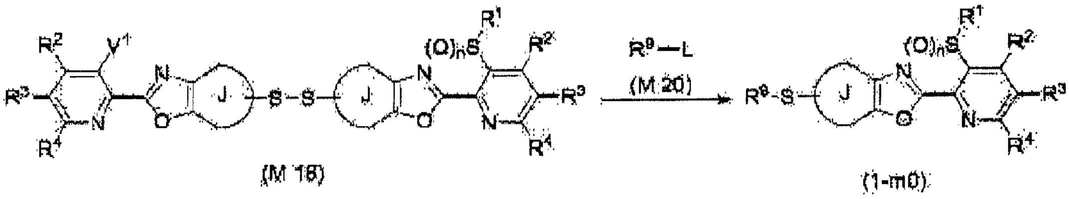


(M18) (1-m0)

In the formula, L represents a bromine atom or an iodine atom, and other symbols represent the same meaning as in the formula (1).

Examples of the reducing agent include tetrakis(dimethylamino)ethylene, hydrazine, and hydrazine hydride.

The reaction is usually carried out in a solvent. Examples of the solvent include ethers such as THF, ethylene glycol dimethyl ether, tert-butylmethyl ether and 1, 4-dioxane, aromatic hydrocarbons such as toluene and xylene, nitriles such as acetonitrile, aprotic polar solvents such as DMF, NMP and DMSO, and mixtures thereof.

Examples of the intermediate compound (M20) include trifluoromethane iodide, pentafluoroethane iodide, heptafluoro-2-iodopropane, and the like.

In the reaction, the intermediate compound (M20) is usually used in a ratio of 2 to 10 mol, and the reducing agent is used in a ratio of 1 to 5 mol, based on 1 mol of the intermediate compound (M18).

The reaction temperature is usually within the range of -80 to 50°C. The reaction time is usually within the range of 0.1 to 24 hours.

After completion of the reaction, the reaction mixture is extracted with an organic solvent, and the organic layer is subjected to post-treatment operations such as drying and concentration, whereby the compound of the present invention (1-m0) in which m is 0 can be isolated. The isolated compound of the present invention (1-m0) in which m is 0 also can be further purified by chromatography, recrystallization, or the like.

(Production Method 18)

The compound of the present invention (1-G=Rf) in which G is a C1 to C3 perfluoroalkyl group in the formula (1) can be produced by reacting the intermediate compound (M16) in which G is $V^1$ with compound (M21) or compound (M21'), in the presence of copper or a copper compound.

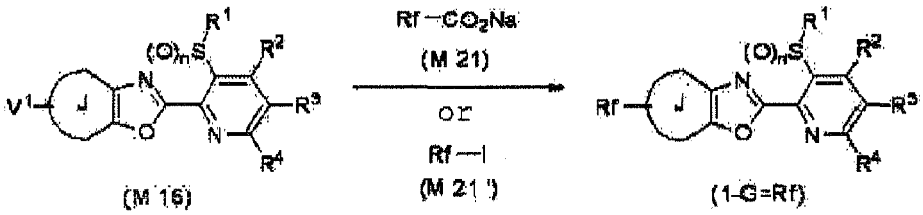


(M 16)

(1-G=Rf)

In the formula, Rf represents a C1 to C3 perfluoroalkyl group, and other symbols represent the same meaning as described above.

Examples of the copper compound include copper (I) iodide.

The reaction is usually carried out in a solvent. Examples of the solvent include aromatic hydrocarbons such as toluene and xylene, aprotic polar solvents such as DMF, NMP and DMSO, and mixtures thereof,

When the compound (M21) is used in the reaction, the compound (M21) is usually used in a ratio of 1 to 10 mol, and copper or the copper compound is usually used in a ratio of 0.5 to 10 mol, based on 1 mol of the intermediate compound (M16).

The reaction temperature is usually within the range of 100 to 200°C, and the reaction time is usually within the range of 0.5 to 48 hours.

When the intermediate compound (M21') is used in the reaction, potassium fluoride may be added. The compound (M21') is usually used in a ratio of 1 to 10 mol, the copper compound is usually used in a ratio of 0.1 to 10 mol, and potassium fluoride is usually used in a ratio of 0.1 to 5 mol, based on 1 mol of the intermediate compound (M16).

The reaction temperature is usually within the range of 0 to 150°C, and the reaction time is usually within the range of 0.5 to 48 hours.

After completion of the reaction, the reaction mixture is extracted with an organic solvent, and the organic layer is subjected to post-treatment operations such as drying and concentration, whereby the compound of the present invention (1-G=Rf) can be isolated. The isolated compound of the present invention (1-G=Rf) also can be further purified by chromatography, recrystallization, or the like.

$V^1$ is preferably a bromine atom or an iodine atom.

Next, specific examples of the compound of the present invention are shown below.

In formula (1-5),

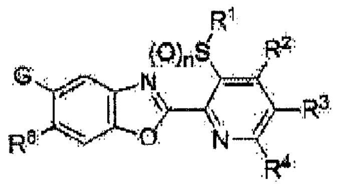

(1-5)

compounds of the present invention wherein $R^2$, $R^3$ and $R^4$ are a hydrogen atom, $R^6$ is a fluorine atom, and $R^1$, G and n are as in the combinations shown in [Table 1] to [Table 11];

[Table 1]

| $R^1$ | G | n |
|---|---|---|
| Me | $CF_3$ | 0 |
| Et | $CF_3$ | 0 |
| Pr | $CF_3$ | 0 |
| *i*Pr | $CF_3$ | 0 |
| t-Bu | $CF_3$ | 0 |
| $CF_3$ | $CF_3$ | 0 |
| $CF_2CF_3$ | $CF_3$ | 0 |
| $CH=CH_2$ | $CF_3$ | 0 |
| $CH_2CH=CH_2$ | $CF_3$ | 0 |
| C≡CH | $CF_3$ | 0 |

(continued)

| $R^1$ | G | n |
|---|---|---|
| $CH_2C\equiv CH$ | $CF_3$ | 0 |
| CycPr | $CF_3$ | 0 |
| $CH_2$CycPr | $CF_3$ | 0 |
| Me | $CF_2CF_3$ | 0 |
| Et | $CF_2CF_3$ | 0 |
| Pr | $CF_2CF_3$ | 0 |
| *i*Pr | $CF_2CF_3$ | 0 |
| t-Bu | $CF_2CF_3$ | 0 |
| $CF_3$ | $CF_2CF_3$ | 0 |
| $CF_2CF_3$ | $CF_2CF_3$ | 0 |
| $CH=CH_2$ | $CF_2CF_3$ | 0 |
| $CH_2CH=CH_2$ | $CF_2CF_3$ | 0 |
| $C\equiv CH$ | $CF_2CF_3$ | 0 |
| $CH_2C\equiv CH$ | $CF_2CF_3$ | 0 |
| CycPr | $CF_2CF_3$ | 0 |
| $CH_2$CycPr | $CF_2CF_3$ | 0 |

[Table 2]

| $R^1$ | G | n |
|---|---|---|
| Me | $CF(CF_3)_2$ | 0 |
| Et | $CF(CF_3)_2$ | 0 |
| Pr | $CF(CF_3)_2$ | 0 |
| *i*Pr | $CF(CF_3)_2$ | 0 |
| t-Bu | $CF(CF_3)_2$ | 0 |
| $CF_3$ | $CF(CF_3)_2$ | 0 |
| $CF_2CF_3$ | $CF(CF_3)_2$ | 0 |
| $CH=CH_2$ | $CF(CF_3)_2$ | 0 |
| $CH_2CH=CH_2$ | $CF(CF_3)_2$ | 0 |
| $C=CH$ | $CF(CF_3)_2$ | 0 |
| $CH_2C\equiv CH$ | $CF(CF_3)_2$ | 0 |
| CycPr | $CF(CF_3)_2$ | 0 |
| $CH_2$CycPr | $CF(CF_3)_2$ | 0 |
| Me | $OCF_3$ | 0 |
| Et | $OCF_3$ | 0 |
| Pr | $OCF_3$ | 0 |
| *i*Pr | $OCF_3$ | 0 |
| t-Bu | $OCF_3$ | 0 |

(continued)

| $R^1$ | G | n |
|---|---|---|
| $CF_3$ | $OCF_3$ | 0 |
| $CF_2CF_3$ | $OCF_3$ | 0 |
| $CH=CH_2$ | $OCF_3$ | 0 |
| $CH_2CH=CH_2$ | $OCF_3$ | 0 |
| $C\equiv CH$ | $OCF_3$ | 0 |
| $CH_2C\equiv CH$ | $OCF_3$ | 0 |
| CycPr | $OCF_3$ | 0 |
| $CH_2$CycPr | $OCF_3$ | 0 |

[Table 3]

| $R^1$ | G | n |
|---|---|---|
| Me | $SCF_3$ | 0 |
| Et | $SCF_3$ | 0 |
| Pr | $SCF_3$ | 0 |
| *i*Pr | $SCF_3$ | 0 |
| t-Bu | $SCF_3$ | 0 |
| $CF_3$ | $SCF_3$ | 0 |
| $CF_2CF_3$ | $SCF_3$ | 0 |
| $CH=CH_2$ | $SCF_3$ | 0 |
| $CH_2CH=CH_2$ | $SCF_3$ | 0 |
| $C\equiv CH$ | $SCF_3$ | 0 |
| $CH_2C\equiv CH$ | $SCF_3$ | 0 |
| CycPr | $SCF_3$ | 0 |
| $CH_2$CycPr | $SCF_3$ | 0 |
| Me | $S(O)CF_3$ | 0 |
| Et | $S(O)CF_3$ | 0 |
| Pr | $S(O)CF_3$ | 0 |
| *i*Pr | $S(O)CF_3$ | 0 |
| *t*-Bu | $S(O)CF_3$ | 0 |
| $CF_3$ | $S(O)CF_3$ | 0 |
| $CF_2CF_3$ | $S(O)CF_3$ | 0 |
| $CH=CH_2$ | $S(O)CF_3$ | 0 |
| $CH_2CH=CH_2$ | $S(O)CF_3$ | 0 |
| $C\equiv CH$ | $S(O)CF_3$ | 0 |
| $CH_2C\equiv CH$ | $S(O)CF_3$ | 0 |
| CycPr | $S(O)CF_3$ | 0 |
| $CH_2$CycPr | $S(O)CF_3$ | 0 |

[Table 4]

| $R^1$ | G | n |
|---|---|---|
| Me | $S(O)_2CF_3$ | 0 |
| Et | $S(O)_2CF_3$ | 0 |
| Pr | $S(O)_2CF_3$ | 0 |
| *i*Pr | $S(O)_2CF_3$ | 0 |
| t-Bu | $S(O)_2CF_3$ | 0 |
| $CF_3$ | $S(O)_2CF_3$ | 0 |
| $CF_2CF_3$ | $S(O)_2CF_3$ | 0 |
| $CH=CH_2$ | $S(O)_2CF_3$ | 0 |
| $CH_2CH=CH_2$ | $S(O)_2CF_3$ | 0 |
| $C\equiv CH$ | $S(O)_2CF_3$ | 0 |
| $CH_2C\equiv CH$ | $S(O)_2CF_3$ | 0 |
| CycPr | $S(O)_2CF_3$ | 0 |
| $CH_2$CycPr | $S(O)_2CF_3$ | 0 |
| Me | $CF_3$ | 1 |
| Et | $CF_3$ | 1 |
| Pr | $CF_3$ | 1 |
| *i*Pr | $CF_3$ | 1 |
| t-Bu | $CF_3$ | 1 |
| $CF_3$ | $CF_3$ | 1 |
| $CF_2CF_3$ | $CF_3$ | 1 |
| $CH=CH_2$ | $CF_3$ | 1 |
| $CH_2CH=CH_2$ | $CF_3$ | 1 |
| $C\equiv CH$ | $CF_3$ | 1 |
| $CH_2C\equiv CH$ | $CF_3$ | 1 |
| CycPr | $CF_3$ | 1 |
| $CH_2$CycPr | $CF_3$ | 1 |

[Table 5]

| $R^1$ | G | n |
|---|---|---|
| Me | $CF_2CF_3$ | 1 |
| Et | $CF_2CF_3$ | 1 |
| Pr | $CF_2CF_3$ | 1 |
| *i*Pr | $CF_2CF_3$ | 1 |
| t-Bu | $CF_2$CF3 | 1 |
| $CF_3$ | $CF_2CF_3$ | 1 |
| $CF_2CF_3$ | $CF_2CF_3$ | 1 |
| $CH=CH_2$ | $CF_2CF_3$ | 1 |

(continued)

| $R^1$ | G | n |
|---|---|---|
| $CH_2CH=CH_2$ | $CF_2CF_3$ | 1 |
| $C{\equiv}CH$ | $CF_2CF_3$ | 1 |
| $CH_2C{\equiv}CH$ | $CF_2CF_3$ | 1 |
| CycPr | $CF_2CF_3$ | 1 |
| $CH_2$CycPr | $CF_2CF_3$ | 1 |
| Me | $CF(CF_3)_2$ | 1 |
| Et | $CF(CF_3)_2$ | 1 |
| Pr | $CF(CF_3)_2$ | 1 |
| *i*Pr | $CF(CF_3)_2$ | 1 |
| t-Bu | $CF(CF_3)_2$ | 1 |
| $CF_3$ | $CF(CF_3)_2$ | 1 |
| $CF_2CF_3$ | $CF(CF_3)_2$ | 1 |
| $CH=CH_2$ | $CF(CF_3)_2$ | 1 |
| $CH_2CH=CH_2$ | $CF(CF_3)_2$ | 1 |
| $C{\equiv}CH$ | $CF(CF_3)_2$ | 1 |
| $CH_2C{\equiv}CH$ | $CF(CF_3)_2$ | 1 |
| CycPr | $CF(CF_3)_2$ | 1 |
| $CH_2$CycPr | $CF(CF_3)_2$ | 1 |

[Table 6]

| $R^1$ | G | n |
|---|---|---|
| Me | $OCF_3$ | 1 |
| Et | $OCF_3$ | 1 |
| Pr | $OCF_3$ | 1 |
| *i*Pr | $OCF_3$ | 1 |
| t-Bu | $OCF_3$ | 1 |
| $CF_3$ | $OCF_3$ | 1 |
| $CF_2CF_3$ | $OCF_3$ | 1 |
| $CH=CH_2$ | $OCF_3$ | 1 |
| $CH_2CH=CH_2$ | $OCF_3$ | 1 |
| $C{\equiv}CH$ | $OCF_3$ | 1 |
| $CH_2C{\equiv}CH$ | $OCF_3$ | 1 |
| CycPr | $OCF_3$ | 1 |
| $CH_2$CycPr | $OCF_3$ | 1 |
| Me | $SCF_3$ | 1 |
| Et | $SCF_3$ | 1 |
| Pr | $SCF_3$ | 1 |

(continued)

| $R^1$ | G | n |
|---|---|---|
| *i*Pr | $SCF_3$ | 1 |
| t-Bu | $SCF_3$ | 1 |
| $CF_3$ | $SCF_3$ | 1 |
| $CF_2CF_3$ | $SCF_3$ | 1 |
| $CH=CH_2$ | $SCF_3$ | 1 |
| $CH_2CH=CH_2$ | $SCF_3$ | 1 |
| C≡CH | $SCF_3$ | 1 |
| $CH_2C≡CH$ | $SCF_3$ | 1 |
| CycPr | $SCF_3$ | 1 |
| $CH_2CycPr$ | $SCF_3$ | 1 |

[Table 7]

| $R^1$ | G | n |
|---|---|---|
| Me | $S(O)CF_3$ | 1 |
| Et | $S(O)CF_3$ | 1 |
| Pr | $S(O)CF_3$ | 1 |
| *i*Pr | $S(O)CF_3$ | 1 |
| t-Bu | $S(O)CF_3$ | 1 |
| $CF_3$ | $S(O)CF_3$ | 1 |
| $CF_2CF_3$ | $S(O)CF_3$ | 1 |
| $CH=CH_2$ | $S(O)CF_3$ | 1 |
| $CH_2CH=CH_2$ | $S(O)CF_3$ | 1 |
| C≡CH | $S(O)CF_3$ | 1 |
| $CH_2C=CH$ | $S(O)CF_3$ | 1 |
| CycPr | $S(O)CF_3$ | 1 |
| $CH_2CycPr$ | $S(O)CF_3$ | 1 |
| Me | $S(O)_2CF_3$ | 1 |
| Et | $S(O)_2CF_3$ | 1 |
| Pr | $S(O)_2CF_3$ | 1 |
| *i*Pr | $S(O)_2CF_3$ | 1 |
| t-Bu | $S(O)_2CF_3$ | 1 |
| $CF_3$ | $S(O)_2CF_3$ | 1 |
| $CF_2CF_3$ | $S(O)_2CF_3$ | 1 |
| $CH=CH_2$ | $S(O)_2CF_3$ | 1 |
| $CH_2CH=CH_2$ | $S(O)_2CF_3$ | 1 |
| C≡CH | $S(O)_2CF_3$ | 1 |
| $CH_2C≡cH$ | $S(O)_2CF_3$ | 1 |

(continued)

| $R^1$ | G | n |
|---|---|---|
| CycPr | $S(O)_2CF_3$ | 1 |
| $CH_2$CycPr | $S(O)_2CF_3$ | 1 |

[Table 8]

| $R^1$ | G | n |
|---|---|---|
| Me | $CF_3$ | 2 |
| Et | $CF_3$ | 2 |
| Pr | $CF_3$ | 2 |
| *i*Pr | $CF_3$ | 2 |
| t-Bu | $CF_3$ | 2 |
| $CF_3$ | $CF_3$ | 2 |
| $CF_2CF_3$ | $CF_3$ | 2 |
| $CH=CH_2$ | $CF_3$ | 2 |
| $CH_2CH=CH_2$ | $CF_3$ | 2 |
| $C\equiv CH$ | $CF_3$ | 2 |
| $CH_3C\equiv CH$ | $CF_3$ | 2 |
| CycPr | $CF_3$ | 2 |
| $CH_2$CycPr | $CF_3$ | 2 |
| Me | $CF_2CF_3$ | 2 |
| Et | $CF_2CF_3$ | 2 |
| Pr | $CF_2CF_3$ | 2 |
| *i*Pr | $CF_2CF_3$ | 2 |
| t-Bu | $CF_2CF_3$ | 2 |
| $CF_3$ | $CF_2CF_3$ | 2 |
| $CF_2CF_3$ | $CF_2CF_3$ | 2 |
| $CH=CH_2$ | $CF_2CF_3$ | 2 |
| $CH_2CH=CH_2$ | $CF_2CF_3$ | 2 |
| $C\equiv CH$ | $CF_2CF_3$ | 2 |
| $CH_2C\equiv CH$ | $CF_2CF_3$ | 2 |
| CycPr | $CF_2CF_3$ | 2 |
| $CH_2$CycPr | $CF_2CF_3$ | 2 |

[Table 9]

| $R^1$ | G | n |
|---|---|---|
| Me | $CF(CF_3)_2$ | 2 |
| Et | $CF(CF_3)_2$ | 2 |
| Pr | $CF(CF_3)_2$ | 2 |

(continued)

| $R^1$ | G | n |
|---|---|---|
| *i*Pr | $CF(CF_3)_2$ | 2 |
| t-Bu | $CF(CF_3)_2$ | 2 |
| $CF_3$ | $CF(CF_3)_2$ | 2 |
| $CF_2CF_3$ | $CF(CF_3)_2$ | 2 |
| $CH{=}CH_2$ | $CF(CF_3)_2$ | 2 |
| $CH_2CH{=}CH_2$ | $CF(CF_3)_2$ | 2 |
| C=CH | $CF(CF_3)_2$ | 2 |
| $CH_2C{\equiv}CH$ | $CF(CF_3)_2$ | 2 |
| CycPr | $CF(CF_3)_2$ | 2 |
| $CH_2$CycPr | $CF(CF_3)_2$ | 2 |
| Me | $OCF_3$ | 2 |
| Et | $OCF_3$ | 2 |
| Pr | $OCF_3$ | 2 |
| *i*Pr | $OCF_3$ | 2 |
| t-Bu | $OCF_3$ | 2 |
| $CF_3$ | $OCF_3$ | 2 |
| $CF_2CF_3$ | $OCF_3$ | 2 |
| $CH{=}CH_2$ | $OCF_3$ | 2 |
| $CH_2CH{=}CH_2$ | $OCF_3$ | 2 |
| $C{\equiv}CH$ | $OCF_3$ | 2 |
| $CH_2C{\equiv}CH$ | $OCF_3$ | 2 |
| CycPr | $OCF_3$ | 2 |
| $CH_2$CycPr | $OCF_3$ | 2 |

[Table 10]

| $R^1$ | G | n |
|---|---|---|
| Me | $SCF_3$ | 2 |
| Et | $SCF_3$ | 2 |
| Pr | $SCF_3$ | 2 |
| *i*Pr | $SCF_3$ | 2 |
| t-Bu | $SCF_3$ | 2 |
| $CF_3$ | $SCF_3$ | 2 |
| $CF_2CF_3$ | $SCF_3$ | 2 |
| $CH{=}CH_2$ | $SCF_3$ | 2 |
| $CH_2CH{=}CH_2$ | $SCF_3$ | 2 |
| $C{\equiv}CH$ | $SCF_3$ | 2 |
| $CH_2C{\equiv}CH$ | $SCF_3$ | 2 |

(continued)

| $R^1$ | G | n |
|---|---|---|
| CycPr | $SCF_3$ | 2 |
| $CH_2$CycPr | $SCF_3$ | 2 |
| Me | $S(O)CF_3$ | 2 |
| Et | $S(O)CF_3$ | 2 |
| Pr | $S(O)CF_3$ | 2 |
| *i*Pr | $S(O)CF_3$ | 2 |
| t-Bu | $S(O)CF_3$ | 2 |
| $CF_3$ | $S(O)CF_3$ | 2 |
| $CF_2CF_3$ | $S(O)CF_3$ | 2 |
| $CH=CH_2$ | $S(O)CF_3$ | 2 |
| $CH_2CH=CH_2$ | $S(O)CF_3$ | 2 |
| $C\equiv CH$ | $S(O)CF_3$ | 2 |
| $CH_2C\equiv CH$ | $S(O)CF_3$ | 2 |
| CycPr | $S(O)CF_3$ | 2 |
| $CH_2$CycPr | $S(O)CF_3$ | 2 |

[Table 11]

| $R^1$ | G | n |
|---|---|---|
| Me | $S(O)_2CF_3$ | 2 |
| Et | $S(O)_2CF_3$ | 2 |
| Pr | $S(O)_2CF_3$ | 2 |
| *i*Pr | $S(O)_2CF_3$ | 2 |
| t-Bu | $S(O)_2CF_3$ | 2 |
| $CF_3$ | $S(O)_2CF_3$ | 2 |
| $CF_2CF_3$ | $S(O)_2CF_3$ | 2 |
| $CH=CH_2$ | $S(O)_2CF_3$ | 2 |
| $CH_2CH=CH_2$ | $S(O)_2CF_3$ | 2 |
| $C=CH$ | $S(O)_2CF_3$ | 2 |
| $CH_2C\equiv CH$ | $S(O)_2CF_3$ | 2 |
| CycPr | $S(O)_2CF_3$ | 2 |
| $CH_2$CycPr | $S(O)_2CF_3$ | 2 |

In [Table 1] to [Table 11] above, Me represents a methyl group, Et represents an ethyl group, Pr represents a propyl group, iPr represents an isopropyl group, t-Bu represents a tert-butyl group, and CycPr represents a cyclopropyl group. In the formula (1-5), compounds of the present invention wherein $R^2$, $R^3$ and $R^4$ are a hydrogen atom, $R^6$ is a chlorine atom, and $R^1$, G and n are as in the combinations shown in [Table 1] to [Table 11];
In the formula (1-5), compounds of the present invention wherein $R^2$, $R^3$ and $R^4$ are a hydrogen atom, $R^5$ is a bromine atom, and $R^1$, G and n are as in the combinations shown in [Table 1] to [Table 11];
In the formula (1-5), compounds of the present invention wherein $R^2$, $R^3$ and $R^4$ are a hydrogen atom, $R^6$ is an iodine atom, and $R^1$, G and n are as in the combinations shown in [Table 1] to [Table 11];

In the formula (1-5), compounds of the present invention wherein $R^2$, $R^3$ and $R^4$ are a hydrogen atom, $R^6$ is a methoxy group, and $R^1$, G and n are as in the combinations shown in [Table 1] to [Table 11];

In the formula (1-5), compounds of the present invention wherein $R^2$, $R^3$ and $R^4$ are a hydrogen atom, $R^6$ is an ethoxy group, and $R^1$, G and n are as in the combinations shown in [Table 1] to [Table 11];

In the formula (1-5), compounds of the present invention wherein $R^2$, $R^3$ and $R^4$ are a hydrogen atom, $R^6$ is a methylsulfanyl group, and $R^1$, G and n are as in the combinations shown in [Table 1] to [Table 11];

In the formula (1-5), compounds of the present invention wherein $R^2$, $R^3$ and $R^4$ are a hydrogen atom, $R^6$ is a methylsulfonyl group, and $R^1$, G and n are as in the combinations shown in [Table 1] to [Table 11];

In the formula (1-5), compounds of the present invention wherein $R^2$, $R^3$ and $R^4$ are a hydrogen atom, $R^6$ is an methylsulfanyl group, and $R^1$, G and n are as in the combinations shown in [Table 1] to [Table 11];

In the formula (1-5), compounds of the present invention wherein $R^2$, $R^3$ and $R^4$ are a hydrogen atom, $R^6$ is an ethylsulfonyl group, and $R^1$, G and n are as in the combinations shown in [Table 1] to [Table 11];

In the formula (1-5), compounds of the present invention wherein $R^2$, $R^3$ and $R^4$ are a hydrogen atom, $R^6$ is a methylamino group, and $R^1$, G and n are as in the combinations shown in [Table 1] to [Table 11];

In the formula (1-5), compounds of the present invention wherein $R^2$, $R^3$ and $R^4$ are a hydrogen atom, $R^6$ is a dimethylamino group, and $R^1$, G and n are as in the combinations shown in [Table 1] to [Table 11];

In the formula (1-5), compounds of the present invention wherein $R^2$, $R^3$ and $R^4$ are a hydrogen atom, $R^6$ is a diethylamino group, and $R^1$, G and n are as in the combinations shown in [Table 1] to [Table 11];

In the formula (1-5), compounds of the present invention wherein $R^2$, $R^3$ and $R^4$ are a hydrogen atom, $R^6$ is a trifluoromethyl group, and $R^1$, G and n are as in the combinations shown in [Table 1] to [Table 11];

In the formula (1-5), compounds of the present invention wherein $R^2$ and $R^4$ are a hydrogen atom, $R^3$ is a chlorine atom, $R^6$ is a fluorine atom, and $R^1$, G and n are as in the combinations shown in [Table 1] to [Table 11];

In the formula (1-5), compounds of the present invention wherein $R^2$ and $R^4$ are a hydrogen atom, $R^3$ is a chlorine atom, $R^6$ is a chlorine atom, and $R^1$, G and n are as in the combinations shown in [Table 1] to [Table 11];

In the formula (1-5), compounds of the present invention wherein $R^2$ and $R^4$ are a hydrogen atom, $R^3$ is a chlorine atom, $R^6$ is a bromine atom, and $R^1$, G and n are as in the combinations shown in [Table 1] to [Table 11];

In the formula (1-5), compounds of the present invention wherein $R^2$ and $R^4$ are a hydrogen atom, $R^3$ is a chlorine atom, $R^6$ is an iodine atom, and $R^1$, G and n are as in the combinations shown in [Table 1] to [Table 11];

In the formula (1-5), compounds of the present invention wherein $R^2$ and $R^4$ are a hydrogen atom, $R^3$ is a chlorine atom, $R^6$ is a methoxy group, and $R^1$, G and n are as in the combinations shown in [Table 1] to [Table 11];

In the formula (1 5), compounds of the present invention wherein $R^2$ and $R^4$ are a hydrogen atom, $R^3$ is a chlorine atom, $R^6$ is an ethoxy group, and $R^1$, G and n are as in the combinations shown in [Table 1] to [Table 11];

In the formula (1-5), compounds of the present invention wherein $R^2$ and $R^4$ are a hydrogen atom, $R^3$ is a chlorine atom, $R^6$ is a methylsulfanyl group, and $R^1$, G and n are as in the combinations shown in [Table 1] to [Table 11];

In the formula (1-5), compounds of the present invention wherein $R^2$ and $R^4$ are a hydrogen atom, $R^3$ is a chlorine atom, $R^6$ is a methylsulfonyl group, and $R^1$, G and n are as in the combinations shown in [Table 1] to [Table 11];

In the formula (1-5), compounds of the present invention wherein $R^2$ and $R^4$ are a hydrogen atom, $R^3$ is a chlorine atom, $R^6$ is an ethylsulfanyl group, and $R^1$, G and n are as in the combinations shown in [Table 1] to [Table 11];

In the formula (1-5), compounds of the present invention wherein $R^2$ and $R^4$ are a hydrogen atom, $R^3$ is a chlorine atom, $R^6$ is an ethylsulfonyl group, and $R^1$, G and n are as in the combinations shown in [Table 1] to [Table 11];

In the formula (1-5), compounds of the present invention wherein $R^2$ and $R^4$ are a hydrogen atom, $R^3$ is a chlorine atom, $R^6$ is a methylamino group, and $R^1$, G and n are as in the combinations shown in [Table 1] to [Table 11];

In the formula (1-5), compounds of the present invention wherein $R^2$ and $R^4$ are a hydrogen atom, $R^3$ is a chlorine atom, $R^6$ is a dimethylamino group, and $R^1$, G and n are as in the combinations shown in [Table 1] to [Table 11];

In the formula (1-5), compounds of the present invention wherein $R^2$ and $R^4$ are a hydrogen atom, $R^3$ is a chlorine atom, $R^6$ is a diethylamino group, and $R^1$, G and n are as in the combinations shown in [Table 1] to [Table 11];

In the formula (1-5), compounds of the present invention wherein $R^2$ and $R^4$ are a hydrogen atom, $R^3$ is a chlorine atom, $R^6$ is a trifluoromethyl group, and $R^1$, G and n are as in the combinations shown in [Table 1] to [Table 11];

In the formula (1-5), compounds of the present invention wherein $R^2$ and $R^4$ are a hydrogen atom, $R^3$ is a trifluoromethyl group, $R^6$ is a fluorine atom, and $R^1$, G and n are as in the combinations shown in [Table 1] to [Table 11];

In the formula (1-5), compounds of the present invention wherein $R^2$ and $R^4$ are a hydrogen atom, $R^3$ is a trifluoromethyl group, $R^6$ is a chlorine atom, and $R^1$, G and n are as in the combinations shown in [Table 1] to [Table 11];

In the formula (1-5), compounds of the present invention wherein $R^2$ and $R^4$ are a hydrogen atom, $R^3$ is a trifluoromethyl group, $R^6$ is a bromine atom, and $R^1$, G and n are as in the combinations shown in [Table 1] to [Table 11];

In the formula (1-5), compounds of the present invention wherein $R^2$ and $R^4$ are a hydrogen atom, $R^3$ is a trifluoromethyl group, $R^6$ is an iodine atom, and $R^1$, G and n are as in the combinations shown in [Table 1] to [Table 11];

In the formula (1-5), compounds of the present invention wherein $R^2$ and $R^4$ are a hydrogen atom, $R^3$ is a trifluoromethyl group, $R^6$ is a methoxy group, and $R^1$, G and n are as in the combinations shown in [Table 1] to [Table 11];

In the formula (1-5), compounds of the present invention wherein $R^2$ and $R^4$ are a hydrogen atom, $R^3$ is a trifluoromethyl group, $R^6$ is an ethoxy group, and $R^1$, G and n are as in the combinations shown in [Table 1] to [Table 11];
In the formula (1-5), compounds of the present invention wherein $R^2$ and $R^4$ are a hydrogen atom, $R^3$ is a trifluoromethyl group, $R^6$ is a methylsulfanyl group, and $R^1$, G and n are as in the combinations shown in [Table 1] to [Table 11];
In the formula (1-5), compounds of the present invention wherein $R^2$ and $R^4$ are a hydrogen atom, $R^3$ is a trifluoromethyl group, $R^6$ is a methylsulfonyl group, and $R^1$, G and n are as in the combinations shown in [Table 1] to [Table 11];
In the formula (1-5), compounds of the present invention wherein $R^2$ and $R^4$ are a hydrogen atom, $R^3$ is a trifluoromethyl group, $R^6$ is an methylsulfanyl group, and $R^1$, G and n are as in the combinations shown in [Table 1] to [Table 11];
In the formula (1-5), compounds of the present invention wherein $R^2$ and $R^4$ are a hydrogen atom, $R^3$ is a trifluoromethyl group, $R^6$ is an ethylsulfonyl group, and $R^1$, G and n are as in the combinations shown in [Table 1] to [Table 11];
In the formula (1-5), compounds of the present invention wherein $R^2$ and $R^4$ are a hydrogen atom, $R^3$ is a trifluoromethyl group, $R^6$ is a methylamino group, and $R^1$, G and n are as in the combinations shown in [Table 1] to [Table 11];
In the formula (1-5), compounds of the present invention wherein $R^2$ and $R^4$ are a hydrogen atom, $R^3$ is a trifluoromethyl group, $R^6$ is a dimethylamino group, and $R^1$, G and n are as in the combinations shown in [Table 1] to [Table 11];
In the formula (1-5), compounds of the present invention wherein $R^2$ and $R^4$ are a hydrogen atom, $R^3$ is a trifluoromethyl group, $R^6$ is a diethylamino group, and $R^1$, G and n are as in the combinations shown in [Table 1] to [Table 11];
In the formula (1-5), compounds of the present invention wherein $R^2$ and $R^4$ are a hydrogen atom, $R^3$ is a trifluoromethyl group, $R^6$ is a trifluoromethyl group, and $R^1$, G and n are as in the combinations shown in [Table 1] to [Table 11];
In formula (1-6),

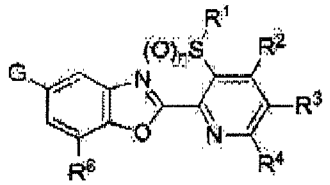
(1-6)

compounds of the present invention wherein $R^2$, $R^3$ and $R^4$ are a hydrogen atom, $R^6$ is a fluorine atom, and $R^1$, G and n are as in the combinations shown in [Table 1] to [Table 11];
In the formula (1-6), compounds of the present invention wherein $R^2$, $R^3$ and $R^4$ are a hydrogen atom, $R^6$ is a chlorine atom, and $R^1$, G and n are as in the combinations shown in [Table 1] to [Table 11];
In the formula (1-6), compounds of the present invention wherein $R^2$, $R^3$ and $R^4$ are a hydrogen atom, $R^6$ is a bromine atom, and $R^1$, G and n are as in the combinations shown in [Table 1] to [Table 11];
In the formula (1-6), compounds of the present invention wherein $R^2$, $R^3$ and $R^4$ are a hydrogen atom, $R^6$ is an iodine atom, and $R^1$, G and n are as in the combinations shown in [Table 1] to [Table 11];
In the formula (1-6), compounds of the present invention wherein $R^2$, $R^3$ and $R^4$ are a hydrogen atom, $R^6$ is a methoxy group, and $R^1$, G and n are as in the combinations shown in [Table 1] to [Table 11];
In the formula (1-6), compounds of the present invention wherein $R^2$, $R^3$ and $R^4$ are a hydrogen atom, $R^6$ is an ethoxy group, and $R^1$, G and n are as in the combinations shown in [Table 1] to [Table 11];
In the formula (1-6), compounds of the present invention wherein $R^2$, $R^3$ and $R^4$ are a hydrogen atom, $R^6$ is a methylsulfanyl group, and $R^1$, G and n are as in the combinations shown in [Table 1] to [Table 11];
In the formula (1-6), compounds of the present invention wherein $R^2$, $R^3$ and $R^4$ are a hydrogen atom, $R^6$ is a methylsulfonyl group, and $R^1$, G and n are as in the combinations shown in [Table 1] to [Table 11];
In the formula (1-6), compounds of the present invention wherein $R^2$, $R^3$ and $R^4$ are a hydrogen atom, $R^6$ is an methylsulfanyl group, and $R^1$, G and n are as in the combinations shown in [Table 1] to [Table 11];
In the formula (1-6), compounds of the present invention wherein $R^2$, $R^3$ and $R^4$ are a hydrogen atom, $R^6$ is an ethylsulfonyl group, and $R^1$, G and n are as in the combinations shown in [Table 1] to [Table 11];
In the formula (1-6), compounds of the present invention wherein $R^2$, $R^3$ and $R^4$ are a hydrogen atom, $R^6$ is a methylamino group, and $R^1$, G and n are as in the combinations shown in [Table 1] to [Table 11];
In the formula (1-6), compounds of the present invention wherein $R^2$, $R^3$ and $R^4$ are a hydrogen atom, $R^6$ is a dimethylamino group, and $R^1$, G and n are as in the combinations shown in [Table 1] to [Table 11];
In the formula (1-6), compounds of the present invention wherein $R^2$, $R^3$ and $R^4$ are a hydrogen atom, $R^6$ is a diethylamino group, and $R^1$, G and n are as in the combinations shown in [Table 1] to [Table 11];
In the formula (1-6), compounds of the present invention wherein $R^2$, $R^3$ and $R^4$ are a hydrogen atom, $R^6$ is a trifluoromethyl group, and $R^1$, G and n are as in the combinations shown in [Table 1] to [Table 11];
In the formula (1-6), compounds of the present invention wherein $R^2$ and $R^4$ are a hydrogen atom, $R^3$ is a chlorine atom, $R^6$ is a fluorine atom, and $R^1$, G and n are as in the combinations shown in [Table 1] to [Table 11];
In the formula (1-6), compounds of the present invention wherein $R^2$ and $R^4$ are a hydrogen atom, $R^3$ is a chlorine atom,

$R^6$ is a chlorine atom, and $R^1$, G and n are as in the combinations shown in [Table 1] to [Table 11];
In the formula (1-6), compounds of the present invention wherein $R^2$ and $R^4$ are a hydrogen atom, $R^3$ is a chlorine atom, $R^6$ is a bromine atom, and $R^1$, G and n are as in the combinations shown in [Table 1] to [Table 11];
In the formula (1-6), compounds of the present invention wherein $R^2$ and $R^4$ are a hydrogen atom, $R^3$ is a chlorine atom, $R^6$ is an iodine atom, and $R^1$, G and n are as in the combinations shown in [Table 1] to [Table 11];
In the formula (1-6), compounds of the present invention wherein $R^2$ and $R^4$ are a hydrogen atom, $R^3$ is a chlorine atom, $R^6$ is a methoxy group, and $R^1$, G and n are as in the combinations shown in [Table 1] to [Table 11];
In the formula (1-6), compounds of the present invention wherein $R^2$ and $R^4$ are a hydrogen atom, $R^3$ is a chlorine atom, $R^6$ is an ethoxy group, and $R^1$, G and n are as in the combinations shown in [Table 1] to [Table 11];
In the formula (1-6), compounds of the present invention wherein $R^2$ and $R^4$ are a hydrogen atom, $R^3$ is a chlorine atom, $R^6$ is a methylsulfanyl group, and $R^1$, G and n are as in the combinations shown in [Table 1] to [Table 11];
In the formula (1-6), compounds of the present invention wherein $R^2$ and $R^4$ are a hydrogen atom, $R^3$ is a chlorine atom, $R^6$ is a methylsulfonyl group, and $R^1$, G and n are as in the combinations shown in [Table 1] to [Table 11];
In the formula (1-6), compounds of the present invention wherein $R^2$ and $R^4$ are a hydrogen atom, $R^3$ is a chlorine atom, $R^6$ is an ethylsulfanyl group, and $R^1$, G and n are as in the combinations shown in [Table 1] to [Table 11];
In the formula (1-6), compounds of the present invention wherein $R^2$ and $R^4$ are a hydrogen atom, $R^3$ is a chlorine atom, $R^6$ is an ethylsulfonyl group, and $R^1$, G and n are as in the combinations shown in [Table 1] to [Table 11];
In the formula (1-6), compounds of the present invention wherein $R^2$ and $R^4$ are a hydrogen atom, $R^3$ is a chlorine atom, $R^6$ is a methylamino group, and $R^1$, G and n are as in the combinations shown in [Table 1] to [Table 11] ;
In the formula (1-6), compounds of the present invention wherein $R^2$ and $R^4$ are a hydrogen atom, $R^3$ is a chlorine atom, $R^6$ is a dimethylamino group, and $R^1$, G and n are as in the combinations shown in [Table 1] to [Table 11] ;
In the formula (1-6), compounds of the present invention wherein $R^2$ and $R^4$ are a hydrogen atom, $R^3$ is a chlorine atom, $R^6$ is a diethylamino group, and $R^1$, G and n are as in the combinations shown in [Table 1] to [Table 11];
In the formula (1-6), compounds of the present invention wherein $R^2$ and $R^4$ are a hydrogen atom, $R^3$ is a chlorine atom, $R^6$ is a trifluoromethyl group, and $R^1$, G and n are as in the combinations shown in [Table 1] to [Table 11];
In the formula (1-6), compounds of the present invention wherein $R^2$ and $R^4$ are a hydrogen atom, $R^3$ is a trifluoromethyl group, $R^6$ is a fluorine atom, and $R^1$, G and n are as in the combinations shown in [Table 1] to [Table 11];
In the formula (1-6), compounds of the present invention wherein $R^2$ and $R^4$ are a hydrogen atom, $R^3$ is a trifluoromethyl group, $R^6$ is a chlorine atom, and $R^1$, G and n are as in the combinations shown in [Table 1] to [Table 11];
In the formula (1-6), compounds of the present invention wherein $R^2$ and $R^4$ are a hydrogen atom, $R^3$ is a trifluoromethyl group, $R^6$ is a bromine atom, and $R^1$, G and n are as in the combinations shown in [Table 1] to [Table 11] ;
In the formula (1-6), compounds of the present invention wherein $R^2$ and $R^4$ are a hydrogen atom, $R^3$ is a trifluoromethyl group, $R^6$ is an iodine atom, and $R^1$, G and n are as in the combinations shown in [Table 1] to [Table 11];
In the formula (1-6), compounds of the present invention wherein $R^2$ and $R^4$ are a hydrogen atom, $R^3$ is a trifluoromethyl group, $R^6$ is a methoxy group, and $R^1$, G and n are as in the combinations shown in [Table 1] to [Table 11] ;
In the formula (1-6), compounds of the present invention wherein $R^2$ and $R^4$ are a hydrogen atom, $R^3$ is a trifluoromethyl group, $R^6$ is an ethoxy group, and $R^1$, G and n are as in the combinations shown in [Table 1] to [Table 11];
In the formula (1-6), compounds of the present invention wherein $R^2$ and $R^4$ are a hydrogen atom, $R^3$ is a trifluoromethyl group, $R^6$ is a methylsulfanyl group, and $R^1$, G and n are as in the combinations shown in [Table 1] to [Table 11];
In the formula (1-6), compounds of the present invention wherein $R^2$ and $R^4$ are a hydrogen atom, $R^3$ is a trifluoromethyl group, $R^6$ is a methylsulfonyl group, and $R^1$, G and n are as in the combinations shown in [Table 1] to [Table 11];
In the formula (1-6), compounds of the present invention wherein $R^2$ and $R^4$ are a hydrogen atom, $R^3$ is a trifluoromethyl group, $R^6$ is an methylsulfanyl group, and $R^1$, G and n are as in the combinations shown in [Table 1] to [Table 11] ;
In the formula (1-6), compounds of the present invention wherein $R^2$ and $R^4$ are a hydrogen atom, $R^3$ is a trifluoromethyl group, $R^6$ is an ethylsulfonyl group, and $R^1$, G and n are as in the combinations shown in [Table 1] to [Table 11];
In the formula (1-6), compounds of the present invention wherein $R^2$ and $R^4$ are a hydrogen atom, $R^3$ is a trifluoromethyl group, $R^6$ is a methylamino group, and $R^1$, G and n are as in the combinations shown in [Table 1] to [Table 11] ;
In the formula (1-6), compounds of the present invention wherein $R^2$ and $R^4$ are a hydrogen atom, $R^3$ is a trifluoromethyl group, $R^6$ is a dimethylamino group, and $R^1$, G and n are as in the combinations shown in [Table 1] to [Table 11];
In the formula (1-6), compounds of the present invention wherein $R^2$ and $R^4$ are a hydrogen atom, $R^3$ is a trifluoromethyl group, $R^6$ is a diethylamino group, and $R^1$, G and n are as in the combinations shown in [Table 1] to [Table 11];
In the formula (1-6), compounds of the present invention wherein $R^2$ and $R^4$ are a hydrogen atom, $R^3$ is a trifluoromethyl group, $R^6$ is a trifluoromethyl group, and $R^1$, G and n are as in the combinations shown in [Table 1] to [Table 11];
In formula (1-7),

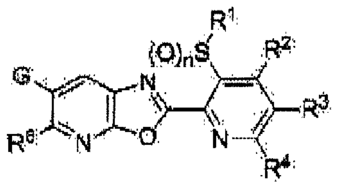

(1-7)

compounds of the present invention wherein $R^2$, $R^3$ and $R^4$ are a hydrogen atom, $R^6$ is a fluorine atom, and $R^1$, G and n are as in the combinations shown in [Table 1] to [Table 11];
In the formula (1-7), compounds of the present invention wherein $R^2$, $R^3$ and $R^4$ are a hydrogen atom, $R^6$ is a chlorine atom, and $R^1$, G and n are as in the combinations shown in [Table 1] to [Table 11];
In the formula (1-7), compounds of the present invention wherein $R^2$, $R^3$ and $R^4$ are a hydrogen atom, $R^6$ is a bromine atom, and $R^1$, G and n are as in the combinations shown in [Table 1] to [Table 11];
In the formula (1-7), compounds of the present invention wherein $R^2$, $R^3$ and $R^4$ are a hydrogen atom, $R^6$ is an iodine atom, and $R^1$, G and n are as in the combinations shown in [Table 1] to [Table 11];
In the formula (1-7), compounds of the present invention wherein $R^2$, $R^3$ and $R^4$ are a hydrogen atom, $R^6$ is a methoxy group, and $R^1$, G and n are as in the combinations shown in [Table 1] to [Table 11];
In the formula (1-7), compounds of the present invention wherein $R^2$, $R^3$ and $R^4$ are a hydrogen atom, $R^6$ is an ethoxy group, and $R^1$, G and n are as in the combinations shown in [Table 1] to [Table 11];
In the formula (1-7), compounds of the present invention wherein $R^2$, $R^3$ and $R^4$ are a hydrogen atom, $R^6$ is a methylsulfanyl group, and $R^1$, G and n are as in the combinations shown in [Table 1] to [Table 11] ;
In the formula (1-7), compounds of the present invention wherein $R^2$, $R^3$ and $R^4$ are a hydrogen atom, $R^6$ is a methylsulfonyl group, and $R^1$, G and n are as in the combinations shown in [Table 1] to [Table 11] ;
In the formula (1-7), compounds of the present invention wherein $R^2$, $R^3$ and $R^4$ are a hydrogen atom, $R^6$ is an ethylsulfanyl group, and $R^1$, G and n are as in the combinations shown in [Table 1] to [Table 11];
In the formula (1-7), compounds of the present invention wherein $R^2$ , $R^3$ and $R^4$ are a hydrogen atom, $R^6$ is an ethylsulfonyl group, and $R^1$, G and n are as in the combinations shown in [Table 1] to [Table 11];
In the formula (1-7), compounds of the present invention wherein $R^2$, $R^3$ and $R^4$ are a hydrogen atom, $R^6$ is a methylamino group, and $R^1$, G and n are as in the combinations shown in [Table 1] to [Table 11];
In the formula (1-7), compounds of the present invention wherein $R^2$, $R^3$ and $R^4$ are a hydrogen atom, $R^6$ is a dimethylamino group, and $R^1$, G and n are as in the combinations shown in [Table 1] to [Table 11];
In the formula (1-7), compounds of the present invention wherein $R^2$, $R^3$ and $R^4$ are a hydrogen atom, $R^6$ is a diethylamino group, and $R^1$, G and n are as in the combinations shown in [Table 1] to [Table 11];
In the formula (1-7), compounds of the present invention wherein $R^2$, $R^3$ and $R^4$ are a hydrogen atom, $R^6$ is a trifluoromethyl group, and $R^1$, G and n are as in the combinations shown in [Table 1] to [Table 11];
In the formula (1-7), compounds of the present invention wherein $R^2$ and $R^4$ are a hydrogen atom, $R^3$ is a chlorine atom, $R^6$ is a fluorine atom, and $R^1$, G and n are as in the combinations shown in [Table 1] to [Table 11];
In the formula (1-7), compounds of the present invention wherein $R^2$ and $R^4$ are a hydrogen atom, $R^3$ is a chlorine atom, $R^6$ is a chlorine atom, and $R^1$, G and n are as in the combinations shown in [Table 1] to [Table 11] ;
In the formula (1-7), compounds of the present invention wherein $R^2$ and $R^4$ are a hydrogen atom, $R^3$ is a chlorine atom, $R^6$ is a bromine atom, and $R^1$, G and n are as in the combinations shown in [Table 1] to [Table 11];
If the formula (1-7), compounds of the present invention wherein $R^2$ and $R^4$ are a hydrogen atom, $R^3$ is a chlorine atom, $R^6$ is an iodine atom, and $R^1$, G and n are as in the combinations shown in [Table 1] to [Table 11];
In the formula (1-7), compounds of the present invention wherein $R^2$ and $R^4$ are a hydrogen atom, $R^3$ is a chlorine atom, $R^6$ is a methoxy group, and $R^1$, G and n are as in the combinations shown in [Table 1] to [Table 11];
In the formula (1-7), compounds of the present invention wherein $R^2$ and $R^4$ are a hydrogen atom, $R^3$ is a chlorine atom, $R^6$ is an ethoxy group, and $R^1$, G and n are as in the combinations shown in [Table 1] to [Table 11] ;
In the formula (1-7), compounds of the present invention wherein $R^2$ and $R^4$ are a hydrogen atom, $R^3$ is a chlorine atom, $R^6$ is a methylsulfanyl group, and $R^1$, G and n are as in the combinations shown in [Table 1] to [Table 11];
In the formula (1-7), compounds of the present invention wherein $R^2$ and $R^4$ are a hydrogen atom, $R^3$ is a chlorine atom, $R^6$ is a methylsulfonyl group, and $R^1$, G and n are as in the combinations shown in [Table 1] to [Table 11] ;
In the formula (1-7), compounds of the present invention wherein $R^2$ and $R^4$ are a hydrogen atom, $R^3$ is a chlorine atom, $R^6$ is an ethylsulfanyl group, and $R^1$, G and n are as in the combinations shown in [Table 1] to [Table 11] ;
In the formula (1-7), compounds of the present invention wherein $R^2$ and $R^4$ are a hydrogen atom, $R^3$ is a chlorine atom, $R^6$ is an ethylsulfonyl group, and $R^1$, G and n are as in the combinations shown in [Table 1] to [Table 11] ;
In the formula (1-7), compounds of the present invention wherein $R^2$ and $R^4$ are a hydrogen atom, $R^3$ is a chlorine atom, $R^6$ is a methylamino group, and $R^1$, G and n are as in the combinations shown in [Table 1] to [Table 11] ;
In the formula (1-7), compounds of the present invention where in $R^2$ and $R^4$ are a hydrogen atom, $R^3$ is a chlorine atom,

$R^6$ is a dimethylamino group, and $R^1$, G and n are as in the combinations shown in [Table 1] to [Table 11];
In the formula (1-7), compounds of the present invention wherein $R^2$ and $R^4$ are a hydrogen atom, $R^3$ is a chlorine atom, $R^6$ is a diethylamino group, and $R^1$, G and n are as in the combinations shown in [Table 1] to [Table 11];
In the formula (1 7), compounds of the present invention wherein $R^2$ and $R^4$ are a hydrogen atom, $R^3$ is a chlorine atom, $R^6$ is a trifluoromethyl group, and $R^1$, G and n are as in the combinations shown in [Table 1] to [Table 11];
In the formula (1-7), compounds of the present invention wherein $R^2$ and $R^4$ are a hydrogen atom, $R^3$ is a trifluoromethyl group, $R^6$ is a fluorine atom, and $R^1$, G and n are as in the combinations shown in [Table 1] to [Table 11];
In the formula (1-7), compounds of the present invention wherein $R^2$ and $R^4$ are a hydrogen atom, $R^3$ is a trifluoromethyl group, $R^6$ is a chlorine atom, and $R^1$, G and n are as in the combinations shown in [Table 1] to [Table 11] ;
In the formula (1-7), compounds of the present invention wherein $R^2$ and $R^4$ are a hydrogen atom, $R^3$ is a trifluoromethyl group, $R^6$ is a bromine atom, and $R^1$, G and n are as in the combinations shown in [Table 1] to [Table 11];
In the formula (1-7), compounds of the present invention wherein $R^2$ and $R^4$ are a hydrogen atom, $R^3$ is a trifluoromethyl group, $R^6$ is an iodine atom, and $R^1$, G and n are as in the combinations shown in [Table 1] to [Table 11];
In the formula (1-7), compounds of the present invention wherein $R^2$ and $R^4$ are a hydrogen atom, $R^3$ is a trifluoromethyl group, $R^6$ is a methoxy group, and $R^1$, G and n are as in the combinations shown in [Table 1] to [Table 11];
In the formula (1-7), compounds of the present invention wherein $R^2$ and $R^4$ are a hydrogen atom, $R^3$ is a trifluoromethyl group, $R^6$ is an ethoxy group, and $R^1$, G and n are as in the combinations shown in [Table 1] to [Table 11];
In the formula (1-7), compounds of the present invention wherein $R^2$ and $R^4$ are a hydrogen atom, $R^3$ is a trifluoromethyl group, $R^6$ is a methylsulfanyl group, and $R^1$, G and n are as in the combinations shown in [Table 1] to [Table 11] ;
In the formula (1-7), compounds of the present invention wherein $R^2$ and $R^4$ are a hydrogen atom, $R^3$ is a trifluoromethyl group, $R^6$ is a methylsulfonyl group, and $R^1$, G and n are as in the combinations shown in [Table 1] to [Table 11];
In the formula (1-7), compounds of the present invention wherein $R^2$ and $R^4$ are a hydrogen atom, $R^3$ is a trifluoromethyl group, $R^6$ is an ethylsulfanyl group, and $R^1$, G and n are as in the combinations shown in [Table 1] to [Table 11];
In the formula (1-7), compounds of the present invention wherein $R^2$ and $R^4$ are a hydrogen atom, $R^3$ is a trifluoromethyl group, $R^6$ is an ethylsulfonyl group, and $R^1$, G and n are as in the combinations shown in [Table 1] to [Table 11];
In the formula (1-7), compounds of the present invention wherein $R^2$ and $R^4$ are a hydrogen atom, $R^3$ is a trifluoromethyl group, $R^5$ is a methylamino group, and $R^1$, G and n are as in the combinations shown in [Table 1] to [Table 11];
In the formula (1-7), compounds of the present invention wherein $R^2$ and $R^4$ are a hydrogen atom, $R^3$ is a trifluoromethyl group, $R^6$ is a dimethylamino group, and $R^1$, G and n are as in the combinations shown in [Table 1] to [Table 11] ;
In the formula (1-7), compounds of the present invention wherein $R^2$ and $R^4$ are a hydrogen atom, $R^3$ is a trifluoromethyl group, $R^6$ is a diethylamino group, and $R^1$, G and n are as in the combinations shown in [Table 1] to [Table 11];
In the formula (1-7), compounds of the present invention wherein $R^2$ and $R^4$ are a hydrogen atom, $R^3$ is a trifluoromethyl group, $R^6$ is a trifluoromethyl group, and $R^1$, G and n are as in the combinations shown in [Table 1] to [Table 11];
In formula (1-3),

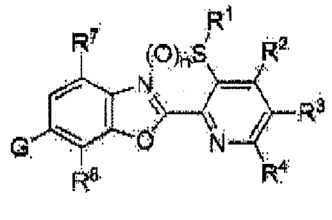

(1-3)

compounds of the present invention wherein $R^2$, $R^3$, $R^4$, $R^7$ and $R^8$ are a hydrogen atom, and $R^1$, G and n are as in the combinations shown in [Table 1] to [Table 11];
In the formula (1-3), compounds of the present invention wherein $R^2$, $R^3$, $R^4$ and $R^8$ are a hydrogen atom, $R^7$ is a fluorine atom, and $R^1$ G and n are as in the combinations shown in [Table 1] to [Table 11];
In the formula (1-3), compounds of the present invention wherein $R^1$, $R^3$, $R^4$ and $R^8$ are a hydrogen atom, $R^7$ is a chlorine atom, and $R^1$, G and n are as in the combinations shown in [Table 1] to [Table 11];
In the formula (1-3), compounds of the present invention wherein $R^2$, $R^3$, $R^4$ and $R^8$ are a hydrogen atom, $R^7$ is a bromine atom, and $R^1$, G and n are as in the combinations shown in [Table 1] to [Table 11];
In the formula (1-3), compounds of the present invention wherein $R^2$, $R^3$, $R^4$ and $R^8$ are a hydrogen atom, $R^7$ is an iodine atom, and $R^1$, G and n are as in the combinations shown in [Table 1] to [Table 11];
In the formula (1-3), compounds of the present invention wherein $R^2$, $R^3$, $R^4$ and $R^8$ are a hydrogen atom, $R^7$ is a methoxy group, and $R^1$, G and n are as in the combinations shown in [Table 1] to [Table 11];
In the formula (1-3), compounds of the present invention wherein $R^2$, $R^3$, $R^4$ and $R^8$ are a hydrogen atom, $R^7$ is an ethoxy group, and $R^1$, G and n are as in the combinations shown in [Table 1] to [Table 11];
In the formula (1-3), compounds of the present invention wherein $R^2$, $R^3$, $R^4$ and $R^8$ are a hydrogen atom, $R^7$ is a

methylsulfanyl group, and $R^1$, G and n are as in the combinations shown in [Table 1] to [Table 11];
In the formula (1-3), compounds of the present invention wherein $R^2$, $R^3$, $R^4$ and $R^8$ are a hydrogen atom, $R^7$ is a methylsulfonyl group, and $R^1$, G and n are as in the combinations shown in [Table 1] to [Table 11];
In the formula (1-3), compounds of the present invention wherein $R^2$, $R^3$, $R^4$ and $R^8$ are a hydrogen atom, $R^7$ is an ethylsulfanyl group, and $R^1$, G and n are as in the combinations shown in [Table 1] to [Table 11];
In the formula (1-3), compounds of the present invention wherein $R^2$, $R^3$, $R^4$ and $R^8$ are a hydrogen atom, $R^7$ is an ethylsulfonyl group, and $R^1$, G and n are as in the combinations shown in [Table 1] to [Table 11];
In the formula (1-3), compounds of the present invention wherein $R^2$, $R^3$, $R^4$ and $R^8$ are a hydrogen atom, $R^7$ is a methylamino group, and $R^1$, G and n are as in the combinations shown in [Table 1] to [Table 11];
In the formula (1-3), compounds of the present invention wherein $R^2$, $R^3$, $R^4$ and $R^8$ are a hydrogen atom, $R^7$ is a dimethylamino group, and $R^1$, G and n are as in the combinations shown in [Table 1] to [Table 11];
In the formula (1-3), compounds of the present invention wherein $R^2$, $R^3$, $R^4$ and $R^8$ are a hydrogen atom, $R^7$ is a diethylamino group, and $R^1$, G and n are as in the combinations shown in [Table 1] to [Table 11];
In the formula (1-3), compounds of the present invention wherein $R^2$, $R^3$, $R^4$ and $R^8$ are a hydrogen atom, $R^7$ is a trifluoromethyl group, and $R^1$, G and n are as in the combinations shown in [Table 1] to [Table 11];
In the formula (1-3), compounds of the present invention wherein $R^2$, $R^4$, $R^7$ and $R^8$ are a hydrogen atom, $R^3$ is a chlorine atom, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a chlorine atom, $R^7$ is a fluorine atom, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a chlorine atom, $R^7$ is a chlorine atom, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a chlorine atom, $R^7$ is a bromine atom, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a chlorine atom, $R^7$ is an iodine atom, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a chlorine atom, $R^7$ is a methoxy group, and $R^1$, G and n are as in the combinations shown in [Table 1] to [Table 11];
In the formula (1 3), compounds of the present invention wherein $R^2$, $R^4$ and $R^8$ are a hydrogen atom, $R^3$ is a chlorine atom, $R^7$ is an ethoxy group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a chlorine atom, $R^7$ is a methylsulfanyl group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a chlorine atom, $R^7$ is a methylsulfonyl group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a chlorine atom, $R^7$ is an methylsulfanyl group, and $R^1$, G and n are as in the combinations shown in [Table 1] to [Table 11];
In the formula (1-3), compounds of the present invention where in $R^2$, $R^4$ and $R^8$ are a hydrogen atom, $R^3$ is a chlorine atom, $R^7$ is an ethylsulfonyl group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a chlorine atom, $R^7$ is a methylamino group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a chlorine atom, $R^7$ is a dimethylamino group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a chlorine atom, $R^7$ is a diethylamino group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a chlorine atom, $R^7$ is a trifluoromethyl group, and $R^1$, G and n are as in the combinations shown in [Table 1] to [Table 11];
In the formula (1-3), compounds of the present invention wherein $R^2$, $R^4$, $R^7$ and $R^8$ are a hydrogen atom, $R^3$ is a trifluoromethyl group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a trifluoromethyl group, $R^7$ is a fluorine atom, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a trifluoromethyl group, $R^7$ is a chlorine atom, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a trifluoromethyl group, $R^7$ is a bromine atom, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a trifluoromethyl group, $R^7$ is an iodine atom, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a trifluoromethyl group, $R^7$ is a methoxy group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a trifluoromethyl

group, $R^7$ is an ethoxy group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a trifluoromethyl group, $R^7$ is a methylsulfanyl group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a trifluoromethyl group, $R^7$ is a methylsulfonyl group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a trifluoromethyl group, $R^7$ is an ethylsulfanyl group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a trifluoromethyl group, $R^7$ is an ethylsulfonyl group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a trifluoromethyl group, $R^7$ is a methylamino group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a trifluoromethyl group, $R^7$ is a dimethylamino group, and $R^1$, G and n are as in the combinations shown in [Table 1] to [Table 11];
In the formula (1-3), compounds of the present invention wherein $R^2$, $R^4$ and $R^8$ are a hydrogen atom, $R^3$ is a trifluoromethyl group, $R^7$ is a diethylamino group, and $R^1$, G and n are as in the combinations shown in [Table 1] to [Table 11];
In the formula (1-3) compounds of the present invention wherein $R^2$, $R^4$ and $R^8$ are a hydrogen atom, $R^3$ is a trifluoromethyl group, $R^7$ is a trifluoromethyl group, and $R^1$, G and n are as in the combinations shown in [Table 1] to [Table 11];
In formula (1-4),

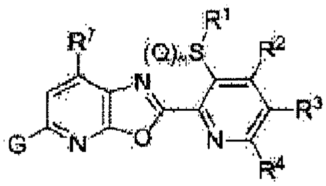
(1-4)

compounds of the present invention wherein $R^2$, $R^3$, $R^4$ and $R^7$ are a hydrogen atom, and $R^1$, G and n are as in the combinations shown in [Table 1] to [Table 11];
In the formula (1-4), compounds of the present invention wherein $R^2$, $R^3$ and $R^4$ are a hydrogen atom, $R^7$ is a fluorine atom, and $R^1$, G and n are as in the combinations shown in [Table 1] to [Table 11];
In the formula (1-4), compounds of the present invention wherein $R^1$, $R^3$ and $R^4$ are a hydrogen atom, $R^7$ is a chlorine atom, and $R^1$, G and n are as in the combinations shown in [Table 1] to [Table 11];
In the formula (1-4), compounds of the present invention wherein $R^2$, $R^3$ and $R^4$ are a hydrogen atom, $R^7$ is a bromine atom, and $R^1$, G and n are as in the combinations shown in [Table 1] to [Table 11];
In the formula (1-4), compounds of the present invention wherein $R^2$, $R^3$ and $R^4$ are a hydrogen atom, $R^7$ is an iodine atom, and $R^1$, G and n are as in the combinations shown in [Table 1] to [Table 11];
In the formula (1-4), compounds of the present invention wherein $R^2$, $R^3$ and $R^4$ are a hydrogen atom, $R^7$ is a methoxy group, and $R^1$, G and n are as in the combinations shown in [Table 1] to [Table 11];
In the formula (1-4), compounds of the present invention where in $R^2$, $R^3$ and $R^4$ are a hydrogen atom, $R^7$ is an ethoxy group, and $R^1$, G and n are as in the combinations shown in [Table 1] to [Table 11];
In the formula (1-4), compounds of the present invention wherein $R^2$, $R^3$ and $R^4$ are a hydrogen atom, $R^7$ is a methylsulfanyl group, and $R^1$, G and n are as in the combinations shown in [Table 1] to [Table 11];
In the formula (1-4), compounds of the present inventionwherein $R^2$, $R^3$ and $R^4$ are a hydrogen atom, $R^1$ is a methylsulfonyl group, and $R^1$, G and n are as in the combinations shown in [Table 1] to [Table 11];
In the formula (1-4), compounds of the present invention wherein $R^2$, $R^3$ and $R^4$ are a hydrogen atom, $R^7$ is an ethylsulfanyl group, and $R^1$, G and n are as in the combinations shown in [Table 1] to [Table 11];
In the formula (1-4), compounds of the present invention wherein $R^2$, $R^3$ and $R^4$ are a hydrogen atom, $R^7$ is an ethylsulfonyl group, and $R^1$, G and n are as in the combinations shown in [Table 1] to [Table 11];
In the formula (1 4), compounds of the present invention wherein $R^2$, $R^3$ and $R^4$ are a hydrogen atom, $R^7$ is a methylamino group, and $R^1$, G and n are as in the combinations shown in [Table 1] to [Table 11];
In the formula (1-4), compounds of the present invention wherein $R^2$, $R^3$ and $R^4$ are a hydrogen atom, $R^7$ is a dimethylamino group, and $R^1$, G and n are as in the combinations shown in [Table 1] to [Table 11];
In the formula (1-4), compounds of the present invention wherein $R^2$, $R^3$ and $R^4$ are a hydrogen atom, $R^7$ is a diethylamino group, and $R^1$, G and n are as in the combinations shown in [Table 1] to [Table 11];
In the formula (1-4), compounds of the present invention wherein $R^2$, $R^3$ and $R^4$ are a hydrogen atom, $R^7$ is a trifluoromethyl group, and $R^1$, G and n are as in the combinations shown in [Table 1] to [Table 11];
In the formula (1-4), compounds of the present invention wherein $R^2$, $R^4$ and $R^7$ are a hydrogen atom, $R^3$ is a chlorine atom, and $R^1$, G and n are as in the combinations shown in [Table 1] to [Table 11];

In the formula (1-4), compounds of the present invention wherein $R^2$ and $R^4$ are a hydrogen atom, $R^3$ is a chlorine atom, $R^7$ is a fluorine atom, and $R^1$, G and n are as in the combinations shown in [Table 1] to [Table 11];
In the formula (1-4), compounds of the present invention wherein $R^2$ and $R^4$ are a hydrogen atom, $R^3$ is a chlorine atom, $R^7$ is a chlorine atom, and $R^1$, G and n are as in the combinations shown in [Table 1] to [Table 11];
In the formula (1-4), compounds of the present invention wherein $R^2$ and $R^4$ are a hydrogen atom, $R^3$ is a chlorine atom, $R^7$ is a bromine atom, and $R^1$, G and n are as in the combinations shown in [Table 1] to [Table 11];
In the formula (1-4), compounds of the present invention wherein $R^2$ and $R^4$ are a hydrogen atom, $R^3$ is a chlorine atom, $R^7$ is an iodine atom, and $R^1$, G and n are as in the combinations shown in [Table 1] to [Table 11];
In the formula (1-4), compounds of the present invention wherein $R^2$ and $R^4$ are a hydrogen atom, $R^3$ is a chloride atom, $R^7$ is a methoxy group, and $R^1$, G and n are as in the combinations shown in [Table 1] to [Table 11];
In the formula (1-4), compounds of the present invention wherein $R^2$ and $R^4$ are a hydrogen atom, $R^3$ is a chlorine atom, $R^7$ is an ethoxy group, and $R^1$, G and n are as in the combinations shown in [Table 1] to [Table 11];
In the formula (1-4), compounds of the present invention wherein $R^2$ and $R^4$ are a hydrogen atom, $R^3$ is a chlorine atom, $R^7$ is a methylsulfanyl group, and $R^1$, G and n are as in the combinations shown in [Table 1] to [Table 11];
In the formula (1-4), compounds of the present invention wherein $R^2$ and $R^4$ are a hydrogen atom, $R^3$ is a chlorine atom, $R^7$ is a methylsulfonyl group, and $R^1$, G and n are as in the combinations shown in [Table 1] to [Table 11];
In the formula (1-4), compounds of the present invention wherein $R^2$ and $R^4$ are a hydrogen atom, $R^3$ is a chlorine atom, $R^7$ is an ethylsulfanyl group, and $R^1$, G and n are as in the combinations shown in [Table 1] to [Table 11];
In the formula (1-4), compounds of the present invention wherein $R^2$ and $R^4$ are a hydrogen atom, $R^3$ is a chlorine atom, $R^7$ is an ethylsulfonyl group, and $R^1$, G and n are as in the combinations shown in [Table 1] to [Table 11];
In the formula (1-4), compounds of the present invention wherein $R^2$ and $R^4$ are a hydrogen atom, $R^3$ is a chlorine atom, $R^7$ is a methylamino group, and $R^1$, G and n are as in the combinations shown in [Table 1] to [Table 11];
In the formula (1-4), compounds of the present invention wherein $R^2$ and $R^4$ are a hydrogen atom, $R^3$ is a chlorine atom, $R^7$ is a dimethylamino group, and $R^1$, G and n are as in the combinations shown in [Table 1] to [Table 11];
In the formula (1-4), compounds of the present invention wherein $R^2$ and $R^4$ are a hydrogen atom, $R^3$ is a chlorine atom, $R^7$ is a diethylamino group, and $R^1$, G and n are as in the combinations shown in [Table 1] to [Table 11];
In the formula (1-4), compounds of the present invention wherein $R^2$ and $R^4$ are a hydrogen atom, $R^3$ is a chlorine atom, $R^7$ is a trifluoromethyl group, and $R^1$, G and n are as in the combinations shown in [Table 1] to [Table 11];
In the formula (1-4), compounds of the present invention wherein $R^2$, $R^4$ and $R^7$ are a hydrogen atom, $R^3$ is a trifluoromethyl group, and $R^1$, G and n are as in the combinations shown in [Table 1] to [Table 11];
In the formula (1-4), compounds of the present invention wherein $R^2$ and $R^4$ are a hydrogen atom, $R^3$ is a trifluoromethyl group, $R^7$ is a fluorine atom, and $R^1$, G and n are as in the combinations shown in [Table 1] to [Table 11];
In the formula (1-4), compounds of the present invention wherein $R^2$ and $R^4$ are a hydrogen atom, $R^3$ is a trifluoromethyl group, $R^7$ is a chlorine atom, and $R^1$, G and n are as in the combinations shown in [Table 1] to [Table 11];
In the formula (1-4), compounds of the present invention wherein $R^2$ and $R^4$ are a hydrogen atom, $R^3$ is a trifluoromethyl group, $R^7$ is a bromine atom, and $R^1$, G and n are as in the combinations shown in [Table 1] to [Table 11];
In the formula (1-4), compounds of the present invention wherein $R^2$ and $R^4$ are a hydrogen atom, $R^3$ is a trifluoromethyl group, $R^7$ is an iodine atom, and $R^1$, G and n are as in the combinations shown in [Table 1] to [Table 11];
In the formula (1-4), compounds of the present invention wherein $R^2$ and $R^4$ are a hydrogen atom, $R^3$ is a trifluoromethyl group, $R^7$ is a methoxy group, and $R^1$, G and n are as in the combinations shown in [Table 1] to [Table 11];
In the formula (1 4), compounds of the present invention wherein $R^2$ and $R^4$ are a hydrogen atom, $R^3$ is a trifluoromethyl group, $R^7$ is an ethoxy group, and $R^1$, G and n are as in the combinations shown in [Table 1] to [Table 11];
In the formula (1-4), compounds of the present invention wherein $R^2$ and $R^4$ are a hydrogen atom, $R^3$ is a trifluoromethyl group, $R^7$ is a methylsulfanyl group, and $R^1$, G and n are as in the combinations shown in [Table 1] to [Table 11];
In the formula (1-4), compounds of the present invention wherein $R^2$ and $R^4$ are a hydrogen atom, $R^3$ is a trifluoromethyl group, $R^7$ is a methylsulfonyl group, and $R^1$, G and n are as in the combinations shown in [Table 1] to [Table 11];
In the formula (1-4), compounds of the present invention wherein $R^2$ and $R^4$ are a hydrogen atom, $R^3$ is a trifluoromethyl group, $R^7$ is an ethylsulfanyl group, and $R^1$, G and n are as in the combinations shown in [Table 1] to [Table 11];
In the formula (1-4), compounds of the present invention wherein $R^2$ and $R^4$ are a hydrogen atom, $R^3$ is a trifluoromethyl group, $R^7$ is an ethylsulfonyl group, and $R^1$, G and n are as in the combinations shown in [Table 1] to [Table 11];
In the formula (1-4), compounds of the present invention wherein $R^2$ and $R^4$ are a hydrogen atom, $R^3$ is a trifluoromethyl group, $R^7$ is a methylamino group, and $R^1$, G and n are as in the combinations shown in [Table 1] to [Table 11];
In the formula (1-4), compounds of the present invention wherein $R^2$ and $R^4$ are a hydrogen atom, $R^3$ is a trifluoromethyl group, $R^7$ is a dimethylamino group, and $R^1$, G and n are as in the combinations shown in [Table 1] to [Table 11];
In the formula (1-4), compounds of the present invention wherein $R^2$ and $R^4$ are a hydrogen atom, $R^3$ is a trifluoromethyl group, $R^7$ is a diethylamino group, and $R^1$, G and n are as in the combinations shown in [Table 1] to [Table 11];
In the formula (1-4), compounds of the present invention wherein $R^2$ and $R^4$ are a hydrogen atom, $R^3$ is a trifluoromethyl group, $R^7$ is a trifluoromethyl group, and $R^1$, G and n are as in the combinations shown in [Table 1] to [Table 11];

In formula (1C-1)

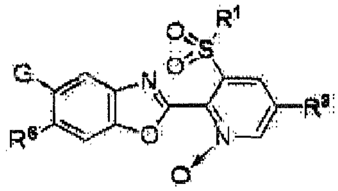

(1C-1)

compounds of the present invention wherein $R^3$ is a hydrogen atom, $R^6$ is a fluorine atom, and $R^1$ and G are as in the combinations of $R^1$ and G shown in [Table 8] to [Table 11];
In the formula (1C-1), compounds of the present invention wherein $R^3$ is a hydrogen atom, $R^6$ is a chlorine atom, and $R^1$ and G are as in the combinations of $R^1$ and G shown in [Table 8] to [Table 11];
In the formula (1C-1), compounds of the present invention wherein $R^3$ is a hydrogen atom, $R^6$ is a bromine atom, and $R^1$ and G are as in the combinations of $R^1$ and G shown in [Table 8] to [Table 11];
In the formula (1C-1), compounds of the present invention wherein $R^3$ is a hydrogen atom, $R^6$ is an iodine atom, and $R^1$ and G are as in the combinations of $R^1$ and G shown in [Table 8] to [Table 11];
In the formula (1C-1), compounds of the present invention wherein $R^3$ is a hydrogen atom, $R^6$ is a methoxy group, and $R^1$ and G are as in the combinations of $R^1$ and G shown in [Table 8] to [Table 11];
In the formula (1C-1), compounds of the present invention wherein $R^3$ is a hydrogen atom, $R^6$ is an ethoxy group, and $R^1$ and G are as in the combinations of $R^1$ and G shown in [Table 8] to [Table 11];
In the formula (1C-1), compounds of the present invention wherein $R^3$ is a hydrogen atom, $R^6$ is a methylsulfanyl group, and $R^1$ and G are as in the combinations of $R^1$ and G shown in [Table 8] to [Table 11];
In the formula (1C-1), compounds of the present invention wherein $R^3$ is a hydrogen atom, $R^6$ is a methylsulfonyl group, and $R^1$ and G are as in the combinations of $R^1$ and G shown in [Table 8] to [Table 11];
In the formula (1C-1), compounds of the present invention wherein $R^3$ is a hydrogen atom, $R^6$ is an ethylsulfanyl group, and $R^1$ and G are as in the combinations of $R^1$ and G shown in [Table 8] to [Table 11];
In the formula (1C-1), compounds of the present invention wherein $R^3$ is a hydrogen atom, $R^6$ is an ethylsulfonyl group, and $R^1$ and G are as in the combinations of $R^1$ and G shown in [Table 8] to [Table 11];
In the formula (1C-1), compounds of the present invention wherein $R^3$ is a hydrogen atom, $R^6$ is a methylamino group, and $R^1$ and G are as in the combinations of $R^1$ and G shown in [Table 8] to [Table 11];
In the formula (1C-1), compounds of the present invention wherein $R^3$ is a hydrogen atom, $R^6$ is a dimethylamino group, and $R^1$ and G are as in the combinations of $R^1$ and G shown in [Table 8] to [Table 11];
In the formula (1C-1), compounds of the present invention wherein $R^3$ is a hydrogen atom, $R^6$ is a diethylamino group, and $R^1$ and G are as in the combinations of $R^1$ and G shown in [Table 8] to [Table 11];
In the formula (IC-1), compounds of the present invention wherein $R^3$ is a hydrogen atom, $R^6$ is a trifluoromethyl group, and $R^1$ and G are as in the combinations of $R^1$ and G shown in [Table 8] to [Table 11];
In formula (1C-2)

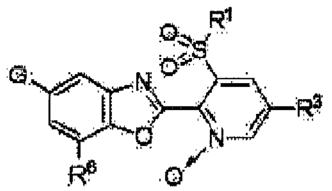

(1C-2)

compounds of the present invention wherein $R^3$ is a hydrogen atom, $R^6$ is a fluorine atom, and $R^1$ and G are as in the combinations of $R^1$ and G shown in [Table 8] to [Table 11];
In the formula (1C-2), compounds of the present invention wherein $R^3$ is a hydrogen atom, $R^6$ is a chlorine atom, and $R^1$ and G are as in the combinations of $R^1$ and G shown in [Table 8] to [Table 11];
In the formula (1C-2), compounds of the present invention wherein $R^3$ is a hydrogen atom, $R^6$ is a bromine atom, and $R^1$ and G are as in the combinations of $R^1$ and G shown in [Table 8] to [Table 11];
In the formula (1C-2), compounds of the present invention wherein $R^3$ is a hydrogen atom, $R^6$ is an iodine atom, and $R^1$ and G are as in the combinations of $R^1$ and G shown in [Table 8] to [Table 11];
In the formula (1C-2), compounds of the present invention wherein $R^3$ is a hydrogen atom, $R^6$ is a methoxy group, and $R^1$ and G are as in the combinations of $R^1$ and G shown in [Table 8] to [Table 11];

In the formula (1C-2), compounds of the present invention wherein $R^3$ is a hydrogen atom, $R^6$ is an ethoxy group, and $R^1$ and G are as in the combinations of $R^1$ and G shown in [Table 8] to [Table 11];
In the formula (1C-2), compounds of the present invention wherein $R^3$ is a hydrogen atom, $R^6$ is a methylsulfanyl group, and $R^1$ and G are as in the combinations of $R^1$ and G shown in [Table 8] to [Table 11];
In the formula (1C-2), compounds of the present invention wherein $R^3$ is a hydrogen atom, $R^6$ is a methylsulfonyl group, and $R^1$ and G are as in the combinations of $R^1$ and G shown in [Table 8] to [Table 11];
In the formula (1C-2), compounds of the present invention wherein $R^3$ is a hydrogen atom, $R^6$ is an ethylsulfanyl group, and $R^1$ and G are as in the combinations of $R^1$ and G shown in [Table 8] to [Table 11];
In the formula (1C-2), compounds of the present invention wherein $R^3$ is a hydrogen atoms, $R^6$ is an ethylsulfonyl group, and $R^1$ and G are as in the combinations of $R^1$ and G shown in [Table 8] to [Table 11];
In the formula (1C-2), compounds of the present invention wherein $R^3$ is a hydrogen atom, $R^6$ is a methylamino group, and $R^1$ and G are as in the combinations of $R^1$ and G shown in [Table 8] to [Table 11];
In the formula (1C-2), compounds of the present invention wherein $R^3$ is a hydrogen atom, $R^6$ is a dimethylamino group, and $R^1$ and G are as in the combinations of $R^1$ and G shown in [Table 8] to [Table 11];
In the formula (1C-2), compounds of the present invention wherein $R^3$ is a hydrogen atom, $R^6$ is a diethylamino group, and $R^1$ and G are as in the combinations of $R^1$ and G shown in [Table 8] to [Table 11];
In the formula (1C-2), compounds of the present invention wherein $R^3$ is a hydrogen atom, $R^6$ is a trifluoromethyl group, and $R^1$ and G are as in the combinations of $R^1$ and G shown in [Table 8] to [Table 11].

Examples of the pest on which the compound of the present invention has an effect include arthropod pests such as pest insects and pest mites and nematoda. Specifically, examples of the pests include those shown below.

Hemiptera: Delphacidae such as Laodelphax striatellus, Nilaparvata lugens, and Sogatella furcifera, Deltocephalidae such as Nephotettix cincticeps, Nephotettix virescens, and Empoasca onukii, Aphididae such as Aphis gossypii, Myzus persicae, Brevicoryne brassicae, Aphis spiraecola, Macrosiphum euphorbiae, Aulacorthum solani, Rhopalosiphum padi, Toxoptera citricidus, and Hyalopterus pruni, Pentatomidae such as Nezara antennata, Riptortus clavetus, Leptocorisa chinensis, Eysarcoris parvus, and Halyomorpha mista, Aleyrodidae such as Trialeurodes vaporariorum, Bemisia tabaci, Dialeurodes citri, and Aleurocanthus spiniferus, Coccidae such as Aonidiella aurantii, Comstockaspis perniciosa, Unaspis citri, Ceroplastes rubens, Icerya purchasi, Planococcus kraunhiae, Pseudococcus longispinis, and Pseudaulacaspis pentagona, Tingidae, Cimicoidea such as Cimex lectularius, and Psyliidae.

Lepidoptera: Pyralidae such as Chilo suppressalis, Tryporyza incertulas, Cnaphalocrocis medinalis, Notarcha derogata, Plodia interpunetella, Ostrinia furnacalis, Hellula undalis, and Pediasia teterrellus, Noctuidae such as Spodoptera litura, Spodoptera exigua, Pseudaletia separata, Mamestra brassicae, Agrotis ipsilon, Plusia nigrisigna, Thoricoplusia spp., Heliothis spp., and Helicoverpa spp., Pieridae such as Pieris rapae, Adoxophyes spp., Tortricidae such as Grapholita molesta, Leguminivora glycinivorella, Matsumuraeses azukivora, Adoxophyes orana fasciata, Adoxophyes honmai., Homona magnanima, Archips fuscocupreanus, and Cydia pomonella, Gracillariidae such as Caloptilia theivora and Phyllonorycter ringoneella, Carposinidae such as Carposina niponensis, Lyonetiidae such as Lyonetia spp., Lymantriidae such as Lymantria spp. and Euproctis spp., Yponomeutidae such as Plutella xylostella, Gelechiidae such as Pectinophora gossypiella and Phthorimaea operculella, Arctiidae such as Hyphantria cunea, and Tineidae such as Tinea translucens and Tineola bisselliella.

Thysanoptera: Thripidae such as Frankliniella occidentalis, Thrips parmi, Scirtothrips dorsalis, Thrips tabaci, and Frankliniella intonsa.

Diptera: Culex such as Culex pipiens pallens, Culex tritaeniorhynchus, and Culex quinquefasciatus, Aedes spp. such as Aedes aegypti and Aedes albopictus, Anopheles spp. such as Anopheles sinensis, Chironomidae, Muscidae such as Musca domestica and Muscina stabulans, Calliphoridae, Sarcophagidae, Fanniidae, Anthomyiidae such as Delia platura and Delia antiqua, Agromyzidae such as Agromyza oryzae, Hydrellia griseola, Liriomyza sativae, Liriomyza trifolii, and Chromatomyia horticola, Chloropidae such as Chlorops oryzae, Tephritidae such as Dacus cucurbitae and Ceratitis capitata, Drosophilidae, Phoridae such as Megaselia spiracularis, Psychodidae such as Clogmia albipunctata, Sciaridae, Simuliidae, Tabanidae such as Tabanus trigonus, Hippoboscidae, and Stomoxys.

Coleoptera: Diabrotica such as Diabrotica virgifera virgifera and Diabrotica undecimpunctata howardi, Scarabaeidae such as Anomala cuprea, Anomala rufocuprea, and Popillia japonica, Curculionidae such as Sitophilus zeamais, Lissorhoptrus oryzophilus, Callosobruchuys chienensis, Echinocnemus squameus, Anthonomus grandis, and Sphenophorus venatus, Tenebrionidae such as Tenebrio molitor and Tribolium castaneum, Chrysomelidae such as Oulema oryzae, Aulacophora femoralis, Phyllotreta striolata, and Leptinotarsa decemlineata, Dermestidae such as Anthrenus verbasci and Dermestes maculates, Anobiidae such as Lasioderma serricorne, Epilachna such as Epilachna vigintioctopunctata, Lyctus brunneus, Scolytidae such as Tomicus piniperda, Bostrychidae, Ptinidae, Cerambycidae such as Anoplophora malasiaca, Agriotes spp., and Paederus fuscipes.

Orthoptera: Locusta migratoria, Gryllotalpa africana, Oxya yezoensis, Oxya japonica, and Grylloidea.

Aphaniptera: Ctenocephalides felis, Ctenocephalides canis, Pulex irritans, and Xenopsylla cheopis.

Anoplura: Pediculus humanus carporis, Pediculus humanus humanus, Phthirus pubis, Haematopinus eurysternus, Dalmalinia ovis, Haematopinus suis, and Linognathus setosus.

Mallophaga: Dalmalinia ovis, Dalmalinia bovis, Menopon gallinae, Trichodectes canis, and Felicola subrostrata.

Hymenoptera: Formicidae such as Monomorium pharaosis, Formica fusca japonica, Ochetellus glaber, Pristomyrmex pungens, Pheidole noda, Acromyrmex spp., Solenopsis spp., and Linepithema humile, Vespidae, Bethylidae, and Tenthredinidae such as Athalia rosae and Athalia japonica.

Nematoda: Aphelenchoides besseyi, Nothotylenchus acris, Meloidogyne incognita, Meloidogyne hapla, Meloidogyne javanica, Heterodera glycines, Globodera rostochiensis, Pratylenchus coffeae, and Pratylenchus neglectus.

Blattodea: Blattella germanica, Periplaneta fuliginosa, Periplaneta americana, Periplaneta brunnea, and Blatta orientalis.

Isoptera: Reticulitermes speratus, Coptotermes formosanus, Incisitermes minor, Cryptotermes domesticus, Odontotermes formosanus, Neotermes koshunensis, Glyptotermes satsumensis, Glyptotermes nakajimai, Glyptotermes fuscus, Glyptotermes kodamai, Glyptotermes kushimensis, Hodotermopsis japonica, Captatermes guangzhoensis, Reticulitermes miyatakei, Reticulitermes flaviceps amamianus, Reticulitermes sp., Nasutitermes takasagoensis, Pericapritermes nitobei, and Sinocapritermes mushae.

Acarina: Tetranychidae such as Tetranychus urticae, Tetranychus kanzawai, Panonychus citri, Panonychus ulmi, and Oligonychus spp., Eriophyidae such as Aculops pelekassi, Phyllocoptruta citri, Aculops lycopersici, Calacarus carinatus, Acaphylla theavagrans, Eriophyes chibaensis, and Aculus schlechtendali, Tarsonemidae such as Polyphagotarsonemus latus, Tenuipalpidae such as Brevipalpus phoenicis, Tuckerellidae, Metastigmata such as Haemaphysalis longicornis, Haemaphysalis flava, Dermacentor taiwanicus, Dermacentor variabilis, Ixodes ovatus, Ixodes persulcatus, Ixodes scapularis, Amblyomma americanum, Boophilus microplus, and Rhipicephalus sanguineus, Acaridae such as Tyrophagus putrescentiae and Tyrophagus similis, Pyroglyphidae such as Dermatophagoides farinae and Dermatophagoides ptrenyssnus, Cheyletidae such as Cheyletus eruditus, Cheyletus malaccensis, Cheyletus moorei, and Cheyletiella yasguri, Sarcoptidae such as Octodectes cynotis and Sacroptes scabiei, Demodicidae such as Demodex canis, Listrophoridae, Cryptostigmata, Dermanyssidae such as Ornithonyssus bacoti, Ornithonyssus sylvairum, and Dermanyssus gallinae, Trombiculidae such as Leptotrombidium akamushi, and Arachnida such as Chiracanthium japonicum and Latrodectus hasseltii.

Chilopoda; Thereuonema hilgendorfi and Scolopendra subspinipes.

Diplopoda: Oxidus gracilis and Nedyopus tambanus.

Isopoda: Armadillidium vulgare.

Gastropoda: Limax marginatus and Limax flavus.

The pest control agent of the present invention contains the compound of the present invention and an inert carrier. The pest control agent of the present invention is usually obtained by mixing the compound of the present invention and an inert carrier such as a solid carrier, a liquid carrier or a gaseous carrier, and adding a surfactant or other auxiliaries for formulation as necessary, to be formulated into emulsifiable concentrates, oil formulations, dust formulations, granules, wettable powders, flowables, microcapsule formulations, aerosols, smoking agents, poisonous bait formulations, resin formulations, shampoo agents, paste formulations, foam agents, carbon dioxide preparations, tablets, and the like. These formulations may be processed into mosquito repellent coil, electric mosquito repellent mat, mosquito repellent liquid formulation, smoking agent, fumigant, sheet formulation, spot-on agent, or oral treatment agent, and used.

The pest control agent of the present invention usually contains the compound of the present invention in an amount of 0.01 to 95% by weight.

Examples of the solid carrier which is used in the formulation include fine powder and granules of clays (kaolin clay, diatomaceous earth, bentonite, Fubasami clay, acid clay, etc.), synthetic hydrated silicon oxide, talc, ceramics, other inorganic minerals (sericite, quartz, sulfur, activated carbon, calcium carbonate, hydrated silica, etc.), fine powder and granulated substances of chemical fertilizers (ammonium sulfate, ammonium phosphate, ammonium nitrate, urea, ammonium chloride, etc.) and the like, synthetic resins (polyester resins such as polypropylene, polyacrylonitrile, polymethylmethacrylate and polyethylene terephthalate, nylon resins such as nylon-6, nylon-11 and nylon-66, polyamide resin, polyvinyl chloride, polyvinylidene chloride, vinyl chloride-propylene copolymer, etc.).

Examples of the liquid carrier include water, alcohols (methanol, ethanol, isopropyl alcohol, butanol, hexanol, benzyl alcohol, ethylene glycol, propylene glycol, phenoxyethanol, etc.), ketones (acetone, methyl ethyl ketone, cyclohexanone, etc.), aromatic hydrocarbons (toluene, xylene, ethylbenzene, dodecylbenzene, phenylxylylethane, methylnaphthalene, etc.), aliphatic hydrocarbons (hexane, cyclohexane, kerosene, light oil, etc.), esters (ethyl acetate, butyl acetate, isopropyl myristate, ethyl oleate, diisopropyl adipate, diisobutyl adipate, propylene glycol monomethyl ether acetate, etc.), nitriles (acetonitrile, isobutyronitrile, etc.), ethers (diisopropyl ether, 1,4-dioxane, ethylene glycol dimethyl ether, diethylene glycol dimethyl ether, diethylene glycol monomethyl ether, propylene glycol monomethyl ether, dipropylene glycol monomethyl ether, 3-methoxy-3-methyl-1-butanol, etc.), acid amides (N,N-dimethylformamide, N,N-dimethylacetamide, etc.), halogenated hydrocarbons (dichloromethane, trichloroethane, carbon tetrachloride, etc.) sulfoxides (dimethyl sulfoxide, etc.), and propylene carbonate and vegetable oils (soybean oil, cottonseed oil, etc.).

Examples of the gaseous carrier include fluorocarbon, butane gas, LPG (liquefied petroleum gas), dimethyl ether, and carbon dioxide.

Examples of the surfactant include nonionic surfactants such as polyoxyethylene alkyl ether, polyoxyethylene alkylaryl ether and polyethylene glycol fatty acid ester, and anionic surfactants such as alkyl sulfonates, alkylbenzene sulfonates and alkylsulfates.

The other auxiliaries for formulation include such as fixing agents, dispersants, colorants and stabilizers, specifically, for example, casein, gelatin, polysaccharides (starch, arabic gum, cellulose derivatives, alginic acid, etc.), lignin derivatives, bentonite, synthetic water-soluble polymers (polyvinyl alcohol, polyvinyl pyrrolidone, polyacrylic acid, etc.), PAP (isopropyl acid phosphate), BHT (2,6-di-tert-butyl-4-methylphenol) and BHA (mixtures of 2-tert-butyl-4-methoxyphenol and 3-tert-butyl-4-methoxyphenol).

Examples of a base material of the resin formulation include vinyl chloride polymer, polyuethane and the like, and a plasticizer such as phthalate esters (dimethyl phthalate, dioctyl phthalate, etc.), adipate esters or stearic acid may be added to these base materials as necessary. The resin formulation is obtained by kneading a compound into the base material using an ordinary kneading apparatus, and then molding it by injection molding, extrusion molding, press molding or the like, and the resin formulation obtained can be further subjected to molding or cutting step as necessary to be processed into a plate, film, taped, reticular or string resin formulation. These resin formulations are processed into, for example, a collar for animal, an ear tag for animal, a sheet formulation, an induction cord, and a gardening pole.

Examples of a base material of the poisonous bait include grain powder, vegetable oil, sugar, crystalline cellulose and the like, and further, an antioxidant such as dibutylhydroxytoluene and nordihydroguaiaretic acid, a preservative such as dehydroacetic acid, a substance for preventing accidental ingestion by children and pets such as red pepper powder, a pest attractant flavor such as cheese flavor, onion flavor and peanut oil or the like are added as necessary.

The method for controlling pests of the present invention is carried out by applying an effective amount of the compound of the present invention to a pest directly or a pest-infested area (plants, soil, in-house, animal body, etc.). In the method for controlling pests of the present invention, the compound is usually used in the form of the pest control agent of the present invention.

When the pest control agent of the present invention is used in pest controlling in the agricultural field, the application amount is usually 1 to 10000 g in the amount of the compound of the present invention per 10000 $m^2$. When the pest control agent of the present invention is formulated into an emulsifiable concentrate, a wettable powder, a flowable or the like, the pest control agent is usually diluted with water for an application so as to have a concentration of the active ingredient of 0.01 to 10000 ppm, and dust formulations, granules and the like are usually applied as they are.

These formulations and formulation solutions diluted with water may be directly applied by being sprayed on a pest or a plant such as crops which should be protected from pests, and also may be applied to a soil in order to control a pest that infests in the soil of cultivated land.

Also, the resin formulation processed into a sheet or string can be also applied by a method such as winding it around crops, spreading it in the vicinity of crops, or spreading it to the soil around crop roots.

When the pest control agent of the present invention is used in controlling the pest that inhabits in the house, the application amount is usually 0.01 to 1000 mg in an amount of the compound of the present invention per 1 $m^2$ of an area to be treated, in the case of using it on a planar area, and is usually 0.01 to 500 mg in an amount of the compound of the present invention per 1 $m^3$ of a space to be treated, in the case of using it in a space. When the pest control agent of the present invention is formulated into an emulsifiable concentrate, a wettable powder, a flowable or the like, the pest control agent is usually diluted with water for an application so as to have a concentration of the active ingredient of 0.1 to 10000 ppm, and oil formulations, aerosols, smoking agents, poisonous bait formulations and the like are applied as they are.

When the arthropod pest control agent of the present invention is used in the control of external parasites on livestock such as cows, horses, pigs, sheep, goats and chickens, and small animals such as dogs, cats, rats and mice, veterinary known methods can be applied to the animals. As specific methods, the formulation is administered, for example, by way of a tablet, mixing in feed, a suppository and injection (intramuscular, subcutaneous, intravenous, intraperitoneal injections, etc.), when systemic control is intended, and the formulation is used, for example, by way of spraying an oil solution or aqueous solution, pour-on or spot-on treatment, washing an animal with a shampoo formulation, or putting a collar or ear tag made of a resin formulation on to an animal, when non-systemic control is intended. The amount of the compound of the present invention when administered to an animal body is usually within the range from 0.1 to 1000 mg per 1 kg of the weight of an animal.

The pest control agent of the present invention can be used in the farmlands where the following crops are grown.

Crops: corn, rice, wheat, barley, rye, oat, sorghum, cotton, soybean, peanut, sarrazin, sugar beet, rapeseed, sunflower, sugar cane, tobacco, etc.

Vegetables: Solanaceae vegetables (eggplant, tomato, green pepper, hot pepper, potato, etc.), Cucurbitaceae vegetables (cucumber, pumpkin, zucchini, watermelon, melon, etc.), Cruciferae vegetables (Japanese radish, turnip, horseradish, kohlrabi, Chinese cabbage, cabbage, brown mustard, broccoli, cauliflower, etc.), Compositae vegetables

(burdock, garland chrysanthemum, artichoke, lettuce, etc.), Liliaceae vegetables (Welsh onion, onion, garlic, asparagus, etc.), Umbelliferae vegetables (carrot, parsley, celery, parsnip, etc.), Chenopodiaceae vegetables (spinach, Swiss chard, etc.), Labiatae vegetables (Japanese mint, mint, basil, etc.), strawberry, sweat potato, yarn, aroid, etc.

Fruit trees: pomaceous fruits (apple, common pear, Japanese pear, Chinese quince, quince, etc.), stone fleshy fruits (peach, plum, nectarine, Japanese plum, cherry, apricot, prune, etc.), citrus plants (Satsuma mandarin, orange, lemon, lime, grapefruits, etc.), nuts (chestnut, walnut, hazel nut, almond, pistachio, cashew nut, macadamia nut, etc.), berry fruits (blueberry, cranberry, blackberry, raspberry, etc.), grape, persimmon, olive, loquat, banana, coffee, date, coconut, oil palm, etc.

Trees other than fruit trees: tea, mulberry, flowering trees and shrubs (azalea, camellia, hydrangea, sasanqua, Illicium religiosum, cherry tree, tulip tree, crape myrtle, fragrant olive, etc.), street trees (ash tree, birch, dogwood, eucalyptus, ginkgo, lilac, maple tree, oak, poplar, cercis, Chinese sweet gum, plane tree, zelkova, Japanese arborvitae, fir tree, Japanese hemlock, needle juniper, pine, spruce, yew, elm, horse-chestnut, etc.), sweet viburnum, Podocarpus macrophyllus, Japanese cedar, Japanese cypress, croton, spindle tree, Japanese photinia, etc.

Grass: zoysia (Japanese lawn grass, mascarene grass, etc.), Bermuda grass (Cynodon dactylon, etc.), bent grass (creeping bent grass, Agrostis stolonifera, Agrostis tenuis, etc.), bluegrass (Kentucky bluegrass, rough bluegrass, etc.), fescue (tall fescue, chewing fescue, creeping fescue, etc.), ryegrass (darnel, perennial ryegrass, etc.), cocksfoot, timothy grass, etc.

Others: flowers (rose, carnation, chrysanthemum, Eustoma grandiflorum Shinners (prairie gentian), gypsophila, gerbera, pot marigold, salvia, petunia, verbena, tulip, aster, gentian, lily, pansy, cyclamen, orchid, lily of the valley, lavender, stock, ornamental kale, primula, poinsttia, gladiolus, cattleya, daisy, cymbidium, begonia, etc.), bio-fuel plants (Jatropha, curcas, safflower, Camelina alyssum, switchgrass, miscanthus, reed canary grass, Arundo donax, kenaf, cassava, willow, algae, etc.), foliage plants, etc.

The crops also contain genetically modified crops.

The pest control agent of the present invention can be used as a mixture with or in combination with other insecticide, miticide, nematicide, fungicide, plant growth regulator, herbicide or synergist. Examples of the active ingredient of said insecticide, miticide, nematicide, fungicide, herbicide and synergist are shown below.

Active Ingredients of Insecticide

(1) Organic phosphorus compounds

acephate, aluminium phosphide, butathiofos, cadusafos, chlorethoxyfos, chlorfenvinphos, chlorpyrifos, chlorpyrifos-methyl, cyanophos: CYAP, diazinon, DCIP (dichlorodiisopropyl ether), dichlofenthion: ECP, dichlorvos: DDVP, dimethoate, dimethylvinphos, disulfoton, EPN, ethion, ethoprophos, etrimfos, fenthion: MPP, fenitrothion: MEP, fosthiazate, formothion, hydrogen phosphide, isofenphos, isoxathion, malathion, mesulfenfos, methidathion: DMTP, monocrotophos, naled: BRP, oxydeprofos: ESP, parathion, phosalone, phosmet: PMP, pirimiphos-methyl, pyridafenthion, quinalphos, phenthoate: PAP, profenofos, propaphos, prothiofos, pyraclorfos, salithion, sulprofos, tebupirimfos, temephos, tetrachlorvinphos, terbufos, thiometon, trichlorphon: DEP, vamidothion, phorate, and cadusafos.

(2) Carbamate compounds

alanycarb, bendiocarb, benfuracarb, BPMC, carbaryl, carbofuran, carbosulfan, cloethocarb, ethiofencarb, fenobucarb, fenothiocarb, fenoxycarb, furathiocarb, isoprocarb: MIPC, metolcarb, methomyl, methiocarb, NAC, oxamyl, pirimicarb, propoxur: PHC, XMC, thiodicarb, xylylcarb, and aldicarb.

(3) Pyrethroid compounds

acrinathrin, allethrin, benfluthrin, beta-cyfluthrin, bifenthrin, cycloprothrin, cyfluthrin, cyhalothrin, cypermethrin, deltamethrin, esfenvalerate, ethofenprox, fenpropathrin, fenvalerate, flucythrinate, flufenoprox, flumethrin, fluvalinate, halfenprox, imiprothrin, permethrin, prallethrin, pyrethrins, resmethrin, sigma-cypermethrin, silafluofen, tefluthrin, tralomethrin, transfluthrin, tetramethrin, phenothrin, cyphenothrin, alpha-cypermethrin, zeta-cypermethrin, lambda-cyhalothrin, gamma-cyhalothrin, furamethrin, tau-fluvalinate, metofluthrin, profluthrin, dimefluthrin, 2,3,5,6-tetrafluoro-4-(methoxymethyl)benzyl (EZ) - (1RS, 3RS; 1RS, 3SR)-2,2-dimethyl-3-prop-1-enylcyclopropanecarboxylate, 2,3,5,6-tetrafluoro-4-methylbenzyl (EZ)-(1RS, 3RS; 1RS, 3SR)-2,2-dimethyl-3-prop-1-enylcyclopropanecarboxylate, and 2,3,5,6-tetrafluoro-4-(methoxymethyl)benzyl (1RS, 3RS; 1RS, 3SR)-2,2-dimethyl-3-(2-methyl-1-propenyl)cyclopropanecarbox ylate, and 2,3,5,6-tetrafluoro-4-(methoxymethyl)benzyl (EZ)-(1RS, 3RS; 1RS, 3SR)-2,2-dimethyl-3-(2-cyano-1-propenyl)cyclopropanecarboxy late.

(4) Nereistoxin compounds

caxtap, bensultap, thiocyclam, monosultap, and bisultap.

(5) Neonicotinoid compounds

imidacloprid, nitenpyram, acetamiprid, thiamethoxam, thiacloprid, dinotefuran, and clothianidin.

(6) Benzoyl urea compounds
chlorfluazuron, bistrifluron, diafenthiuron, diflubenzuron, fluazuron, flucycloxuron, flufenoxuron, hexaflumuron, lufenuron, novaluron, noviflumuron, teflubenzuron, triflumuron, and triazuron.
(7) Phenylpyrazole compounds
acetoprole, ethiprole, fipronil, vaniliprole, pyriprole, and pyrafluprole.
(8) Bt toxins
Living spores derived from Bacillus thuringiensis and produced crystalline toxins and mixtures thereof;
(9) Hydrazine compounds
chromafenozide, halofenozide, methoxyfenozide, and tebufenozide.
(10) Organic chlorine compounds
aldrin, dieldrin, dienochlor, endosulfan, and methoxychlor.
(11) Other active ingredients of insecticide
machine oil, nicotine-sulfate; avermectin-B, bromopropylate, buprofezin, chlorphenapyr, cyantraniliprole, cyromazine, D-D(1, 3-Dichloropropene), emamectin-benzoate, fenazaquin, flupyrazofos, hydroprene, methoprene, indoxacarb, metoxadiazone, milbemycin-A, pymetrazine, pyridalyl, pyriproxyfen, spinosad, sulfluramid, tolfenpyrad, triazamate, flubendiamide, lepimectin, arsenic acid, benclothiaz, calcium cyanamide, calcium polysulfide, chlordane, DDT, DSP, flufenerim, flonicamid, flurimfen, formetanate, metam-ammonium, metam-sodium, methyl bromide, potassium oleate, protrifenbute, spiromesifen, sulfoxaflor, sulfur, metaflumizone, spirotetramat, pyrifluquinazone, spinetoram, chlorantraniliprole, tralopyril, cyantraniliprole, compounds represented by
the following formula (K):

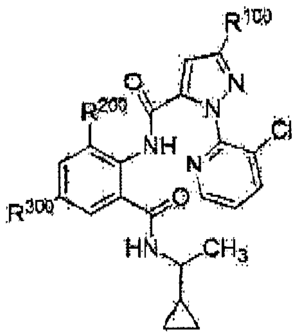

(K)

wherein $R^{100}$ represents chlorine, bromine or a trifluoromethyl group, $R^{200}$ represents chlorine, bromine or a methyl group, and $R^{300}$ represents chlorine, bromine or a cyano group, and compounds represented by
the following formula (L):

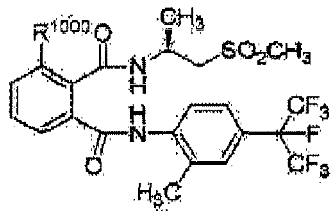

(L)

wherein $R^{1000}$ represents chlorine, bromine or iodine. Active Ingredients of Miticide
acequinocyl, amitraz, benzoximate, bifenaate, bromopropylate, chinomethionat, chlorobenzilate, CPCBS (chlorfenson), clofentezine, cyflumetofen, kelthane (dicofol), etoxazole, fenbutatin oxide, fenothiocarb, fenpyroximate, fluacrypyrim, fluproxyfen, hexythiazox, propargite: BPPS, polynactins, pyridaben, pyrimidifen, tebufenpyrad, tetradifon, spirodiclofen, spiromesifen, spirotetramat, amidoflumet, and cyenopyrafen.

Active Ingredients of Nematicide

DCIP, fosthiazate, levamisol, methyisothiocyanate, morantel tartarate, and imicyafos.

Active Ingredients of Fungicide

azole fungicidal compounds such as propiconazole, prothioconazole, triadimenol, prochloraz, pencanazole,

tebuconazole, flusilazole, diniconazole, bromuconazole, epoxiconazole, difenoconazole, cyproconazole, metconazole, triflumizole, tetraconazole, myclobutanil, fenbuconazole, hexaconazole, fluquinconazole, triticonazole, bitertanol, imazalil, and flutriafol; cyclic amine fungicidal compounds such as fenpropimorph, tridemorph, and fenpropidin; benzimidazole fungicidal compounds such as carbendezim, benomyl, thiabendazole, and thiophanate-methyl; procymidone; cyprodinil; pyrimethanil; diethofencarb; thiuram; fluazinam; mancozeb; iprodione; vinclozolin; chlorothalonil; captan; mepanipyrim; fenpiclonil; fludioxonil; dichlofluanid; folpet; kresoxim-methyl; azoxystrobin; trifloxystrobin; fluoxastrobin; picoxystrobin; pyraclostrobin; dimoxystrobin; pyribencarb; spiroxamine; quinoxyfen; fenhexamid; famoxadone; fenamidone; zoxamide; ethaboxam; amisulbrom; iprovalicarb; benthiavalicarb; cyazofamid; mandipropamid; boscalid; penthiopyrad; metrafenone; fluopiran; bixafen; cyflufenamid; proquinazid; isotianil and tiadinil.

Active Ingredients of Herbicidal Compounds

(1) Phenoxy fatty acid herbicidal compounds
2,4-PA, MCP, MCPB, phenothiol, mecoprop, fluroxypyr, triclopyr, clomeprop, and naproanilide.
(2) Benzoate herbicidal compounds
2,3,6-TBA, dicamba, clopyralid, picloram, aminopyralid, quinclorac, and quinmerac.
(3) Urea herbicidal compounds
diuron, linuron, chlortoluron, isoproturon, fluometuron, isouron, tebuthiuron, methabenzthiazuron, cumyluron, daimuron, and methyl-daimuron.
(4) Triazine herbicidal compounds
atrazine, ametoryn, cyanazine, simazine, propazine, simetryn, dimethametryn, prometryn, metribuzin, triaziflam, and indaziflam.
(5) Bipyridinium herbicidal compounds
paraquat and diquat.
(6) Hydroxybenzonitrile herbicidal compounds
bromoxynil and ioxynil.
(7) Dinitroaniline herbicidal compounds
pendimethalin, prodiamine, and trifluralin.
(8) Organophosphorus herbicidal compounds
amiprofos-methyl, butamifos, bensulide, piperophos, anilofos, glyphosate, glufosinate, glufosinate-P, and bialaphos.
(9) Carbamate herbicidal compounds
di-allate, tri-allate, EPTC, butylate, benthiocarb, esprocarb, molinate, dimepiperate, swep, chlorpropham, phenmedipham, phenisopham, pyributicarb, and asulam.
(10) Acid amide herbicidal compounds
propanil, propyzamide, bromobutide, and etobenzanid.
(11) Chloroacetanilide herbicidal compounds
acetochlor, alachlor, butachlor, dimethenamid, propachlor, metazachlor, metolachlor, pretilachlor, thenylchlor, and pethoxamid.
(12) Diphenyl ether herbicidal compounds
acifluorfen-sodium, bifenox, oxyfluorfen, lactofen, fomesafen, chlomethoxynil, and aclonifen.
(13) Cyclic imide herbicidal compounds
oxadiazon, cinidon-ethyl, carfentrazone-ethyl, surfentrazone, flumiclorac-pentyl, flumioxazin, pyraflufen-ethyl, oxadiargyl, pentoxazone, fluthiacet-methyl, butafenacil, benzfendizone, bencarbazone, and saflufenacil.
(14) Pyrazole herbicidal compounds
benzofenap, pyrazolate, pyrazoxyfen, topramezone, and pyrasulfotole.
(15) Triketone herbicidal compounds
isoxaflutole, benzobicyclon, sulcotrione, mesotrione, tembotrione, and tefuryltrione.
(16) Aryloxyphenoxypropionate herbicidal compounds
clodinafop-propargyl, cyhalofop-butyl, diclofop-methyl, fenoxaprop-ethyl, fluazifop-butyl, haloxyfop-methyl, quizalofop-ethyl, and metamifop.
(17) Trione oxime herbicidal compounds
alloxydim-sodium, sethoxydim, butroxydim, clethodim, cloproxydin, cycloxydim, tepraloxydim, tralkoxydim, and profoxydim.
(18) Sulfonyl urea herbicidal compounds
chlorsulfuron, sulfometuron-methyl, metsulfuron-methyl, chlorimuron-ethyl, tribenuron-methyl, triasulfuron, bensulfuron-methyl, thifensulfuron-methyl, pyrazosulfuron-ethyl, primisulfuron-methyl, nicosulfuron, amidosulfuron, cinosulfuron, imazosulfuron, rimsulfuron, halosulfuron-methyl, prosulfuron, ethametsulfuron-methyl, triflusulfuron-methyl, flazasulfuron, cyclosulfamuron, flupyrsulfuron, sulfosulfuron, azimsulfuron, ethoxysulfuron, oxasulfuron, iodosulfuron-methyl-sodium, foramsulfuron, mesosulfuron-methyl, trifloxysulfuron, tritosulfuron, orthosulfamuron, flucetosulfuron, and propyrisulfuron.

(19) Imidazolinone herbicidal compounds
imazamethabenz-methyl, imazamethapyr, imazamox, imazapyr, imazaquin, and imazethapyr.

(20) Sulfonamide herbicidal compounds
flumetsulam, metasulam, diclosulam, florasulam, cloransulam-methyl, penoxsulam, and pyroxsulam.

(21) Pyrimidinyloxybenzoate herbicidal compounds
pyrithiobac-sodium, bispyribac-sodium, pyriminobac-methyl, pyribenzoxim, pyriftalid, and pyrimisulfan.

(22) Other herbicidal compounds
bentazon, bromacil, terbacil, chlorthiamid, isoxaben, dinoseb, amitrole, cinmethylin, tridiphane, dalapon, diflufenzopyr-sodium, dithiopyr, thiazopyr, flucarbazone-sodium, propoxycarbazone-sodium, mefenacet, flufenacet, fentrazamide, cafenstrole, indanofan, oxaziclomefone, benfuresate, ACN, pyridate, chloridazon, norflurazon, flurtamone, diflufenican, picolinafen, beflubutamid, clomazone, amicarbazone, pinoxaden, pyraclonil, pyroxasulfone, thiencarbazone-methyl, aminocyclopyrachlor, ipfencarbazone, and methiozolin.

Active Ingredients of Synergist

piperonyl butoxide, sesamex, sulfoxide, N-(2-ethylhexyl)-8,9,10-trinorborn-5-ene-2,3-dicarboximide (MGK 264), N-declyimidazole), WARF-antiresistant, TBPT, TPP, IBP, PSCP, methyl iodide ($CH_3I$), t-phenylbutenone, diethylmaleate, DMC, FDMC, ETP, and ETN.

EXAMPLES

Hereinbelow, the present invention will be further described in detail by production examples, formulation examples, test examples, and the like. However, the present invention is not limited to these examples.

First, the production examples for the production of the compounds of the present invention are shown bellow.

Production Example 1 (1)

0.52 g of sodium hydride (60%, oily) was added to a mixture of 1. 39 g of 3-chloro-2-cyanopyridine, 0.9 ml of ethyl mercaptan and 10 ml of DMF under ice cooling, and the mixture was stirred at room temperature for 1 hour. Water was added to the reaction mixture, and the mixture was extracted with ethyl acetate. The organic layer was washed with a saturated aqueous sodium bicarbonate solution and saturated saline water, then dried over anhydrous sodium sulfate, and concentrated under reduced pressure. The resulting residue was applied to a silica gel column chromatography to obtain 1.52 g of 2-cyano-3-ethylsulfanylpyridine. 2-Cyano-3-ethylsulfanylpyridine

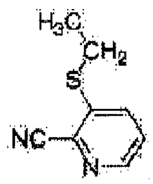

$^1$H-NMR($CDCl_3$)$\delta$:8.49(1H,dd),7.75(1H,dd),7.43(1H,dd),3.06(2H, q),1.38(3H,t)

Production Example 1 (2)

1.4 g of 2-cyano-3-ethylsulfanylpyridine was added to a mixture of 15 ml of concentrated sulfuric acid and 5 ml of water, and the mixture was stirred at 130°C for 2 hours. An aqueous potassium hydroxide solution was added to the reaction mixture cooled to room temperature, and the mixture was extracted with ethyl acetate. Concentrated hydrochloric acid was added to the resulting aqueous layer, and the mixture was extracted with chloroform, and then dried over anhydrous sodium sulfate and concentrated under reduced pressure to obtain 1.15 g of 3-ethylsulfanylpicolinic acid. 3-Ethylsulfanylpicolinic acid

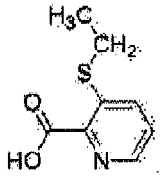

$^1H$-NMR($CDCl_3$)δ:8.31 (1H,d),7.75(1H,d),7.49(1H,dd),2.97 (2H,q) ,1.44(3H,t)

Production Example 1 (3)

A mixture of 0.50 g of 2-amino-5-chloro-4-trifluoromethylphenol synthesized by the method described in WO2009/131237, 0.43 g of 3-ethylsulfanylpicolinic acid, 0.54 g of EDCI hydrochloride and 6 ml of chloroform was stirred at room temperature for 2 hours. Water was added to the reaction mixture, and the mixture was extracted with ethyl acetate. The organic layer was washed with a saturated aqueous sodium bicarbonate solution and saturated saline water, then dried over anhydrous sodium sulfate, and concentrated under reduced pressure. The resulting residue was applied to a silica gel column chromatography to obtain 0.45 g of 3-ethylsulfanyl-N-[4-chloro-2-hydroxy-5-trifluoromethylphen yl]picolinamide. 3-Ethylsulfanyl-N-[4-chloro-2-hydroxy-5-trifluoromethylphen yl]picolinamide

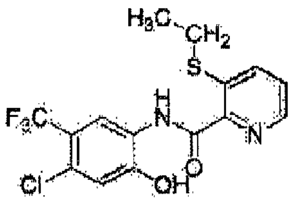

Production Example 1 (4)

A mixture of 0.45 g of 3-ethylsulfanyl-N-[4-chloro-2-hydroxy-5-trifluoromethylphen yl]picolinamide, 0.44 g of di-2-methoxyethylazodicarboxylate (hereinafter referred to as DMEAD), 0.46 g of triphenylphosphine and 15 ml of THF was stirred at 50°C for 1 hour. Water was added to the reaction mixture cooled to room temperature, and the mixture was extracted with ethyl acetate. The organic layer was washed with a saturated aqueous sodium bicarbonate solution and saturated saline water, then dried over anhydrous sodium sulfate, and concentrated under reduced pressure. The resulting residue was applied to a silica gel column chromatography to obtain 0.36 g of 2-(3-ethylsulfanylpyridin-2-yl)-6-chloro-5-trifluoromethylb enzoxazole (hereinafter, referred to as Compound of Present Invention 1-1).

Compound of Present Invention 1-1

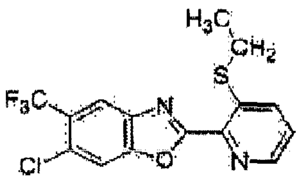

$^1H$-NMR($CDCl_3$)δ:8.59(1H,dd),8.30(1H,s),7.83(1H,s),7.79(1H,dd ),7.43(1H,dd),3.07(2H,q),1.47(3H,t)

Production Example 2

0.36 g of m-chloroperbenzoic acid (purity of 65% or more) was added to a mixture of Compound of Present Invention 1-1 (0.25 g) and 10 ml of chloroform under ice cooling, and then the mixture was stirred at room temperature for 2 hours. A 10% aqueous sodium sulfite solution was added to the reaction mixture, and the mixture was extracted with chloroform. The organic layer was washed with a saturated aqueous sodium bicarbonate solution, then dried over anhydrous sodium sulfate, and concentrated under reduced pressure. The resulting residue was applied to a silica gel column chromatography to obtain 0.28 g of 2-(3-ethylsulfonylpyridin-2-yl)-6-chloro-5-trifluoromethylb enzoxazole (hereinafter, referred to as Compound of Present Invention 1-2).

Compound of Present Invention 1-2

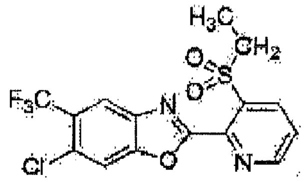

$^1$H-NMR($CDCl_3$)$\delta$:9.03(1H,dd) ,8.59(1H,dd),8.22(1H,s),7.85(1H,s ),7.76(1H,dd),3.96(2H,q),1.43(3H,t)

Production Example 3 (1)

A mixture of 0.40 g of 2-amino-5-(heptafluoroisopropyl)phenol synthesized by the method described in EP1380568, 0.26 g of 3-ethylsulfanylpicolinic acid, 0.33 g of EDCI hydrochloride and 4 ml of pyridine was stirred at room temperature for 3 hours. Water was added to the reaction mixture, and the mixture was extracted with ethyl acetate. The organic layer was washed with a saturated aqueous sodium bicarbonate solution and saturated saline water and dried over anhydrous sodium sulfate, and then concentrated under reduced pressure to obtain 0.68 g of 3-ethylsulfanyl-N-[2-hydroxy-4-(heptafluoroisopropyl)phenyl ]picolinamide. 3-Ethylsulfanyl-N-[2-hydroxy-4-(heptafluoroisopropyl)phenyl ]picolinamide

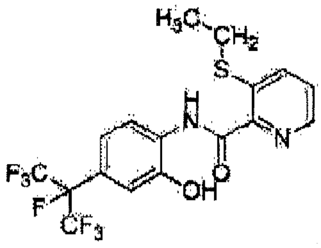

Production Example 3 (2)

A mixture of 0.68 g of 3-ethylsulfanyl-N-[2-hydroxy-4-(heptafluoroisopropyl)phenyl ]picolinamide, 0.57 g of DMEAD, 0.60 g of triphenylphosphine and 15 ml of THF was stirred at room temperature for 1 hour and at 50°C for 1 hour. Water was added to the reaction mixture cooled to room temperature, and the mixture was extracted with ethyl acetate. The organic layer was washed with a saturated aqueous sodium bicarbonate solution and saturated saline water, then dried over anhydrous sodium sulfate, and concentrated under reduced pressure. The resulting residue was applied to a silica gel column chromatography to obtain 0.37 g of 2-(3-ethylsulfanylpyridin-2-yl)-6-(heptafluoroisopropyl)ben zoxazole (hereinafter, referred to as Compound of Present Invention 1-3).

Compound of Present Invention 1-3

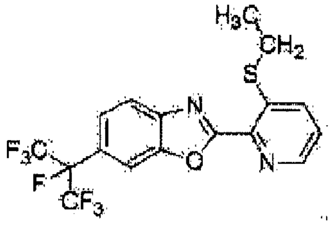

$^1$H-NMR($CDCl_3$)$\delta$:8.60(1H,dd), 8.04(1H,d),7.98(1H,s),7.79(1H,dd ),7.66(1H,d),7.43(1H,dd),3.07(2H,q),1.47(3H,t)

Production Example 4

0.30 g of m-chloroperbenzoic acid (purity of 65% or more) was added to a mixture of Compound of Present invention 1-3 (0.25 g) and 5 ml of chloroform under ice cooling, and then the mixture was stirred at room temperature for 4 hours. A 10% aqueous sodium sulfite solution was added to the reaction mixture, and the mixture was extracted with chloroform. The organic layer was washed with a saturated aqueous sodium bicarbonate solution, then dried over anhydrous sodium sulfate, and concentrated under reduced pressure. The resulting residue was applied to a silica gel column chromatography to obtain 0.24 g of 2-(3-ethylsulfonylpyridin-2-yl)-6-(heptafluoroisopropyl)ben zoxazole (hereinafter, referred to as Compound of Present Invention 1-4).

Compound of Present Invention 1-4

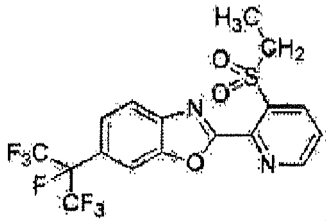

$^1$H-NMR($CDCl_3$)δ:9,04 (1H,dd) 8.60(1H,dd), 8.00-7.94(2H,m),7.76 (1H,dd),7.70(1H,d),4.02(2H,q),1.44(3H,t)

Production Example 5 (1)

0.96 ml of nitric acid (65%) was added to a mixture of 2.41 g of 2-chloro-4-(trifluoromethylsulfanyl)phenol synthesized by the method described in WO2004-010936, 12 ml of acetic acid and 2 ml of concentrated sulfuric acid, and the mixture was stirred at 60°C for 4 hours. Water was added to the reaction mixture cooled to room temperature, and the precipitated solid was filtered, washed with water and hexane, and dried under reduced pressure to obtain 2.15 g of 2-chloro-6-nitro-4-(trifluoromethylsulfanyl)phenol. 2-Chloro-6-nitro-4-(trifluoromethylsulfanyl)phenol

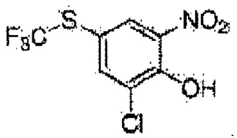

$^1$H-NMR($CDCl_3$)δ:11.26(1H,s),8.39(1H,d),7.98 (1H,d)

Production Example 5 (2)

A mixture of 1.75 g of iron powder, 4.5 ml of acetic acid, 9 ml of ethyl acetate and 4.5 ml of water was stirred at 90°C for 5 minutes, and 2-chloro-6-nitro-4- (trifluoromethylsulfanyl)phenol was added to the reaction mixture, and the mixture was stirred at 90°C for 2 hours. The reaction mixture was cooled to room temperature, and filtered through celite (registered trademark). The filtrate was concentrated under reduced pressure, a saturated aqueous sodium bicarbonate solution was added thereto, and the mixture was extracted with ethyl acetate. The organic layer was dried over anhydrous sodium sulfate and concentrated under reduced pressure. The resulting solid was washed with hexane and chloroform to obtain 1.20 g of 2-amino-6-chloro-4-(trifluoromethylsulfanyl)phenol. 2-Amino-6-chloro-4-(trifluoromethylsulfanyl)phenol

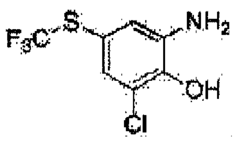

$^1$H-NMR($CDCl_3$)$\delta$:7.05(1H,d),6.91 (1H,d),5.70(1H,brs), 3.99 (2H,b rs)

Production Example 5 (3)

A mixture of 1.0 g of 2-amino-6-chloro-4-(trifluoromethylsulfanyl)phenol, 0.75 g of 3-ethylsufanylpicolinic acid, 0.94 g of EDCI hydrochloride and 10 ml of chloroform was stirred at room temperature for 1 hour. Water was added to the reaction mixture, and the mixture was extracted with ethyl acetate. The organic layer was washed with a saturated aqueous sodium bicarbonate solution and saturated saline water and dried over anhydrous sodium sulfate, and then concentrated under reduced pressure to obtain 1.78 g of 3-ethylsulfanyl-N-[3-chloro-2-hydroxy-5-(trifluoromethylsul fanyl)phenyl]picolinamide. 3-Ethylsulfanyl-N-[3-chloro-2-hydroxy-5-(trifluoromethylsul fanyl)phenyl]picolinamide

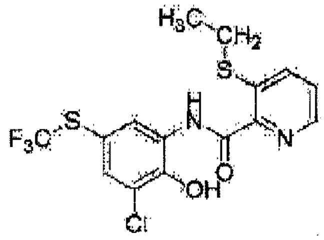

Production Example 5 (4)

A mixture of 1.78 g of 3-ethylsulfanyl-N-[3-chloro-2-hydroxy-5-(trifluoromethylsul fanyl)phenyl]picolinamide, 1.53 g of DMEAD, 1.61 g of triphenylphosphine and 40 ml of THF was stirred at 50°C for 1 hour. Water was added to the reaction mixture cooled to room temperature, and the mixture was extracted with ethyl acetate. The organic layer was washed with a saturated aqueous sodium bicarbonate solution and saturated saline water, then dried over anhydrous sodium sulfate, and concentrated under reduced pressure. The resulting residue was applied to a silica gel column chromatography to obtain 1.14 g of 7-chloro-2-(3-ethylsulfanylpyridin-2-yl)-5-(trifluoromethyl sulfanyl)benzoxazole (hereinafter, referred to as Compound of Present Invention 1-5).

Compound of Present Invention 1-5

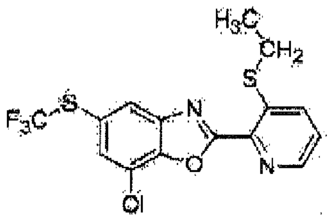

$^1$H-NMR($CDCl_3$)$\delta$:8.62(1H,dd),8.16(1H,d),7.79(1H,dd),7.74(1H,d ),7.43(1H,dd),3.07(2H,q),1.46(3H,t)

Production Example 6

1.78 g of m-chloroperbenzoic acid (purity of 65% or more) was added to a mixture of Compound of Present Invention 1-5 (1.14 g) and 20 ml of chloroform under ice cooling, and then the mixture was stirred under ice cooling for 2 hours. 0.13 g of m-chloroperbenzoic acid (purity of 65% or more) was added to the reacting mixture, and then the mixture was stirred at room temperature for 2 hours. A 10% aqueous sodium sulfite solution was added to the reaction mixture, and the mixture was extracted with chloroform. The organic layer was washed with a saturated aqueous sodium bicarbonate solution, then dried over anhydrous magnesium sulfate, and concentrated under reduced pressure. The resulting residue was applied to a silica gel column chromatography to obtain 0.49 g of 7-chloro-2-(3-ethylsulfonylpyridin-2-yl)-5-(trifluoromethyl sulfanyl)benzoxazole (hereinafter, referred to as Compound of Present Invention 1-6) and 0.70 g of 7-chloro-2-(3-ethylsulfonylpyridin-2-yl)-5-(trifluoromethyl sulfinyl)benzoxazole (hereinafter, referred to as Compound of Present Invention 1-7).

Compound of Present Invention 1-6

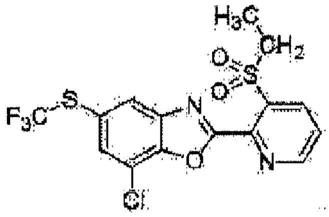

$^1$H-NMR($CDCl_3$)δ:9.07(1H,dd),8.59(1H,dd),8.21(1H,s),7.91(1H,s ),7.79(1H,dd),3.93(2H,q),1.45(3H,t)

Compound of Present Invention 1-7

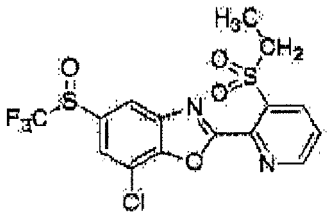

$^1$H-NMR($CDCl_3$)δ:9.07(1H,dd),8.59(1H,dd),8.21(1H,s),7.91(1H,s ),7.79(1H,dd),3.93(2H,q),1.45(3H,t)

Production Example 7

4 ml of aqueous hydrogen peroxide (30%) was added to a mixture of Compound of Present Invention 1-7 (0.35 g), 4 ml of acetonitrile and 27 mg of sodium tungstate dihydrate, and the mixture was stirred at 80°C for 4 hours. Water was added to the reaction mixture cooled to room temperature, and the precipitated solid was obtained by filtration, then the solid and a 10% aqueous sodium sulfite solution were mixed, and the mixture was extracted with ethyl acetate. The organic layer was washed with water and saturated saline water, then dried over anhydrous sodium sulfate, and concentrated under reduced pressure. The resulting residue was applied to a silica gel column chromatography to obtain 0.22 g of 7-chloro-2-(3-ethylsulfonylpyridin-2-yl)-5-(trifluoromethyl sulfonyl)benzoxazole (hereinafter, referred to as Compound of Present Invention 1-8).

Compound of Present Invention 1-8

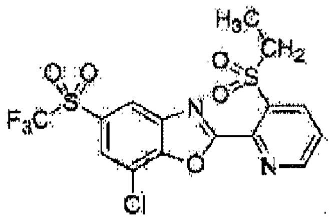

$^1$H-NMR($CDCl_3$)δ:9.08(1H,dd), 8.60 (1H,dd), 6.48 (1H,d), 8.16(1H,d ),7.82(114,dd),3.91(2H,q)1,46(3H,t)

Production Example 8 (1)

0.4 ml of thionyl chloride was added to a mixture of 0.5 g of 3-ethylsulfanylpicolinic acid, 6 ml of toluene and 0.1 ml of DMF under ice cooling, and the mixture was stirred at 100°C for 4 hours. The reaction mixture was cooled to room temperature, and concentrated under reduced pressure. 8 ml of THF was added to the reaction mixture, and the

mixture was added to mixture of 1.23 g of 2-amino-4-(trifluoromethylsulfinyl)phenol and 7 ml of THF, and then the mixture was stirred under ice cooling for 1 hour, and at room temperature overnight. A saturated aqueous sodium bicarbonate solution was added to the reaction mixture, and the mixture was extracted with ethyl acetate. The organic layer was washed with water and saturated saline water, then dried over anhydrous magnesium sulfate, and concentrated under reduced pressure. The resulting solid was washed with hexane to obtain 1.08 g of 3-ethylsulfanyl-N-[2-iodo-4-(trifluoromethyl)phenyl]picolin amide.

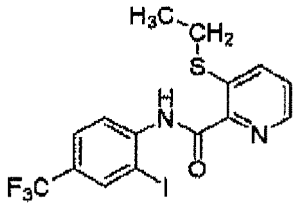

$^1$H-NMR($CDCl_3$)$\delta$:10.90(1H,brs),8.75(1H,d),8.41(1H,dd),8.06(1H ,d),7.75(1H,dd),7.63(1H,dd),7.45(1H,dd), 2,97 (2H, q),1.45(3H, t)

Production Example 8 (2)

A mixture of 1.08 g of 3-ethylsulfanyl-N-[2-iodo-4-(trifluoromethyl)phenyl]picolin amid, 0.09 g of copper(I) iodide, 0.17 g of phenanthroline, 1.56 g of cesium carbonate and 10 ml of dimethoxyethane was stirred under heat-refluxing for 20 hours. A saturated aqueous ammonium chloride solution was added to the reaction mixture cooled to room temperature, and the mixture was extracted with ethyl acetate. The resulting organic layer was filtered with celite (registered trademark), and the filtrate was washed with ammonia water, water and saturated saline water, then dried over anhydrous magnesium sulfate, and concentrated under reduced pressure. The resulting residue was applied to a silica gel chromatography to obtain 0.05 g of 2-(3-ethylsulfanylpyridin-2-yl)-6-trifluoromethylbenzoxazol e (hereinafter, referred to as Compound of Present Invention 1-27).

Compound of Present Invention 1-27

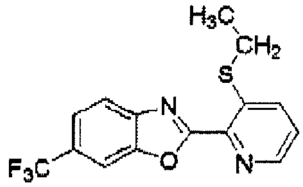

$^1$H-NMR($CDCl_3$)$\delta$:8.60 (1H,dd), 8.02(1H,d),7.97-7.94(1H,m),7.79( 1H,dd),7.71-7.66(1H,m),7.43(1H,dd),3.07(2H,q), 1.48(3H,t) Production Example 9

0.18 g of m-chloroperbenzoic acid (purity of 65% or more) was added to a mixture of Compound of Present Invention 1-27 (0.11 g) and 3 ml of chloroform under ice cooling, and the mixture was stirred at room temperature for 2 hours. A 10% aqueous sodium sulfite solution was added to the reaction mixture, and the mixture was extracted with chloroform. The organic layer was washed with a saturated aqueous sodium bicarbonate solution, then dried over anhydrous magnesium sulfate, and concentrated under reduced pressure to obtain 0.12 g of 2-(3-ethylsulfonylpyridin-2-yl)-6-trifluoromethylbenzoxazol e (hereinafter, referred to as Compound of Present Invention 1-28).

Compound of Present Invention 1-28

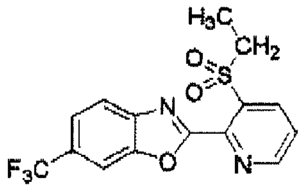

$^1$H-NMR($CDCl_3$)δ:9.04(1H,dd),8.60(1H,dd),7.98-7.93(2H,m),7.76 (1H,dd) , 7.72(1H,dd),4.00(2H,q),1.43(3H,t)

The compounds described in the production examples described above and the compounds produced by the production method according to the method described in the production examples described above are shown in the tables.

The compounds of the present invention represented by the formula (1).

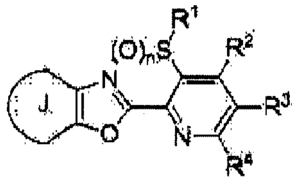

(1)

In the formula, $R^1$, $R^2$, $R^3$, $R^4$, J, $R^6$, $R^7$, $R^8$, G and n represent the combinations shown in [Table 12] to [Table 13] shown below.

[Table 12]

| Compound of Present Invention | $R^1$ | $R^2$ | $R^3$ | $R^4$ | J | $R^6$ | $R^7$ | $R^8$ | G | n |
|---|---|---|---|---|---|---|---|---|---|---|
| 1-1 | Et | H | H | H | J3 | Cl | - | - | $CF_3$ | 0 |
| 1-2 | Et | H | H | H | J3 | Cl | - | - | $CF_3$ | 2 |
| 1-3 | Et | H | H | H | J8 | - | H | H | $CF(CF_3)_2$ | 0 |
| 1-4 | Et | H | H | H | J8 | - | H | H | $CF(CF_3)_2$ | 2 |
| 1-5 | Et | H | H | H | J4 | Cl | - | - | $SCF_3$ | 0 |
| 1-6 | Et | H | H | H | J4 | Cl | - | - | $SCF_3$ | 2 |
| 1-7 | Et | H | H | H | J4 | Cl | - | - | $S(O)CF_3$ | 2 |
| 1-8 | Et | H | H | H | J4 | Cl | - | - | $S(O)_2CF_3$ | 2 |
| 1-9 | Et | H | H | H | J3 | Cl | - | - | $SCF_3$ | 0 |
| 1-10 | Et | H | H | H | J3 | Cl | - | - | $SCF_3$ | 2 |
| 1-11 | Et | H | H | H | J3 | Cl | - | - | $S(O)CF_3$ | 2 |
| 1-12 | Et | H | H | H | J3 | Cl | - | - | $S(O)_2CF_3$ | 2 |
| 1-13 | Et | H | $CF_3$ | H | J3 | Cl | - | - | $SCF_3$ | 0 |
| 1-14 | Et | H | $CF_3$ | H | J3 | Cl | - | - | $SCF_3$ | 2 |
| 1-15 | Et | H | $CF_3$ | H | J3 | Cl | - | - | $S(O)CF_3$ | 2 |
| 1-16 | Et | H | $CF_3$ | H | J3 | Cl | - | - | $S(O)_2CF_3$ | 2 |

[Table 13]

| Compound of Present Invention | $R^1$ | $R^2$ | $R^3$ | $R^4$ | J | $R^6$ | $R^7$ | $R^8$ | G | n |
|---|---|---|---|---|---|---|---|---|---|---|
| 1-17 | Et | H | H | H | J4 | Cl | - | - | $SCF_3$ | 0 |
| 1-18 | Et | H | H | H | J4 | Cl | - | - | $SCF_3$ | 2 |

(continued)

| Compound of Present Invention | $R^1$ | $R^2$ | $R^3$ | $R^4$ | J | $R^6$ | $R^7$ | $R^8$ | G | n |
|---|---|---|---|---|---|---|---|---|---|---|
| 1-19 | Et | H | H | H | J4 | Cl | - | - | $S(O)CF_3$ | 2 |
| 1-20 | Et | H | H | H | J4 | Cl | - | - | $S(O)_2CF_3$ | 2 |
| 1-21 | Et | H | H | H | J5 | Cl | - | - | $CF_3$ | 0 |
| 1-22 | Et | H | H | H | J5 | Cl | - | - | $CF_3$ | 2 |
| 1-23 | Et | H | H | H | J6 | Cl | - | - | $CF_3$ | 0 |
| 1-24 | Et | H | H | H | J6 | Cl | - | - | $CF_3$ | 2 |
| 1-25 | Et | H | H | H | J7 | Cl | - | - | $CF_3$ | 0 |
| 1-26 | Et | H | H | H | J7 | Cl | - | - | $CF_3$ | 2 |
| 1-27 | Et | H | H | H | J8 | - | H | - | $CF_3$ | 0 |
| 1-28 | Et | H | H | H | J8 | - | H | - | $CF_3$ | 2 |
| 1-29 | Et | H | H | H | J9 | - | H | - | $CF_3$ | 0 |
| 1-30 | Et | H | H | H | J9 | - | H | - | $CF_3$ | 2 |

(In [Table 12] to [Table 13] described above, Et represents an ethyl group)

Next, formulation examples of the compound of the present invention are shown. The part means part by weight.

Formulation Example 1

10 parts of any one of Compounds of Present Invention 1-1 to 1-30 is dissolved in a mixture of 35 parts of xylene and 35 parts of N,N-dimethylformamide, and 14 parts of polyoxyethylenestyrylphenyl ether and 6 parts of calcium dodecylbenzenesulfonate are added thereto. The mixture is mixed to obtain each emulsifiable concentrate.

Formulation Example 2

4 parts of sodium lauryl sulfate, 2 parts of calcium lignosulfonate, 20 parts of synthetic hydrous silicon oxide fine powder and 54 parts of diatomaceous earth are mixed, and 20 parts of any one of Compounds of Present Invention 1-1 to 1-30 is further added thereto. The mixture is mixed to obtain each wettable powder.

Formulation Example 3

1 part of synthetic hydrous silicon oxide fine powder, 2 parts of calcium lignosulfonate, 30 parts of bentonite, 65 parts of kaolin clay are added to and mixed with 2 parts of any one of Compounds of Present Invention 1-1 to 1-30. Subsequently, an appropriate amount of water is added to this mixture, and the mixture is further stirred, granulated with a granulator, and forced-air dried to obtain each granule.

Formulation Example 4

1 part of any one of Compounds of Present Invention 1-1 to 1-30 is dissolved in an appropriate amount of acetone, 5 parts of synthetic hydrous silicon oxide fine powder, 0.3 parts of PAP and 93.7 parts of Fubasami clay are added thereto. The mixture is sufficiently stirred and mixed to evaporate and eliminate acetone to obtain each dust formulation.

Formulation Example 5

35 parts of a mixture of polyoxyethylene alkyl ether sulfate ammonium salt and white carbon (weight ratio 1 : 1), 10 parts of any one of Compounds of Present Invention 1-1 to 1-30 and 55 parts of water are mixed, and finely pulverized by wet grinding method to obtain each flowable.

Formulation Example 6

0.1 parts of any one of Compounds of Present Invention 1-1 to 1-30 is dissolved in 5 parts of xylene and 5 parts of trichloroethane, and the mixture is mixed with 89.9 parts of deodorized kerosene to obtain each oil solution.

Formulation Example 7

10 mg of any one of Compounds of Present Invention 1-1 to 1-30 is dissolved in 0.5 ml of acetone, and this solution is applied to 5 g of solid feed powder for animal (solid feed powder for breeding CE-2, product of CLEA Japan, Inc.), and the mixture is uniformly mixed. Subsequently, acetone is evaporated to dryness to obtain each poisonous bait formulation.

Formulation Example 8

0.1 parts of any one of Compounds of Present Invention 1-1 to 1-30 and 49.9 parts of Neothiozol (Chuo Kasei Co., Ltd.) are filled into an aerosol can, and an aerosol valve is attached, then the container is filled with 25 parts of dimethyl ether and 25 parts of LPG and shaken, and an actuator is attached to obtain an oil-based aerosol.

Formulation Example 9

0.6 parts of any one of Compounds of Present Invention 1-1 to 1-30, 0.01 parts of BHT (2,6-di-tert-butyl-4-methylphenol), 5 parts of xylene, 3.39 parts of deodorized kerosene and 1 part of emulsifier {RHEODOL MO-60 (manufactured by Kao Corporation)} are mixed and dissolved, and the resulting solution and 50 parts of distilled water are filled into an aerosol container. A valve is attached to the container, and then 40 parts of a propellant (LPG) is filled under pressure through the valve to obtain an aqueous aerosol.

Formulation Example 10

0.1 g of any one of Compounds of Present Invention 1-1 to 1-30 is dissolved in 2 ml of propylene glycol, and a porous ceramic plate with a size of 4.0 cm × 4.0 cm and 1.2 cm in thickness is impregnated with the solution to obtain a heating type smoking agent.

Formulation Example 11

5 parts of any one of Compounds of Present Invention 1-1 to 1-30 and 95 parts of an ethylene-methyl methacrylate copolymer (a ratio of methyl methacrylate in the copolymer: 10% by weight, Acryft WD301, manufactured by SUMITOMO CHEMICAL Co., Ltd.) are melt-kneaded with a closed pressurizing kneader (manufactured by Moriyama Works), and the resulting kneaded matter is extruded from a molding machine through a molding die to obtain a rod-shaped molded body with a size of 15 cm in length and 3 mm in diameter.

Formulation Example 12

5 parts of any one of Compounds of Present Invention 1-1 to 1-30 and 95 parts of a soft vinyl chloride resin are melt-kneaded with a closed pressurizing kneader (manufactured by Moriyama Works), and the resulting kneaded matter is extruded from a molding machine through a molding die to obtain a rod-shaped molded body with a size of 15 cm in length and 3 mm in diameter.

Formulation Example 13

100 mg of any one of Compounds of Present Invention 1-1 to 1-30, 68.75 mg of lactose, 237.5 mg of corn starch, 43.75 mg of microcrystalline cellulose, 18.75 mg of polyvinylpyrrolidone, 28.75 mg of sodium carboxymethyl starch and 2.5 mg of magnesium stearate are mixed, and the resulting mixture was compressed to an appropriate size to obtain a tablet.

Formulation Example 14

25 mg of any one of Compounds of Present Invention 1-1 to 1-30, 60 mg of lactose, 25 mg of corn starch, 6 mg of carmellose calcium and an appropriate amount of 5% hydroxypropyl methylcellulose, and the resulting mixture is

filled into a hard shell gelatin capsule or a hydroxypropyl methylcellulose capsule to obtain an encapsulated formulation.

Formulation Example 15

Distilled water is added to 1000 mg of any one of Compounds of Present Invention 1-1 to 1-30, 500 mg of fumaric acid, 2000 mg of sodium chloride, 150 mg of methylparaben, 50 mg of propylparaben, 25000 mg of granulated sugar, 13000 mg of sorbitol (70% solution), 100 mg of Veegum K (Vanderbilt Co.), 35 mg of flavor and 500 mg of colorant, such that a final volume is 100 ml, and the mixture is mixed to obtain a suspension for oral administration.

Formulation Example 16

5% by weight of any one of Compounds of Present Invention 1-1 to 1-30 is dissolved in 5% by weight of polysorbate 85, 3% by weight of benzyl alcohol and 30% by weight of propylene glycol, and a phosphate buffer is added to this solution so as to have a pH of 6.0 to 6.5, and then water is added until it reaches the final volume to obtain a liquid formulation for oral administration.

Formulation Example 17

5% by weight of aluminum distearate is dispersed in 57% by weight of fractionated palm oil and 3% by weight of polysorbate 85 by heating. This dispersion is cooled to room temperature, and 25% by weight of saccharin is dispersed in an oily vehicle thereof. 10% by weight of any one of Compounds of Present Invention 1-1 to 1-30 is distributed thereto to obtain a paste formulation for oral administration.

Formulation Example 18

5% by weight of any one of Compounds of Present Invention 1-1 to 1-30 and 95% by weight of limestone filler are mixed, and a granule for oral administration is obtained using wet granulation method.

Formulation Example 19

5 parts of any one of Compounds of Present Invention 1-1 to 1-30 is dissolved in 80 parts of diethylene glycol monoethyl ether, and 15 parts of propylene carbonate is mixed therewith to obtain a spot-on solution.

Formulation Example 20

10 parts of any one of Compounds of Present Invention 1-1 to 1-30 is dissolved in 7C parts of diethylene glycol monoethyl ether, and 20 parts of 2-octyl dodecanol is mixed therewith to obtain a pour-on solution.

Formulation Example 21

60 parts of NIKKOL TEALS-42 (Nikko Chemicals Co., Ltd., 42% aqueous solution of triethanolamine lauryl sulfate) and 20 parts of propylene glycol are added to 0.5 parts of any one of Compounds of Present Invention 1-1 to 1-30, and the mixture is sufficiently stirred and mixed until it becomes a uniform solution, and then 19.5 parts of water is added and further sufficiently stirred and mixed to obtain a shampoo agent as a uniform solution.

Formulation Example 22

0.15% by weight of any one of Compounds of Present Invention 1-1 to 1-30, 95% by weight of an animal feed and 4.85% by weight of a mixture of secondary calcium phosphate, diatomaceous earth, Aerosil and carbonate (or chalk) are sufficiently stirred and mixed to obtain a feed premix for animal.

Formulation Example 23

7.2 g of any one of Compounds of Present Invention 1-1 to 1-30 and 92.8 g of VOSCO S-55 (manufactured by Maruishi Pharmaceutical Co., Ltd.) are dissolved and mixed at 100°C, poured into a suppository mold, and cooled and solidified to obtain a suppository.
Next, the pest control effect of the compound of the present invention is shown as test examples.

Test Example 1

The formulations of Compounds of Present Invention 1-1, 1-2, 1-3, 1-4, 1-6, 1-7, 1-8 and 1-28 as obtained in Formulation Example 5 were diluted with water, so as to have a concentration of the active ingredient of 500 ppm, to prepare a test drug solution.
On the other hand, on a cucumber seedling (the first true leaf stage) planted in a plastic cup was inoculated with about 30 Aphis gossypii (whole stage), and left for a day. 20 ml of the test drug solution was sprayed on the seedling
Six days after spraying, the number of surviving Aphis gossypii parasitic on the leaves of the cucumber was examined, and the controlling value was calculated according to the following equation:

$$\text{Controlling value (\%)} = \{1 - (Cb \times Tai)/(Cai \times Tb)\} \times 100$$

wherein the symbols represent as follows:

Cb: the number of insects in a non-treated section before treatment
Cai: the number of surviving parasitic insects in a non-treated section on observation
Tb: the number of insects in a treated section before treatment
Tai: the number of surviving parasitic insects in a treated section on observation
wherein the non-created section refers to a section where the test drug solution prepared by diluting the formulation obtained as in Formulation Example 5 but not containing the compound of the present invention with the same amount of water as in the treated section was sprayed.

As a result, in the treated section where the test drug solution containing each of Compounds of Present Invention 1-1, 1-2, 1-3, 1-4, 1-6, 1-7, 1-8 and 1-28 was used, the control value was 90% or more.

Test Example 2

The formulation of Compound of Present Invention 1-2 as obtained in Formulation Example 5 was diluted with water, so as to have a concentration of the active ingredient of 500 ppm, to prepare a test drug solution.
On the other hand, a cucumber seedling (the second true leaf stage) planted in a plastic cup was drenched at its foot with 5 ml of the test drug solution, and kept in a greenhouse at 25°C for 7 days. On the cucumber leaf surface was inoculated about 30 Aphis gossypii (whole stage), and further kept in the greenhouse for 6 days, then the number of surviving Aphis gossypii parasitic on the leaves of the cucumber was examined, and the controlling value was calculated according to the following equation:

$$\text{Controlling value (\%)} = \{1 - (Cb \times Tai)/(Cai \times Tb)\} \times 100$$

wherein the symbols represent as follows:

Cb: the number of insects in a non-treated section before treatment
Cai: the number of surviving parasitic insects in a non-treated section on observation
Tb: the number of insects in a treated section before treatment
Tai: the number of surviving parasitic insects in a treated section on observation
wherein the non-treated section refers to a section where the test drug solution prepared by diluting the formulation obtained as in Formulation Example 5 but not containing the compound of the present invention with the same amount of water as in the treated section was sprayed.

As a result, in the treated section where the test drug solution containing Compound of Present Invention 1-2 was used, the controlling value was 90% or more.

Test Example 3

The formulations of Compounds of Present Invention 1-1, 1-2, 1-4 and 1-28 obtained in Formulation Example 5 were diluted with water so as to have a concentration of the active ingredient of 500 ppm to prepare a test drug solution.
On a rice seedling in the second leaf stage planted in a polyethylene cup was sprayed 10 ml of each test drug

solution. After air-drying, 20 third-fourth instar larvae of Nilaparvata lugens were released, and kept in the greenhouse at 25°C. After 6 days, the number of surviving Nilaparvata lugens parasitic on the rice was examined, and the controlling value was calculated according to the following equation:

$$\text{Controlling value (\%)} = \{1 - (Cb \times Tai)/(Cai \times Tb)\} \times 100$$

wherein the symbols represent as follows:

Cb: the number of insects in a non-treated section before treatment
Cai: the number of surviving parasitic insects in a non-treated section on observation
Tb: the number of insects in a treated section before treatment
Tai: the number of surviving parasitic insects in a treated section on observation
wherein the non-treated section refers to a section where the test drug solution prepared by diluting the formulation obtained as in Formulation Example 5 but not containing the compound of the present invention with the same amount of water as in the treated section was sprayed.

As a result, in the treated section where the test drug solution containing each of Compounds of Present Invention 1-1, 1-2, 1-4 and 1-28 was used, the control value was 90% or more.

Test Example 4

The formulations of Compounds of Present Invention 1-4, 1-7 and 1-28 as obtained in Formulation Example 5 were diluted with water, so as to have a concentration of the active ingredient of 500 ppm, to prepare a test drug solution.
On the other hand, a rice seedling (2 weeks after sowing, the second leaf stage) planted in a plastic cup was drenched at its foot with 5 ml of each test drug solution, and kept in a greenhouse of 25°C for 7 days. 20 third-fourth instar larvae of Nilaparvata lugens were released, and further kept in the greenhouse for 6 days, then the number of surviving Nilaparvata lugens parasitic on the rice leaves was examined, and the controlling value was calculated according to the following equation:

$$\text{Controlling value (\%)} = \{1 - (Cb \times Tai)/(Cai \times Tb)\} \times 100$$

wherein the symbols represent as follows:

Cb: the number of insects in a non-treated section before treatment
Cai: the number of surviving parasitic insects in a non-treated section on observation
Tb: the number of insects in a treated section before treatment
Tai: the number of surviving parasitic insects in a treated section on observation
wherein the non-treated section refers to a section where the test drug solution prepared by diluting the formulation obtained as in Formulation Example 5 but not containing the compound of the present invention with the same amount of water as in the treated section was sprayed.

As a result, in the treated section where the test drug solution containing each of Compounds of Present Invention 1-4, 1-7 and 1-28 was used, the control value was 90% or more.

Test Example 5

The formulation of the compound of the present invention as obtained in Formulation Example 5 is diluted with water, so as to have a concentration of the active ingredient of 500 ppm, to prepare a test drug solution.
On the other hand, Bemisia tabaci adult is released on a tomato seedling (the third true leaf stage) planted in a polyethylene cup, and made to lay eggs for about 72 hours. The tomato seedling is kept in a greenhouse for 8 days, and when instar larvae hatch from the eggs, the above test drug solution is sprayed at a rate of 20 ml/cup, and the cup is kept in a greenhouse at 25°C. After 7 days, the number of surviving instar larvae on the tomato leaves is examined, and the controlling value is calculated according to the following equation. The control value is excellent in the treated section where the formulation of the compound of the present invention was used.

$$Controlling\ value\ (\%) = \{1 - (Cb \times Tai)/(Cai \times Tb)\} \times 100$$

In the above equation, the symbols represent as follows:

Cb: the number of instar larvae in a non-treated section before treatment
Cai: the number of surviving instar larvae in a non-treated section on observation
Tb: the number of instar larvae in a treated section before treatment
Tai: the number of surviving instar larvae in a treated section on observation
wherein the non-treated section refers to a section where the test drug solution prepared by diluting the formulation obtained as in Formulation Example 5 but not containing the compound of the present invention with the same amount of water as in the treated section was sprayed.

Test Example 6

The formulations of Compounds of Present Invention 1-1, 1-2, 1-3, 1-4, 1-6, 1-7, 1-8 and 1-28 as obtained in Formulation Example 5 were diluted with water, so as to have a concentration of the active ingredient of 500 ppm, to prepare a test drug solution.

On the other hand, on cabbage at the third leaf stage planted in a polyethylene cup was sprayed, at a rate 20 mL/cup, the test drug solution. After the drug solution was dried, the foliage part was cut off, and then placed in a 50 mL volume cup. Five second instar larvae of Plutella xylostella were released into the cup, and the cup was sealed with a lid. After the cup was kept at 25°C for 5 days, the number of dead insects was counted. The death rate was calculated according to the following equation.

$$Death\ rate\ (\%) = (Number\ of\ dead\ insects/Number\ of\ tested\ insects) \times 100$$

As a result, in the treated section where the test drug solution containing each of Compounds of Present Invention 1-1, 1-2, 1-3, 1-4, 1-6, 1-7, 1-8 and 1-28 was used, the death rate was 80% or more.

Test Example 7

The formulations of Compounds of Present Invention 1-1, 1-2, 1-4, 1-6, 1-7, 1-8 and 1-28 obtained in Formulation Example 5 were diluted with water, so as to have a concentration of the active ingredient of 500 ppm, to prepare a test spray solution.

On the other hand, an apple tree was planted in a plastic cup, and grown until the seventh-eighth true leaf was spread. To the apple tree was sprayed, at a rate of 20 mL/cup, the test drug solution. After the drug solution was dried, 60 first-instar larvae of Adoxophyes orana fasciata were released, and covered with a plastic cup the bottom of which was cut off and on which a filter paper was put, with the plastic cup cover placed upside-down. After 7 days, the number of dead insects was counted, and the death rate was calculated according to the following equation.

$$Death\ rate\ (\%) = (Number\ of\ dead\ insects/Number\ of\ tested\ insects) \times 100$$

As a result, in the treated section where the test drug solution containing each of Compounds of Present Invention 1-1, 1-2, 1-4, 1-6, 1-7, 1-8 and 1-28 was used, the death rate was 90% or more.

Test Example 8

The formulation of the compound of the present invention as obtained in Formulation Example 5 is diluted with water, so as to have a concentration of the active ingredient of 500 ppm, to prepare a test drug solution.

A filter paper having a diameter of 5.5 cm is spread on the bottom of a polyethylene cup having the same diameter and 0.7 ml of the test drug solution is added dropwise onto the filter paper, and 30 mg of sucrose is uniformly

placed as bait. Into the polyethylene cup, 10 female imagoes of Musca domestica are released, and the cup is sealed with a lid. After 24 hours, the life and death of Musca domestica is examined, and the number of dead insects is counted. The death rate is calculated according to the following equation, and the sufficient death rate is obtained.

Death rate (%) = (Number of dead insects/Number of tested insects) × 100

Test Example 9

The formulation of Compound of Present Invention 1-3 as obtained in Formulation Example 5 was diluted with water, so as to have a concentration of the active ingredient of 500 ppm, to prepare a test drug solution.

A filter paper having a diameter of 5.5 cm was spread on the bottom of a polyethylene cup having the same diameter and 0.7 ml of the test drug solution was added dropwise onto the filter paper, and 30 mg of sucrose was uniformly placed as bait. Into the polyethylene cup, 2 male imagoes of Blattella germanica were released, and the cup was sealed with a lid. After 6 days, the life and death of Blattella germanica was examined, the number of dead insects was counted, and the death rate was calculated according to the following equation.

Death rate (%) = (Number of dead insects/Number of tested insects) × 100

As a result, in the treated section where the test drug solution containing Compound of Present Invention 1-3 was used, the death rate was 100%.

Test Example 10

The formulations of Compounds of Present Invention 1-1, 1-2, 1-3, 1-4, 1-6, 1-7, 1-8 and 1-28 as obtained in Formulation Example 5 were diluted with water, so as to have a concentration of the active ingredient of 500 ppm, to prepare a test drug solution.

20 Last-instar larvae of Culex pipiens pallens were released into a liquid to which 0.7 ml of the above test drug solution was added to 100 ml of ion-exchanged water (a concentration of the active ingredient of 3.5 ppm). After One day, the number of dead insects was counted, and the death rate was calculated according to the following equation.

Death rate (%) = (Number of dead insects/Number of tested insects) × 100

As a result, in the treated section where the test drug solution containing each of Compounds of Present Invention 1-1, 1-2, 1-3, 1-4, 1-6, 1-7, 1-8 and 1-28 was used, the death rate was 95% or more.

Test Example 11

Compound of Present Invention 1-2 (2 mg) was weighed in a screw tube (Maruemu No. 5; 27 × 55 mm), 0.2 mL of acetone was added thereto, and the screw tube was sealed with a cap to dissolve the compound. The screw tube was rotated and inverted to uniformly coat the drug solution onto the whole inner wall of the tube. After removing the cap, the solution was air-dried for about 2 hours, then unfed nymphal ticks, Haemaphysalis longicornis (5 ticks/group) were released, and the tube was sealed with the cap. After 2 days, the number of dead insects was counted, and the death rate was calculated according to the following equation.

Death rate (%) = 100 × (Number of dead insects/Number of tested insects)

As a result, in the treated section where the test drug solution containing Compound of Present Invention 1-2 was used, the death rate was 100%.

INDUSTRIAL APPLICABILITY

The compound of the present invention has a controlling effect on pests and is useful as an active ingredient of a pest control agent.

## Claims

**1.** A fused heterocyclic compound represented by formula (1) :

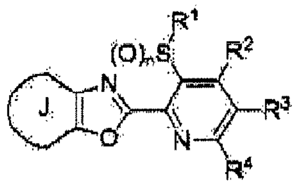

(1)

wherein

$R^1$ represents a C1 to C6 alkyl group optionally having one or more halogen atoms or one or more cyclopropyl groups (wherein the cyclopropyl group optionally has one or more halogen atoms or one or more C1 to C3 alkyl groups), a C2 to C6 alkenyl group optionally having one or more halogen atoms, a C2 to C6 alkynyl group optionally having one or more halogen atoms or a C3 to C6 cycloalkyl group optionally having one or more halogen atoms or one or more C1 to C3 alkyl groups;
$R^2$ and $R^4$ are the same or different and represent a C1 to C3 alkyl group optionally having one or more halogen atoms, a halogen atom or a hydrogen atom;
$R^3$ represents a C1 to C6 alkyl group optionally having one or more halogen atoms, a C2 to C6 alkenyl group optionally having one or more halogen atoms, a C2 to C6 alkynyl group optionally having one or more halogen atoms, a 5- or 6-membered heterocyclic group (wherein the 5- or 6-membered heterocyclic group optionally has one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a cyano group, a nitro group, a halogen atoms, a hydrogen atom or $SF_5$;
$R^5$ represents a C1 to C6 alkyl group optionally having one or more halogen atoms or a hydrogen atom;
n represents 0, 1 or 2;
m represents 0, 1 or 2; and
ring J represents ring J1 or ring J2 of the following formula:

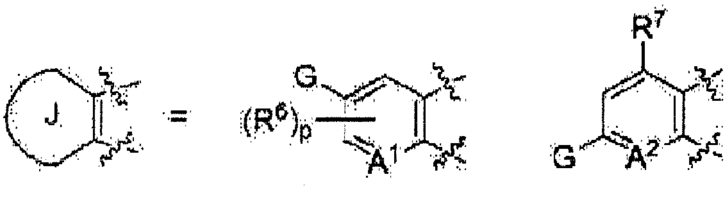

(J) (J1) (J2)

wherein
$A^1$ represents N, CH or $CR^6$;
$A^2$ represents N or $CR^8$;
G represents a C1 to C3 perhaloalkyl group, $OR^9$ or $S(O)_mR^9$ (wherein $R^9$ represents a C1 to C3 perhaloalkyl group);
$R^6$ represents a C1 to C6 alkyl group optionally having one or more halogen atoms, a C2 to C6 alkenyl group

optionally having one or more halogen atoms, a C2 to C6 alkynyl group optionally having one or more halogen atoms, $OR^5$, $S(O)_mR^5$, $S(O)_2NR^5R^{10}$, $NR^5R^{10}$, $NR^5C(O)R^{10}$, $NR^5CO_2R^{10}$, $C(O)R^5$, $CO_2R^5$, $C(O)NR^5R^{10}$, a cyano group or a halogen atom (wherein, when p is 2, $R^6$s may be different from each other);
$R^7$ and $R^8$ are the same or different and represent a C1 to C6 alkyl group optionally having one or more halogen atoms, a C2 to C6 alkynyl group optionally having one or more halogen atoms, a C2 to C6 alkynyl group optionally having one or more halogen atoms, $OR^5$, $S(O)_mR^5$, $S(O)_2NR^5R^{10}$, $NR^5R^{10}$, $NR^5C(O)R^{10}$, $NR^5CO_2R^{10}$, $C(O)R^5$, $CO_2R^5$, $C(O)NR^5R^{10}$, a cyano group, a halogen atom or a hydrogen atom;
$R^{10}$ represents a C1 to C6 alkyl group optionally having one or more halogen atoms or a hydrogen atom; and p represents 0, 1 or 2 (wherein, when p is 0, $A^1$ is $CR^6$), wherein, in $S(O)_mR^5$, when m is 1 or 2, $R^5$ does not represent a hydrogen atom, or
an N-oxide thereof (in the case of the N-oxide, n represents 2, and m represents 2).

**2.** The compound according to claim 1, wherein

$R^1$ is a C1 to C6 alkyl group optionally having one or more halogen atoms or one or more cyclopropyl groups (wherein the cyclopropyl group optionally has one or more halogen atoms or one or more C1 to C3 alkyl groups);
both $R^2$ and $R^4$ are a hydrogen atom;
$R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a pyridyl group, a pyrimidinyl group (wherein the pyridyl group and the pyrimidinyl group optionally have one or more atoms or groups selected from the group consisting of halogen atoms, C1 to C3 alkyl groups optionally having one or more halogen atoms, and C1 to C3 alkoxy groups optionally having one or more halogen atoms), $OR^5$, $S(O)_mR^5$, a halogen atom or a hydrogen atom;
$R^5$ is a C1 to C6 alkyl group optionally having one or more halogen atoms;
$A^1$ is CH or $CR^6$;
$A^2$ is CH;
G is a C1 to C3 perfluoroalkyl group, $OR^{11}$ or $S(O)_mR^{11}$ (wherein $R^{11}$ represents a C1 to C3 perfluoroalkyl group);
$R^6$ is a C1 to C6 alkyl group optionally having one or more halogen atoms, $OR^5$, $S(O)_mR^5$, $NR^5R^{10}$ or a halogen atom;
.$R^7$ is a C1 to C6 alkyl group optionally having one or more halogen atoms, $OR^5$, $S(O)_mR^5$, $NR^5R^{10}$, a halogen atom or a hydrogen atom; and
p is 0 or 1 (wherein, when p is 0, $A^1$ is $CR^6$).

**3.** The compound according to claim 1 or claim 2, wherein the ring J is ring J1.

**4.** The compound according to claim 1 or claim 2, wherein the ring J is ring J2.

**5.** A pest control agent comprising the compound as defined in any one of claim 1 to claim 4, and an inert carrier.

**6.** A method for controlling pests comprising applying an effective amount of the compound as defined in any one of claim 1 to claim 4 to a pest or a pest-infested area.

## INTERNATIONAL SEARCH REPORT

International application No. PCT/JP2014/052813

A. CLASSIFICATION OF SUBJECT MATTER
*C07D413/04*(2006.01)i, *A01N43/76*(2006.01)i, *A01N47/02*(2006.01)i, *A01P7/02*(2006.01)i, *A01P7/04*(2006.01)i, *A61K31/4439*(2006.01)i, *A61P33/14*(2006.01)i

According to International Patent Classification (IPC) or to both national classification and IPC

B. FIELDS SEARCHED

Minimum documentation searched (classification system followed by classification symbols)
C07D413/04, A01N43/76, A01N47/02, A01P7/02, A01P7/04, A61K31/4439, A61P33/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
REGISTRY(STN), CAplus(STN)

C. DOCUMENTS CONSIDERED TO BE RELEVANT

| Category* | Citation of document, with indication, where appropriate, of the relevant passages | Relevant to claim No. |
|---|---|---|
| Y | WO 2009/131237 A1 (Sumitomo Chemical Co., Ltd.), 29 October 2009 (29.10.2009), claims; page 88, lines 5 to 8; preparation examples 41 to 47, 134 to 135, 145 to 148 & CN 102006776 A & EP 2274983 A1 & JP 2009-280574 A & KR 10-2010-0134056 A & US 2011/0039843 A1 & US 2012/0108586 A1 & US 8445522 B2 & US 2013/0190271 A1 | 1-6 |
| Y | JP 2011-105712 A (Sumitomo Chemical Co., Ltd.), 02 June 2011 (02.06.2011), claims; paragraph [0125]; preparation examples 41 to 47, 134 to 135, 145 to 148 & WO 2011/049223 A1 | 1-6 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| | |
|---|---|
| Date of the actual completion of the international search<br>07 April, 2014 (07.04.14) | Date of mailing of the international search report<br>22 April, 2014 (22.04.14) |
| Name and mailing address of the ISA/<br>Japanese Patent Office<br><br>Facsimile No. | Authorized officer<br><br><br>Telephone No. |

Form PCT/ISA/210 (second sheet) (July 2009)

**INTERNATIONAL SEARCH REPORT**

International application No.
PCT/JP2014/052813

C (Continuation). DOCUMENTS CONSIDERED TO BE RELEVANT

| Category* | Citation of document, with indication, where appropriate, of the relevant passages | Relevant to claim No. |
|---|---|---|
| Y | Takuzo HISANO et al., Chem. Pharm. Bull., 1982, Vol.30, p.2996-3004 | 1-6 |
| P,X | JP 2014-5263 A (Sumitomo Chemical Co., Ltd.), 16 January 2014 (16.01.2014), claims; invention compounds 80 to 87 & WO 2013/018928 A1 | 1-6 |
| P,X | WO 2013/180194 A1 (Sumitomo Chemical Co., Ltd.), 05 December 2013 (05.12.2013), claims (Family: none) | 1-6 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004034438 A
- WO 2009131237 A
- EP 1380568 A
- WO 2004010936 A